# EUROPEAN PATENT SPECIFICATION

(11) **EP 3 823 621 B1**
(45) Date of publication and mention of the grant of the patent: **07.10.2026**
(21) Application number: 19746351.6
(22) Date of filing: 15.07.2019
(51) Int. Cl.: A61K 31/4427, A61K 31/4439, A61K 45/06, A61K 9/00, A61K 9/08, A61K 9/20, A61K 9/48, A61P 31/18

(54) **CAPSID INHIBITORS FOR USE IN THE TREATMENT OF HIV**
CAPSIDINHIBITOREN ZUR VERWENDUNG IN DER BEHANDLUNG VON HIV
INHIBITEURS DE CAPSIDES POUR UTILISATION DANS LE TRAITEMENT DU VIH

(30) Priority: 16.07.2018 US 201862698611 P
(43) Date of publication of application: 26.05.2021
(73) Proprietor: GILEAD SCIENCES, INC., Foster City, California 94404 (US)
(72) Inventor: BAUER, Laura Elizabeth, Foster City, California 94404 (US); GORMAN, Eric M., Foster City, California 94404 (US); MULATO, Andrew Stephen, Foster City, California 94404 (US); RHEE, Martin Sunkwang, Foster City, California 94404 (US); ROWE, Charles William, Foster City, California 94404 (US); SELLERS, Scott P., Foster City, California 94404 (US); STEFANIDIS, Dimitrios, Foster City, California 94404 (US); TSE, Winston C., Redwood City, California 94065 (US); YANT, Stephen R., Foster City, California 94404 (US); CHIU, Anna, Foster City, California 94404 (US); LEVINE, Dana, J., Foster City, California 94404 (US)
(74) Representative: Carpmaels & Ransford LLP
(86) International application number: PCT/US2019/041880
(87) International publication number: WO 2020/018459

(56) References cited:
- WO-A1-2018/035359
- WINSTON C. TSE: "Discovery of Novel Potent HIV Capsid Inhibitors with Long-Acting Potential", 14 February 2017 (2017-02-14), XP055632979, Retrieved from the Internet <URL:http://www.natap.org/2017/CROI/croi_17.htm> [retrieved on 20191016]
- LISA M. JARVIS ET AL: "Conquering HIV's capsid", 31 July 2017 (2017-07-31), XP055632804, Retrieved from the Internet <URL:https://pubs.acs.org/doi/pdf/10.1021/cen-09531-bus2> [retrieved on 20191016]

## Description

### FIELD

The present disclosure relates to pharmaceutical compositions, which may be for use in methods for the treatment of a human immunodeficiency virus (HIV) infection in heavily treatment-experienced patients (e.g., patients with multidrug resistant HIV infection).

### BACKGROUND

The viral capsid protein (CA) is essential for multiple stages of the HIV life cycle. During viral maturation following the processing of Gag polyprotein by the HIV protease, CA self-assembles into the conical shaped core characteristic of mature HIV-1 virions. Contained within this capsid core are the viral RNA, nucleocapsid, reverse transcriptase, and integrase. Failure to generate a suitable core precludes infectivity. In addition, CA contributes to multiple essential processes during the early stages of HIV replication, including important roles in regulating proper capsid core disassembly (uncoating) kinetics to ensure efficient and productive viral DNA synthesis via coupled reverse transcription, and contributes to the active transport of pre-integration complexes into the nuclear compartment to support viral DNA integration into transcriptionally active loci. Defects in the proper function of capsid ultimately inhibit efficient nuclear uptake and integration of viral DNA into the host genome.

There are currently a number of antiretroviral drugs available to combat HIV infection. These drugs can be divided into classes based on the viral protein they target or their mode of action. Used alone, these drugs are effective in reducing viral replication. However, the effect is only temporary as the virus readily develops resistance to all known agents used as monotherapy. Combination therapy has proven very effective at both reducing virus and suppressing the emergence of resistance in a number of patients. In the US, where combination therapy is widely available, the number of HIV-related deaths has dramatically declined (Palella, F. J.; Delany, K. M.; Moorman, A. C.; Loveless, M. O.; Furher, J.; Satten, G. A.; Aschman, D. J.; Holmberg, S. D.; N. Engl. J. Med. 1998, 338:853-860).

Despite the success of combination antiretroviral therapy, a significant proportion of patients experience a loss of virologic, immunologic, or clinical benefit from their current regimens, leading to treatment failure and a rebound of the amount of HIV that can be measured in the blood. Initial studies suggest that approximately 30-50% of patients ultimately fail at least one drug in the suppressive combination. Treatment failure can be caused by the emergence of viral resistance. Viral resistance in turn can be caused by the replication rate of HIV-1 during the course of infection combined with the relatively high viral mutation rate associated with the viral polymerase and the lack of adherence of HIV-infected individuals in taking their prescribed medications. Because of structural similarities of the drugs within an antiretroviral class, cross resistance is commonly seen to the other members of that class (for example, virologic failure on a regimen containing a non-nucleoside reverse transcriptase inhibitor (NNRTI) will lead to cross resistance to the other first generation NNRTI agents). As patients experience repeated virologic failure on antiretroviral combination therapy, their viruses develop broad multi-class antiretroviral drug resistance which limits the effectiveness of the next round of antiretroviral therapy. Many heavily treatment-experienced patients have viral resistance to several classes of antiretroviral drugs and there are often not even two drugs that remain fully active to form the core of a new, effective antiretroviral drug regimen. Frequently, these patients have limited options for alternative treatment regimens and are at risk of significant morbidity and mortality.

Thus, there is a need for safe and effective therapies for heavily treatment-experienced patients who have limited treatment options due to antiretroviral drug resistance.

### SUMMARY

The invention is set out in the appended set of claims. The present invention relates to a pharmaceutical composition comprising a sodium salt of the compound of Formula **(Ia):** 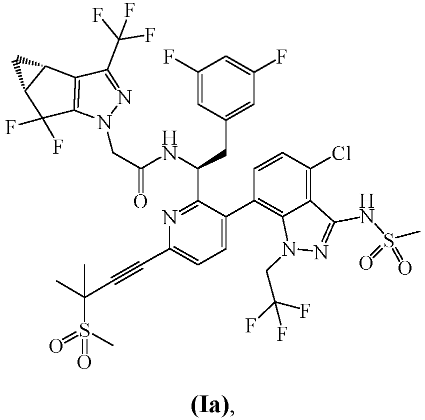 wherein the pharmaceutical composition is in the form of a solution and the solution comprises 35 w/w% to 75 w/w% PEG 300, 10 w/w% to 40 w/w% water and 5 w/w% to 35 w/w% of the sodium salt of the compound of Formula **(Ia).** The invention also relates to the pharmaceutical composition comprising the sodium salt of the compound of Formula **(Ia)** for use in methods of treating human immunodeficiency virus (HIV) infection in a heavily treatment-experienced patient.

In some embodiments, the HIV infection is an HIV-1 infection characterized by HIV-1 mutant resistance to one or more antiretroviral medications. In some embodiments, the HIV infection is an HIV-1 infection characterized by HIV-1 mutant resistance to two or more antiretroviral medications. In some embodiments, the HIV infection is an HIV-1 infection characterized by HIV-1 mutant resistance to three or more antiretroviral medications.

In some embodiments, the HIV-1 mutant is resistant to a protease inhibitor (PI), a nucleoside or nucleotide reverse transcriptase inhibitor (NRTI), a non-nucleoside or non-nucleotide reverse transcriptase inhibitor (NNRTI), or an integrase strand transfer inhibitor (INSTI). In certain embodiments, the HIV-1 mutant resistant to a protease inhibitor is selected from I50V, I84V/L90M, G48V/V82A/L90M, and G48V/V82S. In certain embodiments, the HIV-1 mutant resistant to a nucleoside or nucleotide reverse transcriptase inhibitor is selected from K65R, M184V, and 6TAMs. In certain embodiments, the HIV-1 mutant resistant to a non-nucleoside or non-nucleotide reverse transcriptase inhibitor is selected from K103N, Y181C, Y188L, L100I/K103N, and K103N/Y181C. In certain embodiments, the HIV-1 mutant resistant to a integrase strand transfer inhibitor is selected from Y143R, E138K/Q148K, G140S/Q148R, E92Q/N155H, N155H/Q148R, and R263K/M50I.

In some embodiments, the patient is infected with HIV-1 resistant to at least one antiretroviral medication. In some embodiments, the patient is infected with multidrug resistant HIV-1 which is resistant to at least one antiretroviral medication from each of two different classes of antiretroviral medications. In some embodiments, the patient is infected with multidrug resistant HIV-1 which is resistant to at least one antiretroviral medication from each of three different classes of antiretroviral medications. In some embodiments, the different classes of antiretroviral medications are selected from a nucleoside or nucleotide reverse transcriptase inhibitor (NRTI), a non-nucleoside or non-nucleotide reverse transcriptase inhibitor (NNRTI), a protease inhibitor (PI), and an integrase strand transfer inhibitor (INSTI).

In some embodiments, the NRTI is selected from emtricitabine, lamivudine (3TC), zidovudine (azidothymidine (AZT)), didanosine (ddI), dideoxyinosine, tenofovir, tenofovir alafenamide, tenofovir disoproxil fumarate, stavudine (d4T), zalcitabine (dideoxycytidine, ddC), and abacavir.

In some embodiments, the NNRTI is selected from efavirenz, etravirine, rilpivirine, nevirapine, and delavirdine.

In some embodiments, the PI is selected from amprenavir, atazanavir, darunavir, fosamprenavir, indinavir, lopinavir, nelfinavir, ritonavir, saquinavir, and tipranavir.

In some embodiments, the INSTI is selected from raltegravir, elvitegravir, dolutegravir, and bictegravir.

In some embodiments, the patient had been previously treated with at least one antiretroviral medication for at least 3 months, at least 6 months, at least 9 months, or at least 12 months.

In some embodiments, the patient failed a prior HIV treatment regimen including administration of at least one antiretroviral medication. In certain embodiments, the prior treatment regimen included administration of at least one antiretroviral medication from each of two different classes of antiretroviral medications. In certain embodiments, the prior treatment regimen included administration of at least one antiretroviral medication from each of three different classes of antiretroviral medications. In some embodiments, the different classes of antiretroviral medications are selected from a nucleoside reverse transcriptase inhibitor (NRTI), a non-nucleoside reverse transcriptase inhibitor (NNRTI), a protease inhibitor (PI), and an integrase strand transfer inhibitor (INSTI).

In certain embodiments, the pharmaceutical composition comprising the sodium salt of the compound of Formula **(Ia)** is administered subcutaneously, intramuscularly, or intravenously.

In some embodiments, the pharmaceutical composition comprising the sodium salt of the compound of Formula **(Ia)** is administered subcutaneously.

In some embodiments, the the sodium salt of the compound of Formula **(Ia)** is formulated with one or more other compounds selected from HIV protease inhibitors, HIV non-nucleoside or non-nucleotide inhibitors of reverse transcriptase, HIV nucleoside or nucleotide inhibitors of reverse transcriptase, HIV integrase inhibitors, HIV non-catalytic site (or allosteric) integrase inhibitors, HIV nucleoside reverse transcriptase translocation inhibitors, and pharmacokinetic enhancers. In some embodiments, the HIV nucleoside reverse transcriptase translocation inhibitor is 4'-ethynyl-2-fluoro-2'-deoxyadenosine triphosphate (MK-8591).

In some embodiments, the pharmaceutical composition comprising the sodium salt of the compound of Formula **(Ia)** is administered once daily. In some embodiments, the pharmaceutical composition comprising the sodium salt of the compound of Formula **(Ia)** is administered once every 1 week, 2 weeks, 4 weeks, 8 weeks, 12 weeks, 16 weeks, 20 weeks, 24 weeks, or 48 weeks.

In some embodiments, the patient has a viral load of greater than about 200 copies of HIV-1 RNA/mL, greater than about 500 copies of HIV-1 RNA/mL, greater than about 750 copies of HIV-1 RNA/mL, greater than about 1000 copies of HIV-1 RNA/mL, or greater than about 2000 copies of HIV-1 RNA/mL at the time of beginning administration of the pharmaceutical composition comprising the sodium salt of the compound of Formula **(Ia).**

In some embodiments, the patient is failing an HIV treatment regimen including administration of at least one antiretroviral medication at the time of beginning administration of the pharmaceutical composition comprising the sodium salt of the compound of Formula **(Ia).**

In some embodiments, administration of the pharmaceutical composition comprising the sodium salt of the compound of Formula **(Ia)** results in a decrease in the viral load in the patient. In some embodiments, the viral load is decreased by about 0.5 log₁₀ to about 2.5 log₁₀ after administration of the pharmaceutical composition comprising the sodium salt of the compound of Formula **(Ia)** for about 24 weeks as compared to the viral load at the time of beginning administration of the pharmaceutical composition comprising the sodium salt of the compound of Formula **(Ia).** In some embodiments, the viral load is decreased by about 0.5 log₁₀, about 1 log₁₀, about 1.5 log₁₀, about 2 log₁₀, or about 2.5 log₁₀ after administration of the pharmaceutical composition comprising the sodium salt of the compound of Formula **(Ia)** for about 24 weeks as compared to the viral load at the time of beginning administration of the pharmaceutical composition comprising the sodium salt of the compound of Formula **(Ia).**

In some embodiments, the viral load in the patient is about 200 copies of HIV-1 RNA/mL or less after administration of the pharmaceutical composition comprising the sodium salt of the compound of Formula **(Ia)** for about 24 weeks. In some embodiments, the viral load in the patient is about 50 copies of HIV-1 RNA/mL or less after administration of the pharmaceutical composition comprising the sodium salt of the compound of Formula **(Ia)** for about 24 weeks.

In some embodiments, the patient is concurrently treated with at least one additional antiretroviral medication. In some embodiments, the additional antiretroviral medication is selected from a nucleoside reverse transcriptase inhibitor (NRTI), a non-nucleoside reverse transcriptase inhibitor (NNRTI), a protease inhibitor (PI), an integrase strand transfer inhibitor (INSTI), a gp41 fusion inhibitor, a CCR5 co-receptor antagonist, and combinations thereof.

In some embodiments, the NRTI is selected from emtricitabine, lamivudine (3TC), zidovudine (azidothymidine (AZT)), didanosine (ddI), dideoxyinosine, tenofovir, tenofovir alafenamide, tenofovir disoproxil fumarate, stavudine (d4T), zalcitabine (dideoxycytidine, ddC), and abacavir.

In some embodiments, the NNRTI is selected from efavirenz, etravirine, rilpivirine, nevirapine, and delavirdine.

In some embodiments, the PI is selected from amprenavir, atazanavir, darunavir, fosamprenavir, indinavir, lopinavir, nelfinavir, ritonavir, saquinavir, and tipranavir.

In some embodiments, the INSTI is selected from raltegravir, elvitegravir, dolutegravir, and bictegravir.

In some embodiments, the gp41 fusion inhibitor is selected from albuvirtide, enfuvirtide, BMS-986197, enfuvirtide biobetter, enfuvirtide biosimilar, HIV-1 fusion inhibitors (P26-Bapc), ITV-1, ITV-2, ITV-3, ITV-4, PIE-12 trimer, and sifuvirtide.

In some embodiments, the CCR5 co-receptor antagonist is selected from aplaviroc, vicriviroc, maraviroc, cenicriviroc, PRO-140, adaptavir (RAP-101), nifeviroc (TD-0232), anti-GP120/CD4 or CCR5 bispecific antibodies, B-07, MB-66, polypeptide C25P, TD-0680, and vMIP (Haimipu).

Also included in the present disclosure is a pharmaceutical composition according to the claims for use in a method of treating human immunodeficiency virus-1 (HIV-1) infection in a heavily treatment-experienced patient with multidrug resistant HIV-1, the method including administering to the patient a therapeutically effective amount of the pharmaceutical composition comprising the sodium salt of the compound of Formula **(Ia):** 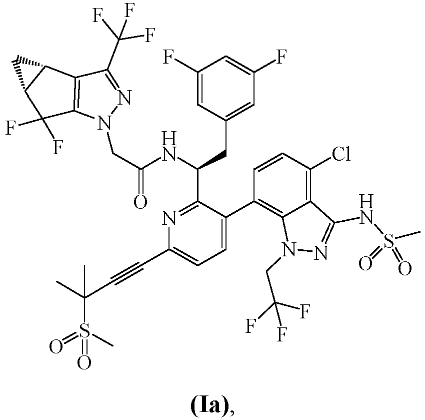 where the patient had been previously treated with an HIV treatment regimen including administration of at least one antiretroviral medication and failed the treatment regimen; and administration of the compound results in a reduction in HIV viral load in the patient.

In some embodiments, the multidrug resistant HIV-1 is resistant to at least one antiretroviral medication from each of two different classes of antiretroviral medications. In some embodiments, the multidrug resistant HIV-1 is resistant to at least one antiretroviral medication from each of three different classes of antiretroviral medications. In some embodiment, the different classes of antiretroviral medications are selected from a nucleoside reverse transcriptase inhibitor (NRTI), a non-nucleoside reverse transcriptase inhibitor (NNRTI), a protease inhibitor (PI), and an integrase strand transfer inhibitor (INSTI).

In some embodiments, the patient had been previously treated with at least one antiretroviral medication for at least 3 months, at least 6 months, at least 9 months, or at least 12 months. In some embodiments, the prior treatment regimen included administration of at least one antiretroviral medication from each of two different classes of antiretroviral medications. In some embodiments, the prior treatment regimen included administration of at least one antiretroviral medication from each of three different classes of antiretroviral medications. In some embodiments, the different classes of antiretroviral medications are selected from a nucleoside reverse transcriptase inhibitor (NRTI), a non-nucleoside reverse transcriptase inhibitor (NNRTI), a protease inhibitor (PI), and an integrase strand transfer inhibitor (INSTI).

Provided in the present disclosure is a pharmaceutical composition according to the claims for use in a method of treating human immunodeficiency virus-1 (HIV-1) infection in a heavily treatment-experienced patient with multidrug resistant HIV-1, the method including administering to the patient a therapeutically effective amount of the pharmaceutical composition comprising the sodium salt of the compound of Formula **(Ia):** 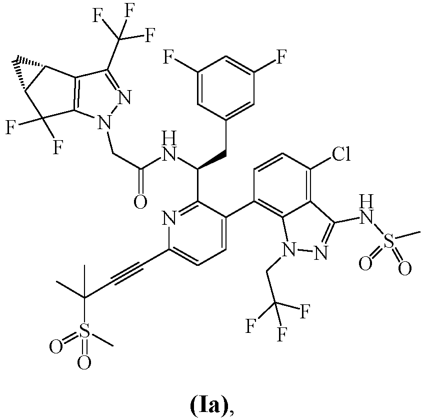 where the multidrug resistant HIV-1 is resistant to at least one antiretroviral medication from each of two different classes of antiretroviral medications, where the different classes of antiretroviral medications are selected from a nucleoside reverse transcriptase inhibitor (NRTI), a non-nucleoside reverse transcriptase inhibitor (NNRTI), a protease inhibitor (PI), and an integrase strand transfer inhibitor (INSTI); the patient had been previously treated with an HIV treatment regimen including administration of at least one antiretroviral medication and failed the treatment regimen; the patient has a viral load of greater than about 200 copies of HIV-1 RNA/mL at the time of beginning administration of the pharmaceutical composition comprising the sodium salt of the compound of Formula **(Ia);** and administration of the compound results in a reduction in HIV viral load in the patient.

Also provided is a pharmaceutical composition according to the claims for use in a method of reducing the viral load associated with an HIV infection in a heavily treatment-experienced patient with multidrug resistant HIV, the method including administering to the patient a therapeutically effective amount of the pharmaceutical composition comprising the sodium salt of the compound of Formula **(Ia):**

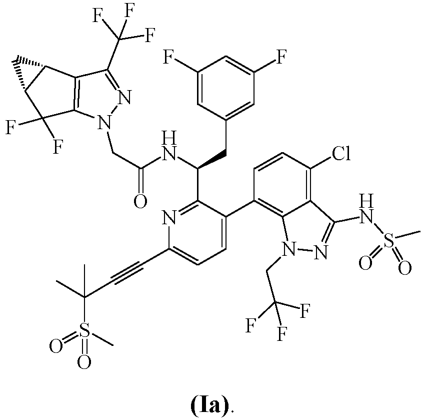

Additional embodiments of the current disclosure are disclosed herein.

### BRIEF DESCRIPTION OF THE DRAWINGS

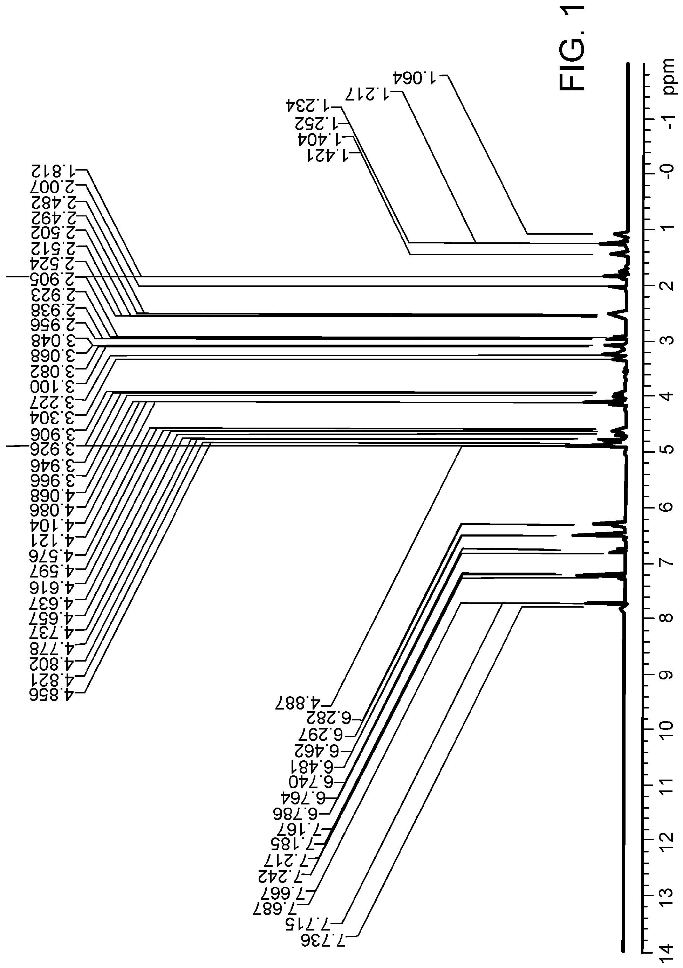

FIG. 1 shows the ¹H NMR (400 MHz, methanol-*d*₄) of the compound of Formula **(Ia):** *N*-((*S*)-1-(3-(4-chloro-3-(methylsulfonamido)-1-(2,2,2-trifluoroethyl)-1*H*-indazol-7-yl)-6-(3-methyl-3-(methylsulfonyl)but-1-yn-1-yl)pyridin-2-yl)-2-(3,5-difluorophenyl)ethyl)-2-((3bS,4aR)-5,5-difluoro-3-(trifluoromethyl)-3b,4,4a,5-tetrahydro-1*H-*cyclopropa[3,4]cyclopenta[1,2-*c*]pyrazol-1-yl)acetamide.

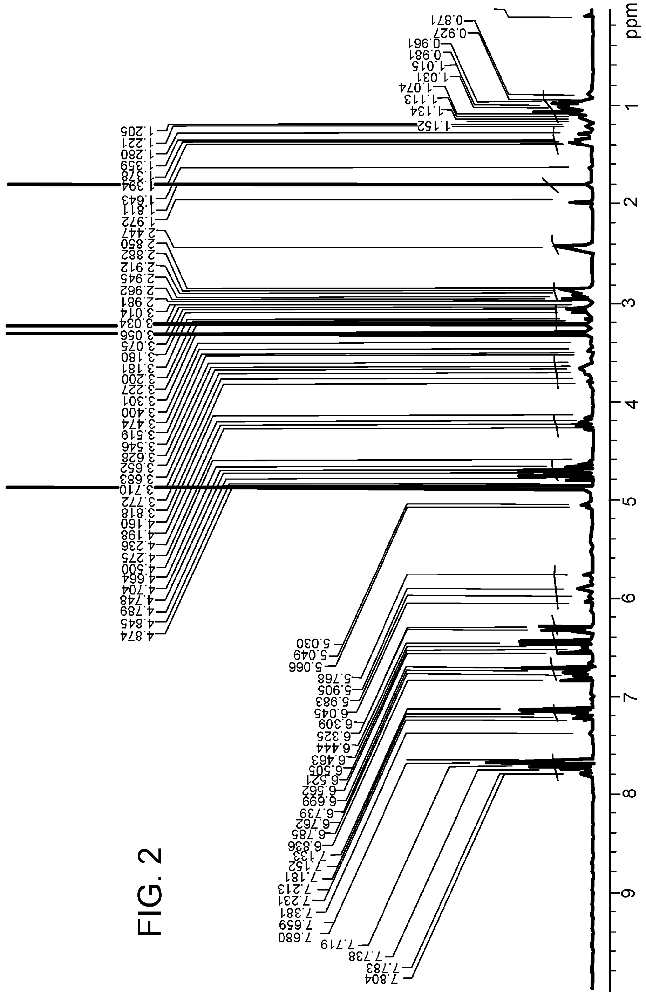

FIG. 2 (reference) shows the ¹H NMR (400 MHz, methanol-*d*₄) of the compound of Formula **(Ib):** N-((S)-1-(3-(4-chloro-3-(cyclopropanesulfonamido)-1-(2,2-difluoroethyl)-1H-indazol-7-yl)-6-(3-methyl-3-(methylsulfonyl)but-1-yn-1-yl)pyridin-2-yl)-2-(3,5-difluorophenyl)ethyl)-2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamide.

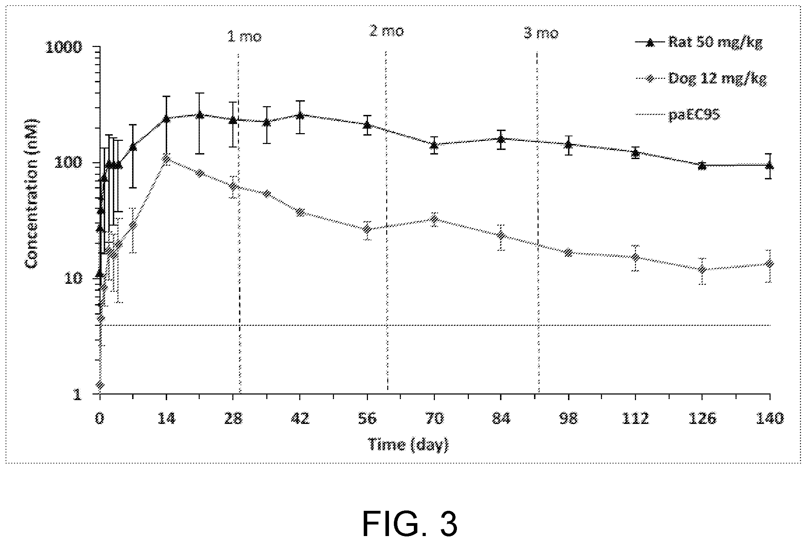

FIG. 3 (reference) shows a plot of plasma concentration over time of 400 mg/mL of the compound of Formula **(Ia)** in 10% ethanol, 13% water, and 77% PEG 200 when dosed subcutaneously in dogs at 12 mg/kg and rats at 50 mg/kg.

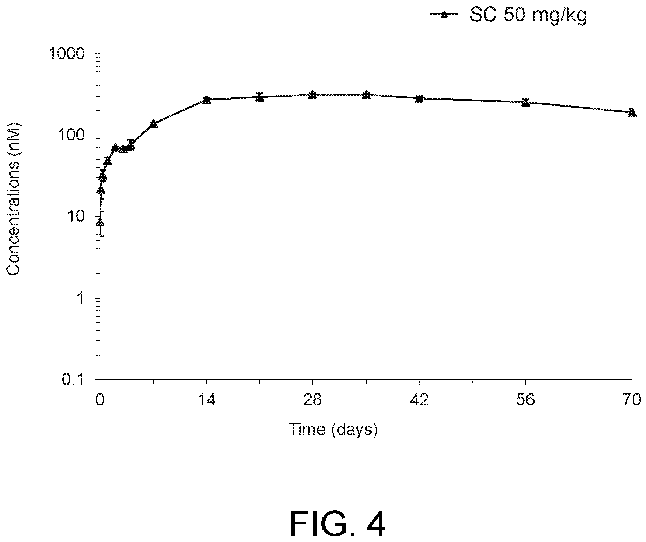

FIG. 4 shows a plot of plasma concentration over time of 300 mg/mL of the compound of Formula **(Ia)** in 15% water and 85% PEG 300 when dosed subcutaneously in rats at 50 mg/kg.

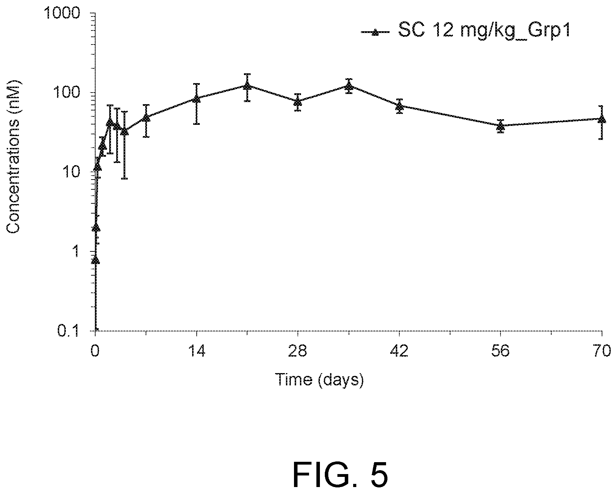

FIG. 5 shows a plot of plasma concentration over time of 400 mg/mL of the compound of Formula **(Ia)** in 15% water and 85% PEG 300 when dosed subcutaneously in dogs at 12 mg/kg.

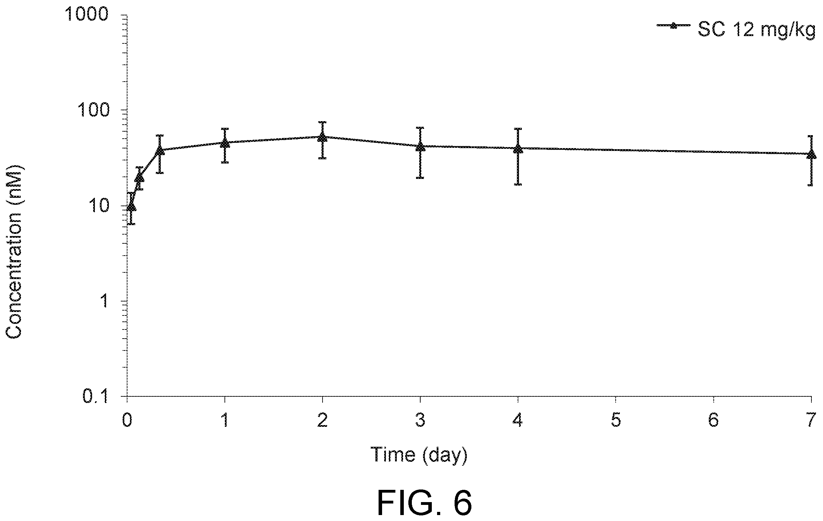

FIG. 6 shows a plot of plasma concentration over time of 300 mg/mL of the compound of Formula **(Ia)** sodium salt in a formulation of 22.6% water/50% PEG 300/1.1% NaOH/26.3% Formula **(Ia)** when dosed subcutaneously in dogs at 12 mg/kg.

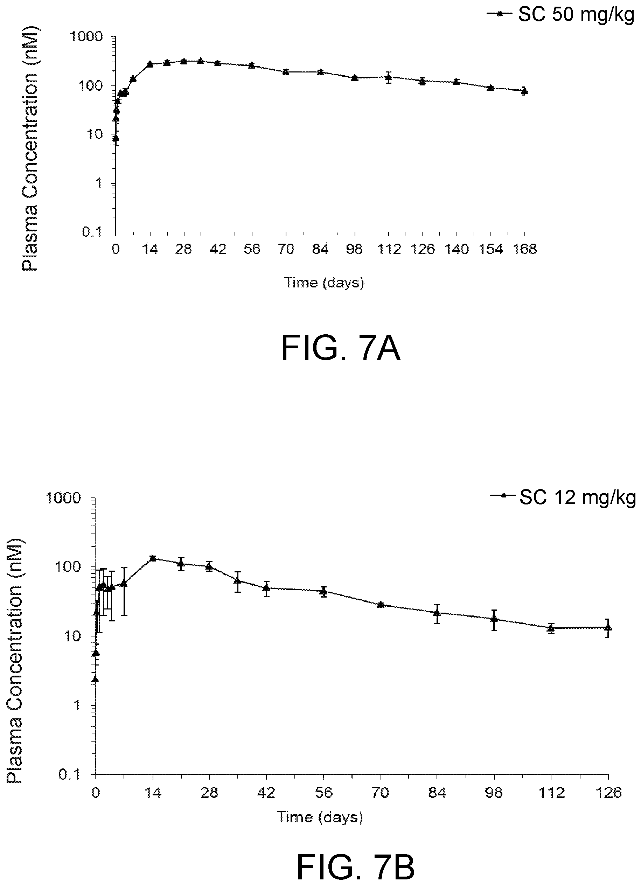

FIGS. 7A and 7B (reference) show plots of the plasma concentration over time of 300 mg/ml of the compound of Formula **(Ia)** in a solution of 9.8 w/w% water, 65.0 w/w% PEG 300, and 25.2 w/w% of a compound of Formula **(Ia)** when dosed subcutaneously in rats at 50 mg/kg (FIG. 7A) and in dogs at 12 mg/kg (FIG. 7B).

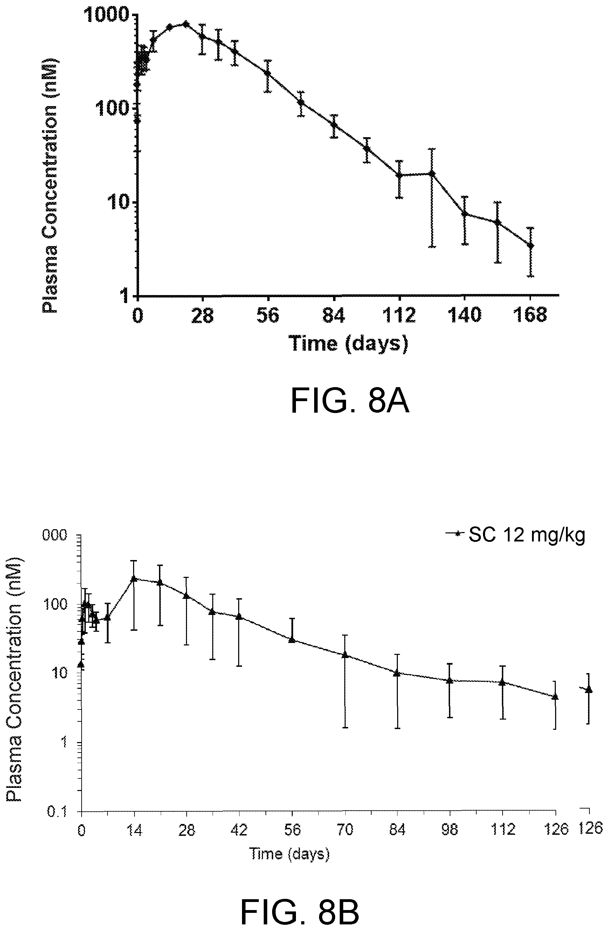

FIGS. 8A and 8B show plots of the plasma concentration over time of 150 mg/ml of the compound of Formula **(Ia)** in a solution of 27.92 w/w% water, 58.04 w/w% PEG 300, 13.47 w/w% of a compound of Formula **(Ia),** and 0.58 w/w% NaOH when dosed subcutaneously in rats at 50 mg/kg (FIG. 8A) and in dogs at 12 mg/kg (FIG. 8B).

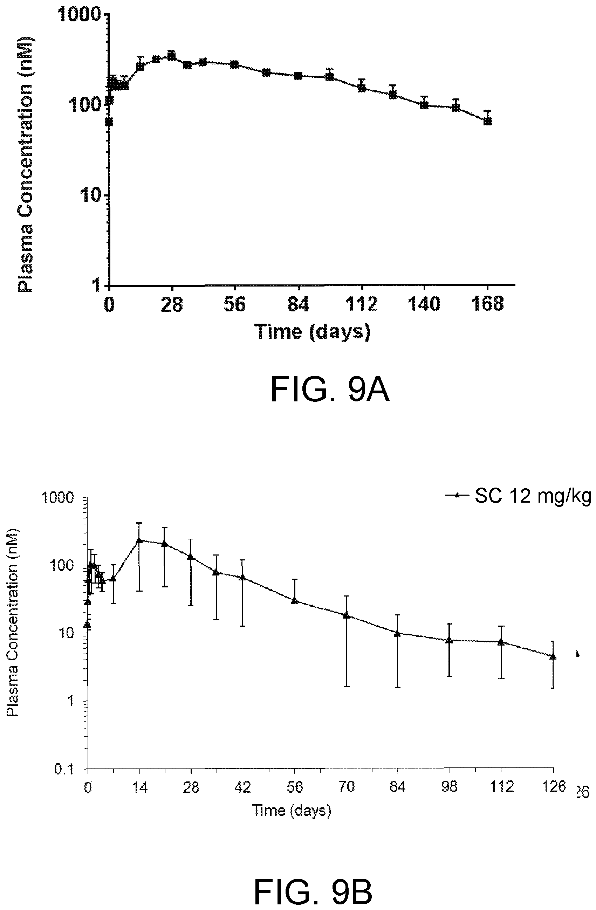

FIGS. 9A and 9B show plots of the plasma concentration over time of 300 mg/ml of a compound of Formula **(Ia)** in a solution of 23.2 w/w% water, 50.0 w/w% PEG 300, 25.7 w/w% of a compound of Formula **(Ia),** and 1.1 w/w% NaOH when dosed subcutaneously in rats at 50 mg/kg (FIG. 9A) and in dogs at 12 mg/kg (FIG. 9B).

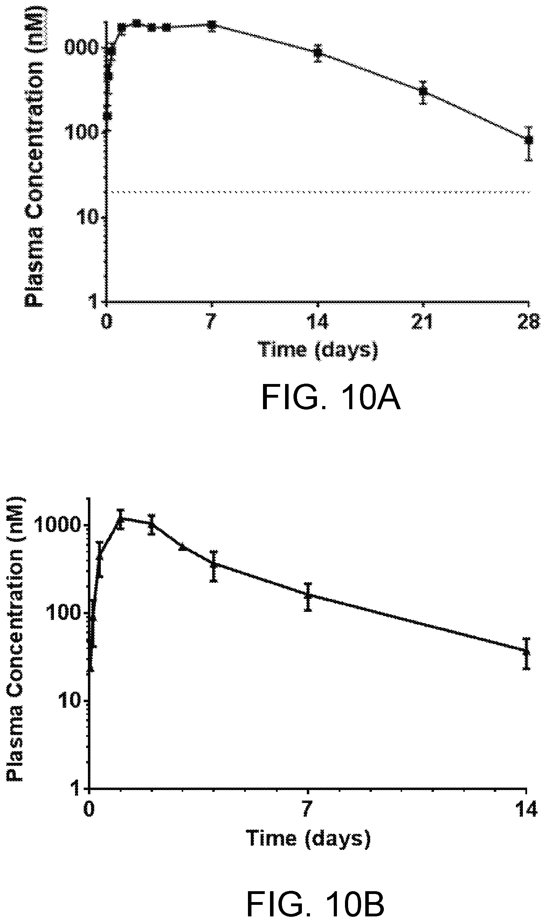

FIGS. 10A and 10B (reference) show plots of the plasma concentration over time of 50 mg/ml of a compound of Formula **(Ia)** in a solution of 30.07 w/w% water, 64.40 w/w% PEG 300, 4.68 w/w% of a sodium salt of the compound of Formula **(Ia),** and 0.85 w/w% poloxamer 188 when dosed subcutaneously in rats at 50 mg/kg (FIG. 10A) and in dogs at 6 mg/kg (FIG. 10B).

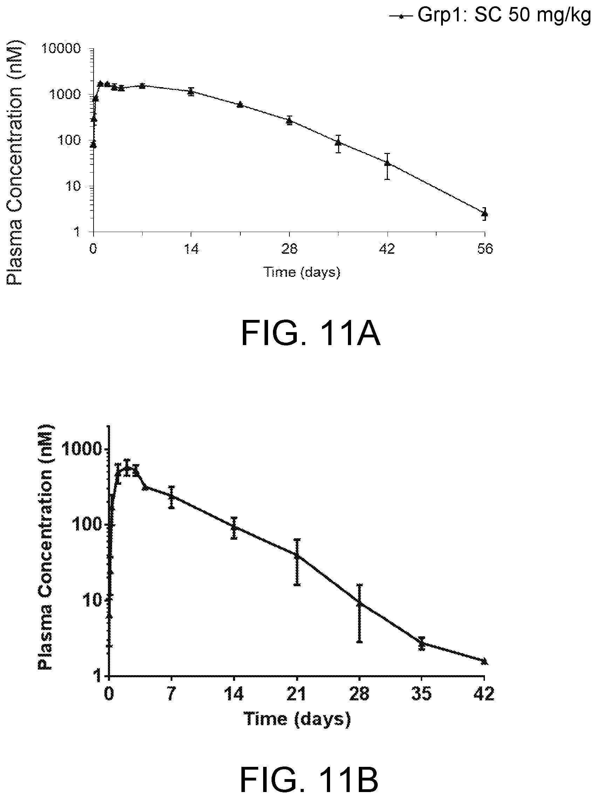

FIGS. 11A and 11B show plots of the plasma concentration over time of 75 mg/ml of a compound of Formula **(Ia)** in a solution of 29.21 w/w% water, 62.55 w/w% PEG 300, 6.97 w/w% of a sodium salt of the compound of Formula **(Ia),** and 1.68 w/w% poloxamer 188 when dosed subcutaneously in rats at 50 mg/kg (FIG. 11A) and in dogs at 6 mg/kg (FIG. 11B).

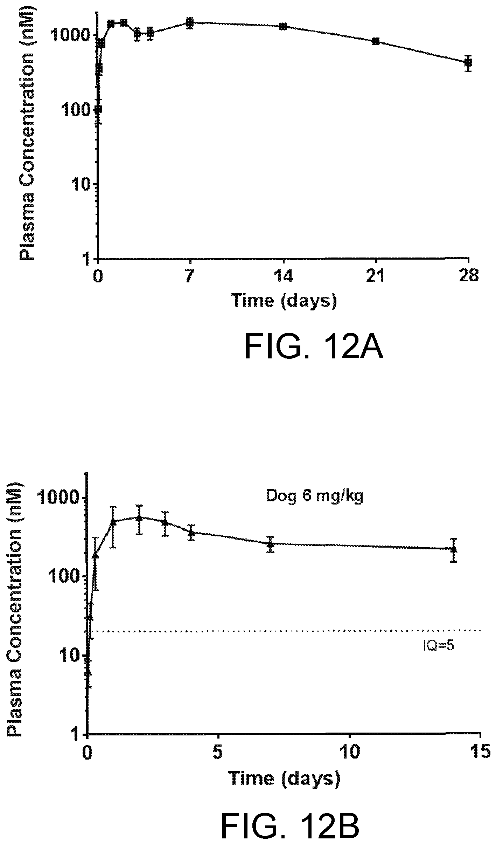

FIGS. 12A and 12B show plots of the plasma concentration over time of 100 mg/ml of a compound of Formula **(Ia)** in a solution of 28.36 w/w% water, 60.73 w/w% PEG 300, 9.23 w/w% of a sodium salt of a compound of Formula **(Ia),** and 1.68 w/w% poloxamer 188 when dosed subcutaneously in rats at 50 mg/kg (FIG. 12A) and in dogs at 6 mg/kg (FIG. 12B).

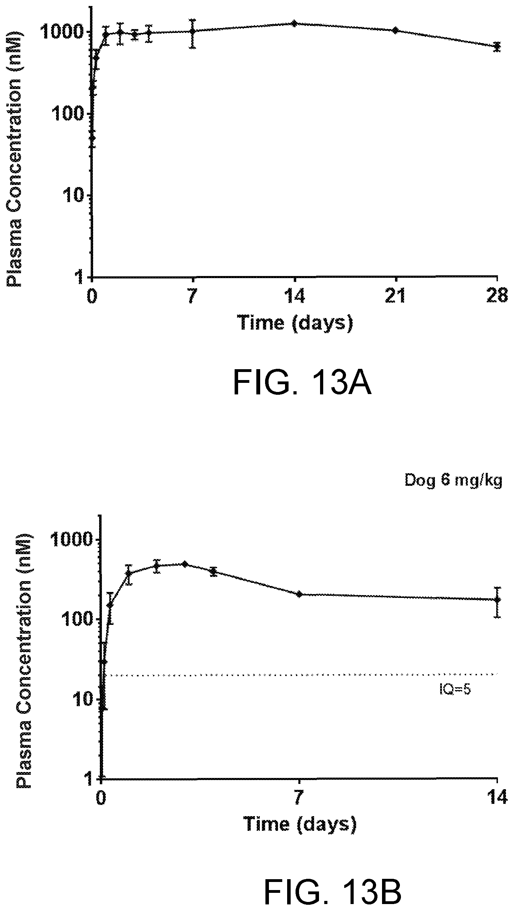

FIGS. 13A and 13B show plots of the plasma concentration over time of 125 mg/ml of a compound of Formula **(Ia)** in a solution of 27.51 w/w% water, 58.92 w/w% PEG 300, 11.48 w/w% of a sodium salt of the compound of Formula **(Ia),** and 2.09 w/w% poloxamer 188 when dosed subcutaneously in rats at 50 mg/kg (FIG. 13A) and in dogs at 6 mg/kg (FIG. 13B).

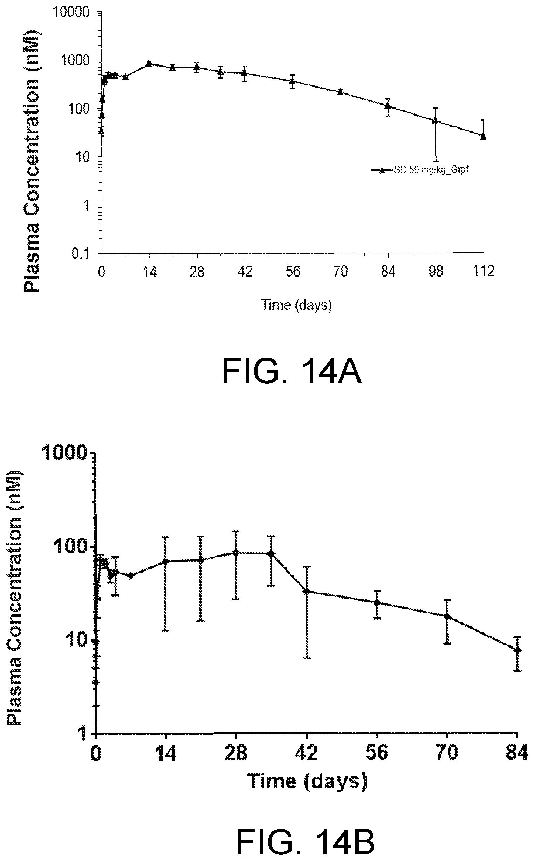

FIG. 14A shows a plot of the plasma concentration over time of 150 mg/ml of the compound of Formula **(Ia)** in a solution of 26.6 w/w% water, 56.97 w/w% PEG 300, 13.39 w/w% of a compound of Formula **(Ia),** 0.57 w/w% NaOH, and 2.49 w/w% poloxamer 188 when dosed subcutaneously in rats at 50 mg/kg. FIG. 14B shows a plot of the plasma concentration over time of 150 mg/ml of the compound of Formula **(Ia)** in a solution of 26.6 w/w% water, 56.97 w/w% PEG 300, 13.39 w/w% of a compound of Formula **(Ia),** 0.57 w/w% NaOH, and 2.49 w/w% poloxamer 188 when dosed subcutaneously in dogs at 6 mg/kg.

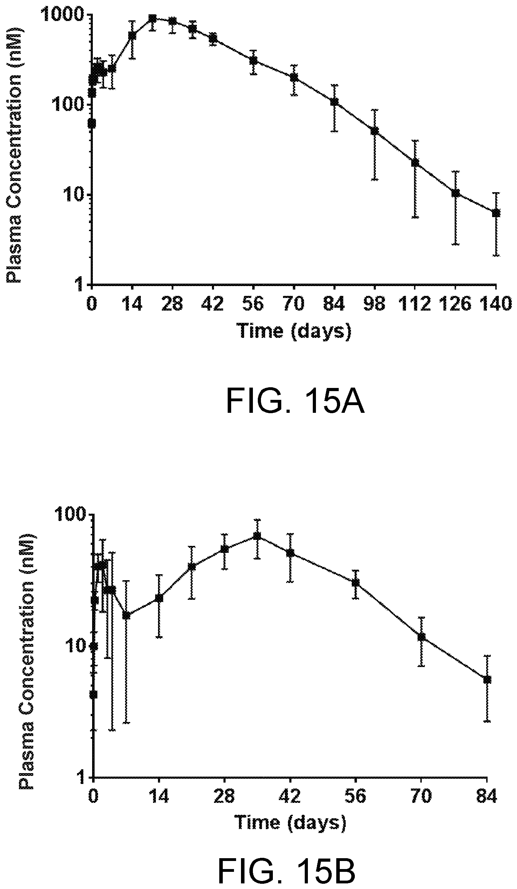

FIGS. 15A and 15B show plots of the plasma concentration over time of 300 mg/ml of the compound of Formula **(Ia)** in a solution of 21.97 w/w% water, 47.05 w/w% PEG 300, 25.21 w/w% of a compound of Formula **(Ia),** 1.08 w/w% of NaOH, and 4.69 w/w% poloxamer 188 when dosed subcutaneously in rats at 50 mg/kg (FIG. 15A) and in dogs at 6 mg/kg (FIG. 15B).

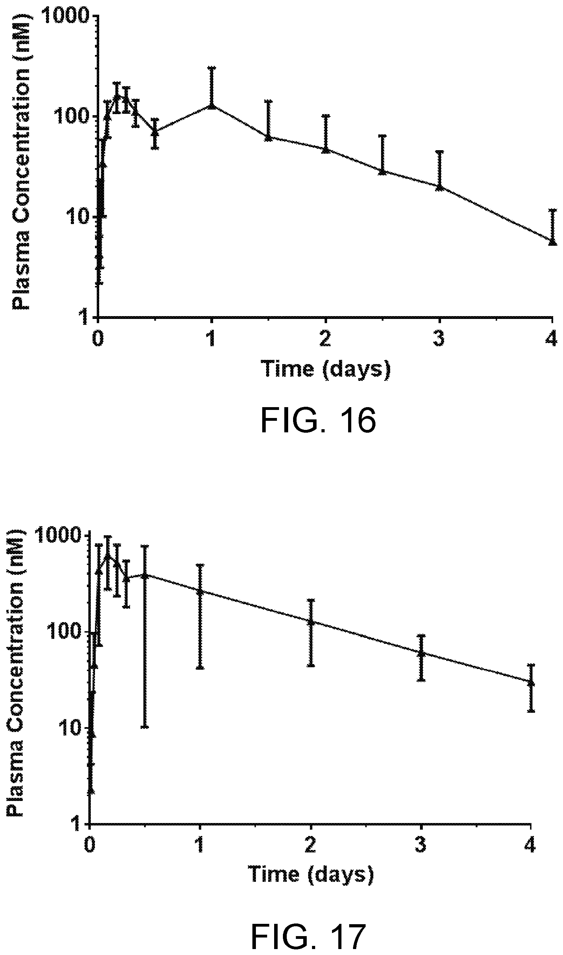

FIG. 16 (reference) shows a plot of the plasma concentration over time of a compound of Formula **(Ia)** in 100% glycerol monocaprylocaprate when orally administered in dogs as liquidfilled capsules at 7.5 mg fixed dose.
FIG. 17 (reference) shows a plot of the plasma concentration over time of a tablet prepared from a spray-dried dispersion of a sodium salt of the compound of Formula **(Ia)** (composition described in Table T with an outer film coating made of Opadry^{®} II White 85F18422, wherein the outer film coating provides a 3% weight gain based on the uncoated tablet) when orally administered in dogs at 300 mg fixed dose.

### DETAILED DESCRIPTION

The description below is made with the understanding that the present disclosure is to be considered as an exemplification of the claimed subject matter, and is not intended to limit the appended claims to the specific embodiments illustrated. The headings used throughout this disclosure are provided for convenience and are not to be construed to limit the claims in any way. Embodiments illustrated under any heading may be combined with embodiments illustrated under any other heading.

Unless defined otherwise, all technical and scientific terms used herein have the same meaning as commonly understood by one of ordinary skill in the art.

When trade names are used herein, it is intended to independently include the tradename product and the active pharmaceutical ingredient(s) of the tradename product.

Reference to "about" a value or parameter herein includes (and describes) embodiments that are directed to that value or parameter *per se.* In certain embodiments, the term "about" includes the indicated amount ± 10%. In other embodiments, the term "about" includes the indicated amount ± 8%. In other embodiments, the term "about" includes the indicated amount ± 5%. In other embodiments, the term "about" includes the indicated amount ± 3%. In certain other embodiments, the term "about" includes the indicated amount ± 1%. Also, the term "about X" includes description of "X".

As used herein and in the appended claims, the singular forms "a" and "an" and "the" include plural referents unless the context clearly dictates otherwise. Thus, for example, reference to "the compound" includes a plurality of such compounds and reference to "the assay" includes reference to one or more assays, and so forth.

As an additional example, reference to "a pharmaceutically acceptable excipient" includes both a single pharmaceutically acceptable excipient and a plurality of pharmaceutically acceptable excipients.

As used herein, "HIV" or "Human Immunodeficiency Virus" refers to HIV-1 and/or to HIV-2.

As used herein, a "heavily treatment-experienced patient" refers to an HIV-infected patient who has limited treatment options due to a multidrug resistant HIV infection. For example, in some embodiments, the "heavily treatment-experienced patient" is a patient with HIV who has developed resistance to an antiretroviral medication from at least one class of antiretroviral medications selected from the group consisting of NRTIs, NNRTIs, PIs, and INSTIs.

In some embodiments, "multidrug resistant HIV infection" means resistance to an antiretroviral medication from at least one class of antiretroviral medications selected from the group consisting of NRTIs, NNRTIs, PIs, and INSTIs. In some embodiments, "multidrug resistant HIV infection" means resistance to at least one antiretroviral medication from two classes of antiretroviral medications selected from the group consisting of NRTIs, NNRTIs, PIs, and INSTIs. In some embodiments, "multidrug resistant HIV infection" means resistance to at least one antiretroviral medication from three classes of antiretroviral medications selected from the group consisting of NRTIs, NNRTIs, PIs, and INSTIs. In some embodiments, "multidrug resistant HIV infection" means resistance to at least one antiretroviral medication from each of the four classes of antiretroviral medications selected from the group consisting of NRTIs, NNRTIs, PIs, and INSTIs.

As used herein, the term "NRTI(s)" refers to nucleoside reverse transcriptase inhibitor(s) or nucleotide reverse transcriptase inhibitor(s).

As used herein, the term "NNRTI(s)" refers to non-nucleoside reverse transcriptase inhibitor(s) or non-nucleotide reverse transcriptase inhibitor(s).

As used herein, the term "PI(s)" refers to protease inhibitor(s).

As used herein, the term "INSTI(s)" refers to integrase strand transfer inhibitor(s).

As used herein, the term "fail" or "failed" when referring to HIV therapy or an HIV treatment regimen means a treatment outcome which precludes the use of the same agent or class in the future in a patient with HIV. This could be due to inadequate initial viral response due to pre-existing viral resistance, viral rebound due to emergent viral resistance, or inability of a patient to continue a treatment due to intolerability or safety issues.

As used herein, the term "Cₘₐₓ" refers to the maximum observed plasma/serum concentration of drug.

"Pharmaceutically acceptable" refers to compounds, salts, compositions, dosage forms and other materials that are useful in preparing a pharmaceutical composition that is suitable for veterinary or human pharmaceutical use.

"Pharmaceutically acceptable excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsifier which has been approved by the United States Food and Drug Administration as being acceptable for use in humans or domestic animals.

"Pharmaceutically acceptable salt" refers to a salt of a compound that is pharmaceutically acceptable and that possesses (or can be converted to a form that possesses) the desired pharmacological activity of the parent compound. Such salts include acid addition salts formed with inorganic acids such as hydrochloric acid, hydrobromic acid, sulfuric acid, nitric acid, phosphoric acid, and the like; or formed with organic acids such as acetic acid, benzenesulfonic acid, benzoic acid, camphorsulfonic acid, citric acid, ethanesulfonic acid, fumaric acid, glucoheptonic acid, gluconic acid, lactic acid, maleic acid, malonic acid, mandelic acid, methanesulfonic acid, 2-naphthalenesulfonic acid, oleic acid, palmitic acid, propionic acid, stearic acid, succinic acid, tartaric acid, p-toluenesulfonic acid, trifluoroacetic acid, trimethylacetic acid, and the like, and salts formed when an acidic proton present in the parent compound is replaced by either a metal ion, for example, an alkali metal ion (for example, a sodium or potassium), an alkaline earth ion (for example, calcium or magnesium), or an aluminum ion; or coordinates with an organic base such as diethanolamine, triethanolamine, N-methylglucamine and the like. Also included in this definition are ammonium and substituted or quaternary ammonium salts (e.g., quaternary ammonium salts containing the N,N,N-trimethylethanolammonium (choline) cation). Representative non-limiting lists of pharmaceutically acceptable salts can be found in S.M. Berge et al., J. Pharma Sci., 66(1), 1-19 (1977), and Remington: The Science and Practice of Pharmacy, R. Hendrickson, ed., 21st edition, Lippincott, Williams & Wilkins, Philadelphia, PA, (2005), at p. 732, Table 38-5.

"Patient" and "patients" refer to humans, domestic animals (for example, dogs and cats), farm animals (for example, cattle, horses, sheep, goats and pigs), laboratory animals (for example, mice, rats, hamsters, guinea pigs, pigs, rabbits, dogs, and monkeys), and the like.

As used herein, "treatment" or "treating" is an approach for obtaining beneficial or desired results. For purposes of the present disclosure, beneficial or desired results include, but are not limited to, alleviation of a symptom and/or diminishment of the extent of a symptom and/or preventing a worsening of a symptom associated with a disease or condition. In one embodiment, "treatment" or "treating" includes one or more of the following: a) inhibiting the disease or condition (for example, decreasing one or more symptoms resulting from the disease or condition, and/or diminishing the extent of the disease or condition); b) slowing or arresting the development of one or more symptoms associated with the disease or condition (for example, stabilizing the disease or condition, delaying the worsening or progression of the disease or condition); and/or c) relieving the disease or condition, for example, causing the regression of clinical symptoms, ameliorating the disease state, delaying the progression of the disease, increasing the quality of life, and/or prolonging survival.

As used herein, the term "therapeutically effective amount" or "effective amount" refers to an amount that is effective to elicit the desired biological or medical response, including the amount of a compound that, when administered to a patient for treating a disease, is sufficient to effect such treatment for the disease or to an amount that is effective to protect against the contracting or onset of a disease. The effective amount will vary depending on the compound, the disease, and its severity and the age, weight, etc., of the patient to be treated. The effective amount can include a range of amounts. As is understood in the art, an effective amount may be in one or more doses, for example, a single dose or multiple doses may be required to achieve the desired treatment outcome. An effective amount may be considered in the context of administering one or more therapeutic agents, and a single agent may be considered to be given in an effective amount if, in conjunction with one or more other agents, a desirable or beneficial result may be or is achieved. Suitable doses of any co-administered compounds may optionally be lowered due to the combined action (for example, additive or synergistic effects) of the compounds.

Except as expressly defined otherwise, the present disclosure includes all tautomers of compounds detailed herein, even if only one tautomer is expressly represented (for example, both tautomeric forms are intended and described by the presentation of one tautomeric form where a pair of two tautomers may exist). For example, if reference is made to a compound containing an amide (for example, by structure or chemical name), it is understood that the corresponding imidic acid tautomer is included by this disclosure and described the same as if the amide were expressly recited either alone or together with the imidic acid. Where more than two tautomers may exist, the present disclosure includes all such tautomers even if only a single tautomeric form is depicted by chemical name and/or structure.

It is understood by one skilled in the art that this disclosure includes the pharmaceutical composition comprising a sodium salt of a compound of Formula **(Ia),** wherein the compound of Formula **(Ia)** can be enriched at any or all atoms above naturally occurring isotopic ratios with one or more isotopes such as, but not limited to, deuterium (²H or D).

Disclosed are pharmaceutical compositions according to the claims comprising compounds in which from 1 to n hydrogen atoms attached to a carbon atom can be replaced by a deuterium atom or D, in which n is the number of hydrogen atoms in the molecule. As known in the art, the deuterium atom is a non-radioactive isotope of the hydrogen atom. Such compounds can increase resistance to metabolism, and thus can be useful for increasing the half-life of the compounds when administered to a mammal. See, for example, Foster, "Deuterium Isotope Effects in Studies of Drug Metabolism," Trends Pharmacol. Sci., 5(12):524-527 (1984). Such compounds are synthesized by means well known in the art, for example, by employing starting materials in which one or more hydrogen atoms have been replaced by deuterium.

Examples of isotopes that can be incorporated into the disclosed compounds also include isotopes of hydrogen, carbon, nitrogen, oxygen, phosphorous, fluorine, chlorine, and iodine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. Substitution with positron emitting isotopes, such as ¹¹C, ¹⁸F, ¹⁵O and ¹³N, can be useful in positron emission tomography (PET) studies for examining substrate receptor occupancy. Isotopically-labeled compounds of Formula **(Ia)** can generally be prepared by conventional techniques known to those skilled in the art or by processes analogous to those described in the Examples as set out below using an appropriate isotopically-labeled reagent in place of the non-labeled reagent previously employed.

Compounds described herein can have chiral centers and/or geometric isomeric centers (E- and Z- isomers), and it is to be understood that all such optical, enantiomeric, diastereoisomeric and geometric isomers are encompassed. Where compounds are represented in their chiral form, it is understood that the embodiment encompasses, but is not limited to, the specific diastereomerically or enantiomerically enriched form. Where chirality is not specified but is present, it is understood that the embodiment is directed to either the specific diastereomerically or enantiomerically enriched form; or a racemic or scalemic mixture of such compound(s).

Also provided are also pharmaceutically acceptable hydrates, solvates, tautomeric forms, polymorphs, and prodrugs of the compounds described herein.

In an embodiment, the current disclosure relates to the use of a pharmaceutical composition according to the claims for use in a method of treating an infection caused by the HIV virus comprising administering a therapeutically effective amount to a patient in need thereof, where the patient is a heavily treatment-experienced patient that has a multidrug resistant HIV infection.

The present disclosure includes a pharmaceutical composition according to the claims, wherein the pharmaceutical composition comprises a sodium salt of the compound of Formula (Ia), N-((S)-1-(3-(4-chloro-3-(methylsulfonamido)-1-(2,2,2-trifluoroethyl)-1H-indazol-7-yl)-6-(3-methyl-3-(methylsulfonyl)but-1-yn-1-yl)pyridin-2-yl)-2-(3,5-difluorophenyl)ethyl)-2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamide, having the following structure: 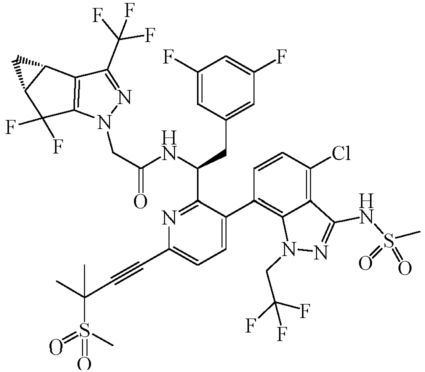 (Ia), for use in methods of treating an HIV infection in heavily treatment-experienced patients with multidrug resistant HIV infection.

The compound of Formula **(Ia)** is active against major HIV-1 mutants selected by protease inhibitors (PIs), nucleoside reverse transcriptase inhibitors (NRTIs), non-nucleoside reverse transcriptase inhibitors (NNRTIs), and/or integrase inhibitors (INSTIs).

### Pharmaceutical Compositions for use in Methods of Treatment

Provided in this disclosure is a pharmaceutical composition according to the claims for use in a method of treating human immunodeficiency virus (HIV) infection in a heavily treatment-experienced patient that includes administering to the patient a therapeutically effective amount of the pharmaceutical composition comprising the sodium salt of the compound of Formula **(Ia):**

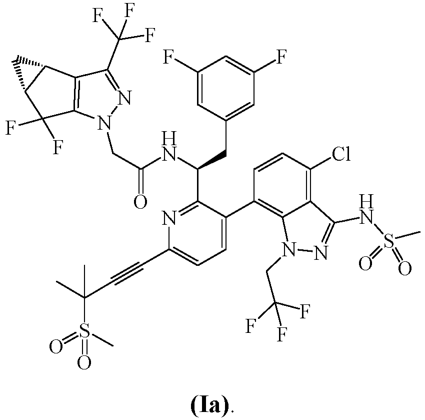

In some embodiments, the heavily treatment-experienced patient is infected with multidrug resistant HIV. In some embodiments, the heavily treatment-experienced patient has a multidrug resistant HIV infection and is on a failing HIV treatment regimen. In some embodiments, the heavily treatment-experienced patient has a viral load greater than about 1,000 copies of HIV RNA/mL.

In some embodiments, the HIV infection is an HIV-1 infection. In some embodiments, the HIV-1 infection is characterized by HIV-1 mutant resistance to an antiretroviral medication, for example, to one, two, three, four, or more classes of antiretroviral medications (e.g., PIs, NRTIs, NNRTIs, INSTIs, etc.). In some embodiments, the HIV-1 infection is characterized by HIV-1 mutant resistance to one or more classes of antiretroviral medications. In some embodiments, the HIV-1 infection is characterized by HIV-1 mutant resistance to two or more classes of antiretroviral medications. In some embodiments, the HIV-1 infection is characterized by HIV-1 mutant resistance to three or more classes of antiretroviral medications.

In some embodiments, the HIV-1 infection is characterized by an HIV-1 mutant that includes, but is not limited to:
(a) an HIV-1 mutant resistant to a PI (e.g., I50V, I84V/L90M, G48V/V82A/L90M, G48V/V82S, etc.);
(b) an HIV-1 mutant resistant to an NRTI (e.g., K65R, M184V, 6TAMs, etc.);
(c) an HIV-1 mutant resistant to an NNRTI (e.g., K103N, Y181C, Y188L, L100I/K103N, K103N/Y181C, etc.); and/or
(d) an HIV-1 mutant resistant to an INSTI (Y143R, E138K/Q148K, G140S/Q148R, E92Q/N155H, N155H/Q148R, R263K/M50I, etc.).

In some embodiments, the patient is infected with HIV-1 that is resistant to at least one antiretroviral medication. In some embodiments, the patient is infected with multidrug resistant HIV-1. In some embodiments, the patient is infected with multidrug resistant HIV-1 that is resistant to at least one, two, three, four, or more antiretroviral medications. In some embodiments, the patient is infected with multidrug resistant HIV-1 that is resistant to at least one antiretroviral medication from each of two different classes of antiretroviral medications. In some embodiments, the patient is infected with multidrug resistant HIV-1 that is resistant to at least one antiretroviral medication from each of three different classes of antiretroviral medications. In some embodiments, the different classes of antiretroviral medications are selected from a nucleoside reverse transcriptase inhibitor (NRTI), a non-nucleoside reverse transcriptase inhibitor (NNRTI), a protease inhibitor (PI), and an integrase strand transfer inhibitor (INSTI). In some embodiments, the different classes of antiretroviral medications are selected from an NRTI, an NNRTI, and a PI. In some embodiments, the patient is infected with multidrug resistant HIV-1 that is resistant to at least one NRTI and at least one NNRTI. In some embodiments, the patient is infected with multidrug resistant HIV-1 that is resistant to at least one NRTI and at least one PI. In some embodiments, the patient is infected with multidrug resistant HIV-1 that is resistant to at least one NRTI and at least one INSTI. In some embodiments, the patient is infected with multidrug resistant HIV-1 that is resistant to at least one NNRTI and at least one PI. In some embodiments, the patient is infected with multidrug resistant HIV-1 that is resistant to at least one NNRTI and at least one INSTI. In some embodiments, the patient is infected with multidrug resistant HIV-1 that is resistant to at least one PI and at least one INSTI. In some embodiments, the patient is infected with multidrug resistant HIV-1 that is resistant to at least one NRTI, at least one NNRTI, and at least one PI. In some embodiments, the patient is infected with multidrug resistant HIV-1 that is resistant to at least one NRTI, at least one NNRTI, and at least one INSTI. In some embodiments, the patient is infected with multidrug resistant HIV-1 that is resistant to at least one NRTI, at least one PI, and at least one INSTI. In some embodiments, the patient is infected with multidrug resistant HIV-1 that is resistant to at least one NNRTI, at least one PI, and at least one INSTI. In some embodiments, the patient is infected with multidrug resistant HIV-1 that is resistant to at least one NRTI, at least one NNRTI, at least one PI, and at least one INSTI.

In some embodiments, the patient is infected with multidrug resistant HIV-1 that is resistant to at least one antiretroviral medication that is an NRTI. Examples of NRTIs include, but are not limited to, emtricitabine (FTC; Emtriva^{®}), lamivudine (3TC; Epivir^{®}), zidovudine (azidothymidine (AZT); Retrovir^{®}), didanosine (ddI; Videx-EC^{®}), dideoxyinosine (Videx^{®}), tenofovir, tenofovir alafenamide (Vemlidy^{®}), tenofovir disoproxil fumarate (Viread^{®}), stavudine (d4T; Zerit^{®}), zalcitabine (dideoxycytidine, ddC; Hivid^{®}), and abacavir (Ziagen^{®}).

In some embodiments, the patient is infected with multidrug resistant HIV-1 that is resistant to at least one antiretroviral medication that is an NNRTI. Examples of NNRTIs include, but are not limited to, efavirenz (Sustiva^{®}), etravirine (Intelence^{®}), rilpivirine (Edurant^{®}), nevirapine (Viramune^{®}), and delavirdine (Rescriptor^{®}).

In some embodiments, the patient is infected with multidrug resistant HIV-1 that is resistant to at least one antiretroviral medication that is a PI. Examples of PIs include, but are not limited to, amprenavir (Agenerase^{®}), atazanavir (Reyataz^{®}), darunavir (Prezista^{®}), fosamprenavir (Telzir^{®}, Lexiva^{®}), indinavir (Crixivan^{®}), lopinavir (Kaletra^{®}), nelfinavir (Viracept^{®}), ritonavir (Norvir^{®}), saquinavir (Invirase^{®}), and tipranavir (Aptivus^{®}).

In some embodiments, the patient is infected with multidrug resistant HIV-1 that is resistant to at least one antiretroviral medication that is an INSTI. Examples of INSTIs include, but are not limited to, raltegravir (Isentress^{®}), elvitegravir (Vitekta^{®}), dolutegravir (Tivicay^{®}), cabotegravir, and bictegravir.

In some embodiments, the patient is infected with multidrug resistant HIV-1 that is resistant to at least one antiretroviral medication that is a gp41 fusion inhibitor. Examples of gp41 fusion inhibitors include, but are not limited to, albuvirtide, enfuvirtide, BMS-986197, enfuvirtide biobetter, enfuvirtide biosimilar, HIV-1 fusion inhibitors (P26-Bapc), ITV-1, ITV-2, ITV-3, ITV-4, PIE-12 trimer and sifuvirtide.

In some embodiments, the patient is infected with multidrug resistant HIV-1 that is resistant to at least one antiretroviral medication that is a CCR5 co-receptor antagonist. Examples of CCR5 co-receptor antagonists include, but are not limited to, aplaviroc, vicriviroc, maraviroc, cenicriviroc, PRO-140, adaptavir (RAP-101), nifeviroc (TD-0232), anti-GP120/CD4 or CCR5 bispecific antibodies, B-07, MB-66, polypeptide C25P, TD-0680, and vMIP (Haimipu).

In some embodiments, the patient has been previously treated with at least one antiretroviral medication before being treated with a pharmaceutical composition comprising the sodium salt of the compound of Formula **(Ia).** In some embodiments, the patient has been previously treated with at least one antiretroviral medication for at least 3 months, such as at least 4 months, at least 5 months, at least 6 months, at least 7 months, at least 8 months, at least 9 months, at least 10 months, at least 11 months, at least 12 months, at least 18 months, or at least 24 months. In some embodiments, the patient has been previously treated with at least one antiretroviral medication for at least 3 months. In some embodiments, the patient has been previously treated with at least one antiretroviral medication for at least 6 months. In some embodiments, the patient has been previously treated with at least one antiretroviral medication for at least 9 months. In some embodiments, the patient has been previously treated with at least one antiretroviral medication for at least 12 months. In some embodiments, the patient has been previously treated with at least one antiretroviral medication for at least 18 months. In some embodiments, the patient has been previously treated with at least one antiretroviral medication for at least 24 months. In some embodiments, the patient has been previously treated with at least one antiretroviral medication for at least 30 months. In some embodiments, the patient has been previously treated with at least one antiretroviral medication for at least 36 months.

In some embodiments, the patient has failed or is failing an HIV treatment regimen before being treated with a pharmaceutical composition comprising the sodium salt of the compound of Formula **(Ia).** In some embodiments, the prior HIV treatment regimen included administering at least one antiretroviral medication. In some embodiments, the patient infected with HIV has relapsed after an initial response to the prior HIV treatment regimen, for example, antiretroviral therapy. In some embodiments, the patient has a viral load of greater than about 50 copies of HIV RNA/mL after about 48 weeks of therapy, for example, antiretroviral therapy, before being treated with a pharmaceutical composition comprising the sodium salt of the compound of Formula **(Ia).**

In some embodiments, the prior treatment regimen includes administering at least one antiretroviral medication from each of two different classes of antiretroviral medications. In some embodiments, the prior treatment regimen includes administering at least one antiretroviral medication from each of three different classes of antiretroviral medications. In some embodiments, the different classes of antiretroviral medications are selected from a nucleoside reverse transcriptase inhibitor (NRTI), a non-nucleoside reverse transcriptase inhibitor (NNRTI), a protease inhibitor (PI), and an integrase strand transfer inhibitor (INSTI). In some embodiments, the different classes of antiretroviral medications are selected from an NRTI, an NNRTI, and a PI. In some embodiments, the prior treatment regimen includes administering at least one NRTI and at least one NNRTI. In some embodiments, the prior treatment regimen includes administering at least one NRTI and at least one PI. In some embodiments, the prior treatment regimen includes administering at least one NRTI and at least one INSTI. In some embodiments, the prior treatment regimen includes administering at least one NNRTI and at least one PI. In some embodiments, the prior treatment regimen includes administering at least one NNRTI and at least one INSTI. In some embodiments, the prior treatment regimen includes administering at least one PI and at least one INSTI. In some embodiments, the prior treatment regimen includes administering at least one NRTI, at least one NNRTI, and at least one PI. In some embodiments, the prior treatment regimen includes administering at least one NRTI, at least one NNRTI, and at least one INSTI. In some embodiments, the prior treatment regimen includes administering at least one NRTI, at least one PI, and at least one INSTI. In some embodiments, the prior treatment regimen includes administering at least one NNRTI, at least one PI, and at least one INSTI.

In some embodiments, the prior treatment regimen includes administering at least one antiretroviral medication that is a gp41 fusion inhibitor.

In some embodiments, the prior treatment regimen includes administering at least one antiretroviral medication that is a CCR5 co-receptor antagonist.

In some embodiments, the prior treatment regimen includes administering at least one antiretroviral medication that is an NRTI. Examples of NRTIs include, but are not limited to, emtricitabine (FTC; Emtriva^{®}), lamivudine (3TC; Epivir^{®}), zidovudine (azidothymidine (AZT); Retrovir^{®}), didanosine (ddI; Videx-EC^{®}), dideoxyinosine (Videx^{®}), tenofovir, tenofovir alafenamide (Vemlidy^{®}), tenofovir disoproxil fumarate (Viread^{®}), stavudine (d4T; Zerit^{®}), zalcitabine (dideoxycytidine, ddC; Hivid^{®}), and abacavir (Ziagen^{®}).

In some embodiments, the prior treatment regimen includes administering at least one antiretroviral medication that is an NNRTI. Examples of NNRTIs include, but are not limited to, efavirenz (Sustiva^{®}), etravirine (Intelence^{®}), rilpivirine (Edurant^{®}), nevirapine (Viramune^{®}), and delavirdine (Rescriptor^{®}).

In some embodiments, the prior treatment regimen includes administering at least one antiretroviral medication that is a PI. Examples of PIs include, but are not limited to, amprenavir (Agenerase^{®}), atazanavir (Reyataz^{®}), darunavir (Prezista^{®}), fosamprenavir (Telzir^{®}, Lexiva^{®}), indinavir (Crixivan^{®}), lopinavir (Kaletra^{®}), nelfinavir (Viracept^{®}), ritonavir (Norvir^{®}), saquinavir (Invirase^{®}), and tipranavir (Aptivus^{®}).

In some embodiments, the prior treatment regimen includes administering at least one antiretroviral medication that is an INSTI. Examples of INSTIs include, but are not limited to, raltegravir (Isentress^{®}), elvitegravir (Vitekta^{®}), dolutegravir (Tivicay^{®}), cabotegravir, and bictegravir.

In some embodiments, the prior treatment regimen includes administering at least one antiretroviral medication that is a gp41 fusion inhibitor. Examples of gp41 fusion inhibitors include, but are not limited to, albuvirtide, enfuvirtide, BMS-986197, enfuvirtide biobetter, enfuvirtide biosimilar, HIV-1 fusion inhibitors (P26-Bapc), ITV-1, ITV-2, ITV-3, ITV-4, PIE-12 trimer and sifuvirtide.

In some embodiments, the prior treatment regimen includes administering at least one antiretroviral medication that is a CCR5 co-receptor antagonist. Examples of CCR5 co-receptor antagonists include, but are not limited to, aplaviroc, vicriviroc, maraviroc, cenicriviroc, PRO-140, adaptavir (RAP-101), nifeviroc (TD-0232), anti-GP120/CD4 or CCR5 bispecific antibodies, B-07, MB-66, polypeptide C25P, TD-0680, and vMIP (Haimipu).

In some embodiments, the heavily treatment-experienced patient infected with HIV has a viral load of about 200 copies of HIV-1 RNA/mL (c/mL) to about 1,000,000 c/mL at the time of beginning administration of the pharmaceutical composition comprising the sodium salt of the compound of Formula **(Ia)** such as a viral load of about 200 c/mL to about 500,000 c/mL, about 200 c/mL to about 250,000 c/mL, about 200 c/mL to about 100,000 c/mL, about 200 c/mL to about 50,000 c/mL, about 200 c/mL to about 25,000 c/mL, about 200 c/mL to about 10,000 c/mL, about 200 c/mL to about 5,000 c/mL, about 200 c/mL to about 3,000 c/mL, about 200 c/mL to about 2,000 c/mL, about 200 c/mL to about 1,000 c/mL, about 200 c/mL to about 750 c/mL, about 200 c/mL to about 500 c/mL, about 500 c/mL to about 1,000,000 c/mL, about 500 c/mL to about 500,000 c/mL, about 500 c/mL to about 250,000 c/mL, about 500 c/mL to about 100,000 c/mL, about 500 c/mL to about 50,000 c/mL, about 500 c/mL to about 25,000 c/mL, about 500 c/mL to about 10,000 c/mL, about 500 c/mL to about 5,000 c/mL, about 500 c/mL to about 3,000 c/mL, about 500 c/mL to about 2,000 c/mL, about 500 c/mL to about 1,000 c/mL, about 500 c/mL to about 750 c/mL, about 750 c/mL to about 1,000,000 c/mL, about 750 c/mL to about 500,000 c/mL, about 750 c/mL to about 250,000 c/mL, about 750 c/mL to about 100,000 c/mL, about 750 c/mL to about 50,000 c/mL, about 750 c/mL to about 25,000 c/mL, about 750 c/mL to about 10,000 c/mL, about 750 c/mL to about 5,000 c/mL, about 750 c/mL to about 3,000 c/mL, about 750 c/mL to about 2,000 c/mL, about 750 c/mL to about 1,000 c/mL, about 1,000 c/mL to about 1,000,000 c/mL, about 1,000 c/mL to about 500,000 c/mL, about 1,000 c/mL to about 250,000 c/mL, about 1,000 c/mL to about 100,000 c/mL, about 1,000 c/mL to about 50,000 c/mL, about 1,000 c/mL to about 25,000 c/mL, about 1,000 c/mL to about 10,000 c/mL, about 1,000 c/mL to about 5,000 c/mL, about 1,000 c/mL to about 3,000 c/mL, about 1,000 c/mL to about 2,000 c/mL, about 2,000 c/mL to about 1,000,000 c/mL, about 2,000 c/mL to about 500,000 c/mL, about 2,000 c/mL to about 250,000 c/mL, about 2,000 c/mL to about 100,000 c/mL, about 2,000 c/mL to about 50,000 c/mL, about 2,000 c/mL to about 25,000 c/mL, about 2,000 c/mL to about 10,000 c/mL, about 2,000 c/mL to about 5,000 c/mL, about 2,000 c/mL to about 3,000 c/mL, about 3,000 c/mL to about 1,000,000 c/mL, about 3,000 c/mL to about 500,000 c/mL, about 3,000 c/mL to about 250,000 c/mL, about 3,000 c/mL to about 100,000 c/mL, about 3,000 c/mL to about 50,000 c/mL, about 3,000 c/mL to about 25,000 c/mL, about 3,000 c/mL to about 10,000 c/mL, about 3,000 c/mL to about 5,000 c/mL, about 5,000 c/mL to about 1,000,000 c/mL, about 5,000 c/mL to about 500,000 c/mL, about 5,000 c/mL to about 250,000 c/mL, about 5,000 c/mL to about 100,000 c/mL, about 5,000 c/mL to about 50,000 c/mL, about 5,000 c/mL to about 25,000 c/mL, about 5,000 c/mL to about 10,000 c/mL, about 10,000 c/mL to about 1,000,000 c/mL, about 10,000 c/mL to about 500,000 c/mL, about 10,000 c/mL to about 250,000 c/mL, about 10,000 c/mL to about 100,000 c/mL, about 10,000 c/mL to about 50,000 c/mL, about 10,000 c/mL to about 25,000 c/mL, about 25,000 c/mL to about 1,000,000 c/mL, about 25,000 c/mL to about 500,000 c/mL, about 25,000 c/mL to about 250,000 c/mL, about 25,000 c/mL to about 100,000 c/mL, about 25,000 c/mL to about 50,000 c/mL, about 50,000 c/mL to about 1,000,000 c/mL, about 50,000 c/mL to about 500,000 c/mL, about 50,000 c/mL to about 250,000 c/mL, about 50,000 c/mL to about 100,000 c/mL, about 100,000 c/mL to about 1,000,000 c/mL, about 100,000 c/mL to about 500,000 c/mL, about 100,000 c/mL to about 250,000 c/mL, about 250,000 c/mL to about 1,000,000 c/mL, about 250,000 c/mL to about 500,000 c/mL, or about 500,000 c/mL to about 1,000,000 c/mL.

In some embodiments, the patient has a viral load of greater than about 200 copies of HIV-1 RNA/mL (c/mL) at the time of beginning administration of the pharmaceutical composition comprising the sodium salt of the compound of Formula **(Ia)** such as a viral load greater than about 500 c/mL, about 750 c/mL, about 1,000 c/mL, about 2,000 c/mL, about 3,000 c/mL, about 5,000 c/mL, about 10,000 c/mL, about 25,000 c/mL, about 50,000 c/mL, about 100,000 c/mL, about 250,000 c/mL, about 500,000 c/mL, or greater than about 1,000,000 c/mL at the time of beginning administration of the pharmaceutical composition comprising the sodium salt of the compound of Formula **(Ia).** In some embodiments, the patient has a viral load of greater than about 200 c/mL at the time of beginning administration of the pharmaceutical composition comprising the sodium salt of the compound of Formula **(Ia).** In some embodiments, the patient has a viral load of greater than about 500 c/mL at the time of beginning administration of the pharmaceutical composition comprising the sodium salt of the compound of Formula **(Ia).** In some embodiments, the patient has a viral load of greater than about 750 c/mL at the time of beginning administration of the pharmaceutical composition comprising the sodium salt of the compound of Formula **(Ia).** In some embodiments, the patient has a viral load of greater than about 1,000 c/mL at the time of beginning administration of the pharmaceutical composition comprising the sodium salt of the compound of Formula **(Ia).** In some embodiments, the patient has a viral load of greater than about 2,000 c/mL at the time of beginning administration of the pharmaceutical composition comprising the sodium salt of the compound of Formula **(Ia).**

The pharmaceutical composition comprising the sodium salt of the compound of Formula **(Ia)** can be administered to a patient in accordance with an effective dosing regimen for a desired period of time or duration, such as at least about one day, at least about one week, at least about one month, at least about 2 months, at least about 3 months, at least about 4 months, at least about 6 months, or at least about 12 months, or longer. In some embodiments, the pharmaceutical composition comprising the sodium salt of the compound of Formula **(Ia)** is administered on a daily or intermittent schedule. In some embodiments, the pharmaceutical composition comprising the sodium salt of the compound of Formula **(Ia)** is administered weekly. In some embodiments, the pharmaceutical composition comprising the sodium salt of the compound of Formula **(Ia)** is administered bi-monthly (i.e., every two weeks). In some embodiments, the pharmaceutical composition comprising the sodium salt of the compound of Formula **(Ia)** is administered monthly. In some embodiments, the pharmaceutical composition comprising the sodium salt of the compound of Formula **(Ia)** is administered every two months. In some embodiments, the pharmaceutical composition comprising the sodium salt of the compound of Formula **(Ia)** is administered every three months. In some embodiments, the pharmaceutical composition comprising the sodium salt of the compound of Formula **(Ia)** is administered every four months. In some embodiments, the pharmaceutical composition comprising the sodium salt of the compound of Formula **(Ia)** is administered every five months. In some embodiments, the pharmaceutical composition comprising the sodium salt of the compound of Formula **(Ia)** is administered every 6 months. In some embodiments, the pharmaceutical composition comprising the sodium salt of the compound of Formula **(Ia)** is administered every five months. In some embodiments, the pharmaceutical composition comprising the sodium salt of the compound of Formula **(Ia)** is administered every 12 months.

In some embodiments, the pharmaceutical composition comprising the sodium salt of the compound of Formula **(Ia)** is administered every nine months.

In some embodiments, administration of the pharmaceutical composition comprising the sodium salt of the compound of Formula **(Ia)** results in a decrease in the viral load in the patient. In some embodiments, the viral load is decreased by about 0.5 log₁₀ to about 2.5 log₁₀ after administration of the pharmaceutical composition comprising the sodium salt of the compound of Formula **(Ia)** for a certain amount of time as compared to the viral load at the time of beginning administration of the pharmaceutical composition comprising the sodium salt of the compound of Formula **(Ia).** For example, the viral load is decreased by about 0.5 log₁₀, about 1 log₁₀, about 1.5 log₁₀, about 2 log₁₀, or about 2.5 log₁₀ after administration of the compound of pharmaceutical composition comprising the sodium salt of the Formula **(Ia)** for a certain amount of time as compared to the viral load at the time of beginning administration of the pharmaceutical composition comprising the sodium salt of the compound of Formula **(Ia).** In some embodiments, the viral load is decreased by about 0.5 log₁₀ after administration of the pharmaceutical composition comprising the sodium salt of the compound of Formula **(Ia)** for about 24 weeks as compared to the viral load at the time of beginning administration of the pharmaceutical composition comprising the sodium salt of the compound of Formula **(Ia).** In some embodiments, the viral load is decreased by about 1 log₁₀ after administration of the pharmaceutical composition comprising the sodium salt of the compound of Formula **(Ia)** for about 24 weeks as compared to the viral load at the time of beginning administration of the pharmaceutical composition comprising the sodium salt of the compound of Formula **(Ia).** In some embodiments, the viral load is decreased by about 1.5 log₁₀ after administration of the pharmaceutical composition comprising the sodium salt of the compound of Formula **(Ia)** for about 24 weeks as compared to the viral load at the time of beginning administration of the pharmaceutical composition comprising the sodium salt of the compound of Formula **(Ia).** In some embodiments, the viral load is decreased by about 2 log₁₀ after administration of the pharmaceutical composition comprising the sodium salt of the compound of Formula **(Ia)** for about 24 weeks as compared to the viral load at the time of beginning administration of the pharmaceutical composition comprising the sodium salt of the compound of Formula **(Ia).** In some embodiments, the viral load is decreased by about 2.5 log₁₀ after administration of the pharmaceutical composition comprising the sodium salt of the compound of Formula **(Ia)** for about 24 weeks as compared to the viral load at the time of beginning administration of the pharmaceutical composition comprising the sodium salt of the compound of Formula **(Ia).**

In some embodiments, the viral load in the patient is about 200 c/mL or less after administration of the pharmaceutical composition comprising the sodium salt of the compound of Formula **(Ia)** for a certain amount of time, such as about 175 c/mL or less, about 150 c/mL or less, about 125 c/mL or less, about 100 c/mL or less, about 75 c/mL or less, or about 50 c/mL or less. In some embodiments, the viral load in the patient is about 200 c/mL or less after administration of the pharmaceutical composition comprising the sodium salt of the compound of Formula **(Ia)** for about 24 weeks. In some embodiments, the viral load in the patient is about 200 c/mL or less after administration of the pharmaceutical composition comprising the sodium salt of the compound of Formula **(Ia)** for about 24 weeks. In some embodiments, the viral load in the patient is about 100 c/mL or less after administration of the pharmaceutical composition comprising the sodium salt of the compound of Formula **(Ia)** for about 24 weeks. In some embodiments, the viral load in the patient is about 50 c/mL or less after administration of the pharmaceutical composition comprising the sodium salt of the compound of Formula **(Ia)** for about 24 weeks.

In certain embodiments, the heavily treatment-experienced patient is concurrently being treated with at least one additional antiretroviral medication. In some embodiments, the antiretroviral medication is selected from an NRTI, an NNRTI, a PI, an INSTI, a gp41 fusion inhibitor, and a CCR5 co-receptor antagonist.

In some embodiments, the patient is concurrently being treated with at least one NRTI. Examples of NRTIs include, but are not limited to, emtricitabine (FTC; Emtriva^{®}), lamivudine (3TC; Epivir^{®}), zidovudine (azidothymidine (AZT); Retrovir^{®}), didanosine (ddI; Videx-EC^{®}), dideoxyinosine (Videx^{®}), tenofovir, tenofovir alafenamide (Vemlidy^{®}), tenofovir disoproxil fumarate (Viread^{®}), stavudine (d4T; Zerit^{®}), zalcitabine (dideoxycytidine, ddC; Hivid^{®}), and abacavir (Ziagen^{®}).

In some embodiments, the patient is concurrently being treated with at least one NNRTI. Examples of NNRTIs include, but are not limited to, efavirenz (Sustiva^{®}), etravirine (Intelence^{®}), rilpivirine (Edurant^{®}), nevirapine (Viramune^{®}), and delavirdine (Rescriptor^{®}).

In some embodiments, the patient is concurrently being treated with at least one PI. Examples of PIs include, but are not limited to, amprenavir (Agenerase^{®}), atazanavir (Reyataz^{®}), darunavir (Prezista^{®}), fosamprenavir (Telzir^{®}, Lexiva^{®}), indinavir (Crixivan^{®}), lopinavir (Kaletra^{®}), nelfinavir (Viracept^{®}), ritonavir (Norvir^{®}), saquinavir (Invirase^{®}), and tipranavir (Aptivus^{®}).

In some embodiments, the patient is concurrently being treated with at least one INSTI. Examples of INSTIs include, but are not limited to, raltegravir (Isentress^{®}), elvitegravir (Vitekta^{®}), dolutegravir (Tivicay^{®}), cabortegravir, and bictegravir.

In some embodiments, the patient is concurrently being treated with at least one gp41 fusion inhibitor. Examples of gp41 fusion inhibitors include, but are not limited to, albuvirtide, enfuvirtide, BMS-986197, enfuvirtide biobetter, enfuvirtide biosimilar, HIV-1 fusion inhibitors (P26-Bapc), ITV-1, ITV-2, ITV-3, ITV-4, PIE-12 trimer, and sifuvirtide.

In some embodiments, the patient is concurrently being treated with at least one CCR5 co-receptor antagonist. Examples of CCR5 co-receptor antagonists include, but are not limited to, aplaviroc, vicriviroc, maraviroc, cenicriviroc, PRO-140, adaptavir (RAP-101), nifeviroc (TD-0232), anti-GP120/CD4 or CCR5 bispecific antibodies, B-07, MB-66, polypeptide C25P, TD-0680, and vMIP (Haimipu).

Also provided in this disclosure is a pharmaceutical composition according to the claims for use in a method of treating an HIV-1 infection in a heavily treatment-experienced patient with multidrug resistant HIV-1 that includes administering a therapeutically effective amount of the pharmaceutical composition comprising the sodium salt of the compound of Formula **(Ia)** to a patient that had been previously treated with an HIV treatment regimen that includes the administration of at least one antiretroviral medication and had failed the treatment regimen. In some embodiments, the HIV treatment regimen includes administration of at least one antiretroviral medication such as those described herein. In some embodiments, administration of the pharmaceutical composition comprising the sodium salt of the compound of Formula **(Ia)** results in a reduction in HIV viral load in the patient.

Also disclosed is a pharmaceutical composition according to the claims for use in a method of treating an HIV-1 infection in a heavily treatment-experienced patient with multidrug resistant HIV-1 that includes administering a therapeutically effective amount of the pharmaceutical composition comprising the sodium salt of the compound of Formula **(Ia)** to a patient that had been previously treated with an HIV treatment regimen that includes the administration of at least one antiretroviral medication and had failed the treatment regimen, where the multidrug resistant HIV-1 is resistant to at least one antiretroviral medication from each of two different classes of antiretroviral medications. In some embodiments, the different classes of antiretroviral medications are selected from an NRTI, an NNRTI, a PI, and an INSTI. In some embodiments, the patient has a viral load of greater than about 200 c/mL at the time of beginning administration of the pharmaceutical composition comprising the sodium salt of the compound of Formula **(Ia)** and administration of the compound results in a reduction in HIV viral load in the patient.

In some embodiments, disclosed is a pharmaceutical composition according to the claims for use in a method of treating an HIV-1 infection in a heavily treatment-experienced patient with multidrug resistant HIV-1 that includes administering a therapeutically effective amount of the pharmaceutical composition comprising the sodium salt of the compound of Formula **(Ia)** to a patient that had been previously treated with an HIV treatment regimen that includes the administration of at least one antiretroviral medication, and had failed the treatment regimen, where the multidrug resistant HIV-1 is resistant to at least one antiretroviral medication from each of three different classes of antiretroviral medications. In some embodiments, the different classes of antiretroviral medications are selected from an NRTI, an NNRTI, a PI, and an INSTI. In some embodiments, the patient has a viral load of greater than about 200 c/mL at the time of beginning administration of the pharmaceutical composition comprising the sodium salt of the compound of Formula **(Ia)** and administration of the compound results in a reduction in HIV viral load in the patient.

### Pharmaceutical Compositions

Pharmaceutical compositions disclosed herein contain 5 w/w% to 35 w/w% of a sodium salt of the compound of Formula **(Ia),** together with 35 w/w% to 75 w/w% PEG 300, 10 w/w% to 40 w/w% water, and optionally other therapeutic agents. The pharmaceutical compositions of the invention are in the form of a solution.

Pharmaceutical compositions comprising the sodium salt of the compound of Formula **(Ia)** can be prepared with conventional carriers (for example, inactive ingredient or excipient material) which can be selected in accord with ordinary practice. All compositions can optionally contain excipients such as those set forth in the Rowe et al., Handbook of Pharmaceutical Excipients, 5th edition, American Pharmacists Association, 1986. Excipients can include ascorbic acid and other antioxidants, chelating agents such as EDTA, carbohydrates such as dextrin, hydroxyalkylcellulose, hydroxyalkylmethylcellulose, stearic acid and the like.

Injectable solutions can be prepared in sterile form, and generally can be isotonic. All compositions can optionally contain excipients such as those set forth in the Rowe et al., Handbook of Pharmaceutical Excipients, 5th edition, American Pharmacists Association, 1986. Excipients can include ascorbic acid and other antioxidants, chelating agents such as EDTA, carbohydrates such as dextrin, hydroxyalkylcellulose, hydroxyalkylmethylcellulose, stearic acid and the like.

The active ingredient (the sodium salt of the compound of Formula **(Ia))** is presented as a pharmaceutical composition. The composition, both for veterinary and for human use, contains 5 w/w% to 35 w/w% of a sodium salt of the compound of Formula **(Ia)** together with 35 w/w% to 75 w/w% PEG 300, 10 w/w% to 40 w/w% water, and optionally other therapeutic ingredients. In one embodiment, the pharmaceutical composition further comprises a therapeutically effective amount of one or more (for example, one, two, three, or four; or one or two; or one to three; or one to four) additional therapeutic agents as defined herein. In some embodiments, the pharmaceutical composition further comprises one other therapeutic ingredient. The carrier(s) are "acceptable" in the sense of being compatible with the other ingredients of the composition and physiologically innocuous to the recipient thereof.

In some embodiments, the pharmaceutical compositions disclosed herein further comprise one other therapeutic agent selected from the group consisting of tenofovir alafenamide, tenofovir alafenamide hemifumarate, and bictegravir. In some embodiments, the pharmaceutical compositions disclosed herein further comprise one other therapeutic agent selected from the group consisting of tenofovir alafenamide hemifumarate and bictegravir. In some embodiments, the pharmaceutical compositions disclosed herein further comprise tenofovir alafenamide. In some embodiments, the pharmaceutical compositions disclosed herein further comprise tenofovir alafenamide hemifumarate. In some embodiments, the pharmaceutical compositions disclosed herein further comprise bictegravir.

The compositions include those suitable for various administration routes. The compositions can conveniently be presented in unit dosage form and can be prepared by any of the methods well known in the art of pharmacy. Such methods include the step of bringing into association the active ingredient (a sodium salt of the compound of Formula **(Ia))** with one or more inactive ingredients (PEG 300 and water). The compositions can be prepared by uniformly and intimately bringing into association the active ingredient with liquid carriers. Techniques and formulations generally are found in Remington: The Science and Practice of Pharmacy, 21st Edition, Lippincott Williams and Wilkins, Philadelphia, Pa., 2006.

The pharmaceutical compositions of the present disclosure can be in the form of a sterile injectable solution.

In some embodiments, the amount of water in the solution comprising PEG 300 and water is about 25 w/w%, about 23 w/w%, about 20 w/w%, about 17 w/w%, about 15 w/w%, or about 10 w/w%. In some embodiments, the amount of PEG 300 in the solution comprising PEG 300 and water is about 75 w/w%, about 70 w/w%, about 65 w/w%, about 60 w/w%, about 55 w/w%, about 50 w/w%, or about 45 w/w%. In some embodiments, the solution comprising PEG 300 and water further comprises a base. In some embodiments, the solution comprising PEG 300 and water further comprises an inorganic base. In some embodiments, the inorganic base is sodium hydroxide or sodium ethoxide. In some embodiments, the sodium hydroxide or sodium ethoxide is in an amount of about 3 w/w%, about 2 w/w%, about 1 w/w%, or about 0.5 w/w%.

The formulations suitable for parenteral administration (for example, intramuscular (IM) and subcutaneous (SC) administration) will include one or more excipients. Excipients should be compatible with the other ingredients of the formulation and physiologically innocuous to the recipient thereof. Examples of suitable excipients are well known to the person skilled in the art of parenteral formulation and can be found, for example, in the Handbook of Pharmaceutical Excipients (eds. Rowe, Sheskey & Quinn), 6th edition 2009.

Examples of solubilizing excipients in a parenteral formulation (for example, an SC or IM formulation) include, but are not limited to, polysorbates (such as polysorbate 20 or 80) and poloxamers (such as poloxamer 338, 188, or 207). In some embodiments, disclosed herein is a parenteral administration (for example, an SC or IM formulation) according to the claims that comprises a pharmaceutical composition comprising the sodium salt of the compound of Formula **(Ia)** and a poloxamer. In some embodiments, the poloxamer is poloxamer 338. In some embodiments, the poloxamer is poloxamer 188. In some embodiments, the amount of poloxamer in a parenteral administration disclosed herein is less than about 5%, such as less than about 3%, about 2%, about 1%, or about 0.5%.

In certain embodiments, excipients include N-methyl-2- pyrrolidone (NMP), dimethyl sulfoxide, polyethylene glycol and/or tetraglycol/glycofurol.

In general, poloxamers are synthetic non-ionic triblock of linear copolymers having a central hydrophobic chain of polyoxypropylene adjacent to two hydrophilic polypropylene oxide, in certain instances in a 4:2:4 weight ratio. Accordingly, in certain embodiments, the pharmaceutical composition comprising the sodium salt of the compound of Formula **(Ia)** includes a block copolymer comprised of one polyoxypropylene segment and two hydrophilic polypropylene oxide segments. In certain embodiments, the ratio of the polyoxypropylene segment to the two hydrophilic polypropylene oxide segments is 4:2:4 (hydrophilic polypropylene oxide: polyoxypropylene: hydrophilic polypropylene oxide). Poloxamers are generally understood to have the following structure: 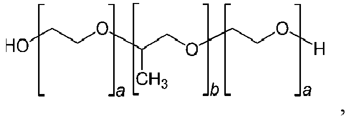 where a and b are integers. For example, a is between about 2 and about 130 and b is between about 15 and about 67. Poloxamer 188, for example, is understood to have a molecular weight from about 7680 to about 9510 Daltons (where a is about 80 and b is about 27) (see, for example, International Journal of PharmTech Research, Vol. 1, No.2, pp 299-303, April-June 2009). In some instances, poloxamer 188 has an average molecular weight of about 8400 Daltons. Poloxamer 338 has a molecular weight in the range of from about 12700 Da to about 17400 Da (where a is about 141 and b is about 44).

In some embodiments, poloxamers are synthetic non-ionic triblock of linear copolymers having a central hydrophobic chain of polypropylene oxide (PPO) adjacent to two hydrophilic chains of polyethylene oxide (PEO), in certain instances in a 4:2:4 weight ratio. Accordingly, in certain embodiments, the pharmaceutical composition comprising the sodium salt of the compound of Formula **(Ia)** includes a block copolymer comprised of one PPO segment and two PEO segments. In certain embodiments, the ratio of the PPO segment to the two PEO segments is 4:2:4 (PEO:PPO:PEO). Poloxamers are generally understood to have the following structure: 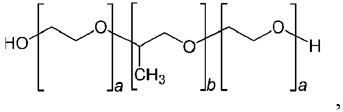 where a and b are integers. For example, a is between about 2 and about 130 and b is between about 15 and about 67. Poloxamer 188, for example, is understood to have a molecular weight from about 7680 to about 9510 Daltons (where a is about 80 and b is about 27) (see, for example, International Journal of PharmTech Research, Vol. 1, No.2, pp 299-303, April-June 2009). In some instances, poloxamer 188 has an average molecular weight of about 8400 Daltons. Poloxamer 338 has a molecular weight in the range of from about 12700 Da to about 17400 Da (where a is about 141 and b is about 44).

In some embodiments, the pharmaceutical composition comprising the sodium salt of the compound of Formula **(Ia)** is a solution that comprises a poloxamer. In some embodiments, the pharmaceutical composition comprising the sodium salt of the compound of Formula **(Ia)** is a solution that comprises poloxamer 188. In some embodiments, the pharmaceutical composition comprising the sodium salt of the compound of Formula **(Ia)** is a solution that does not comprise a poloxamer. In some embodiments, the pharmaceutical composition comprising the sodium salt of the compound of Formula **(Ia)** is a solution that does not comprise poloxamer 188.

In general, polyethylene glycol (PEG) is a polyether having a general formula H-(O-CH₂-CH₂)ₙ-OH. In certain embodiments, the PEG may be "capped" by an alkyl group. In those embodiments, the capped PEG is of the formula alkyl-( O-CH₂-CH₂)ₙ-O-alkyl (for example, CH₃-(O-CH₂-CH₂)ₙ-OCH₃).

According to the present invention, the The pharmaceutical composition comprising a sodium salt of the compound of Formula **(Ia)** comprises a compound of Formula **(Ia)** PEG 300, and water.

The amount of water in the solution comprising the sodium salt of the compound of Formula **(Ia),** PEG 300, and water is about 10 w/w% to about 40 w/w%. In some embodiments, the amount of water in the solution comprising the sodium salt of the compound of Formula (Ia), PEG 300, and water is about 10.0 w/w%, about 10.1 w/w%, about 10.2 w/w%, about 10.3 w/w%, about 10.4 w/w%, about 10.5 w/w%, about 10.6 w/w%, about 10.7 w/w%, about 10.8 w/w%, about 10.9 w/w%, about 11.0 w/w%, about 11.1 w/w%, about 11.2 w/w%, about 11.3 w/w%, about 11.4 w/w%, about 11.5 w/w%, about 11.6 w/w%, about 11.7 w/w%, about 11.8 w/w%, about 11.9 w/w%, or about 12.0 w/w%.

The amount of PEG 300 in the solution comprising the sodium salt of the compound of Formula **(Ia),** PEG 300, and water is about 35 w/w% to about 75 w/w%. In some embodiments, the amount of PEG 300 in the solution comprising the sodium salt of the compound of Formula **(Ia),** PEG 300, and water is about 50 w/w%, about 55 w/w%, about 60 w/w%, about 65 w/w%, about 70 w/w%, or about 75 w/w%. In some embodiments, the amount of PEG 300 in the solution comprising the sodium salt of the compound of Formula **(Ia),** PEG 300, and water is about 65 w/w%. In some embodiments, the amount of PEG 300 in the solution comprising the sodium salt of the compound of Formula **(Ia),** PEG 300, and water is about 65.0 w/w%.

The amount of the sodium salt of the compound of Formula (Ia) in the solution comprising the sodium salt of the compound of Formula (Ia), PEG 300, and water is about 5 w/w% to about 35 w/w%.

In some embodiments, the amount of the sodium salt of the compound of Formula **(Ia)** in the solution comprising the sodium salt of the compound of Formula **(Ia),** PEG 300, and water is about 15 w/w% to about 35 w/w%. In some embodiments, the amount of the sodium salt of the compound of Formula **(Ia)** in the solution comprising the sodium salt of the compound of Formula **(Ia),** PEG 300, and water is about 20 w/w% to about 35 w/w%. In some embodiments, the amount of the sodium salt of the compound of Formula **(Ia)** in the solution comprising the sodium salt of the compound of Formula **(Ia),** PEG 300, and water is about 24 w/w% to about 26 w/w%. In some embodiments, the amount of the sodium salt of the compound of Formula **(Ia)** in the solution comprising the sodium salt of the compound of Formula **(Ia),** PEG 300, and water is about 24.5 w/w%, about 24.6 w/w%, about 24.7 w/w%, about 24.8 w/w%, about 24.9 w/w%, about 25.0 w/w%, about 25.1 w/w%, about 25.2 w/w%, about 25.3 w/w%, about 25.4 w/w%, or about 25.5 w/w%. In some embodiments, the amount of the sodium salt of the compound of Formula **(Ia)** in the solution comprising the sodium salt of the compound of Formula **(Ia),** PEG 300, and water is about 25 w/w%. In some embodiments, the amount of the sodium salt of the compound of Formula **(Ia)** in the solution comprising the sodium salt of the compound of Formula **(Ia),** PEG 300, and water is about 25.2 w/w%.

In some embodiments, the solution comprises about 10 w/w% water, about 65.0 w/w% PEG 300, and about 25 w/w% of the sodium salt of the compound of Formula **(Ia).**

In some embodiments, the concentration of the compound of Formula **(Ia)** in the solution comprising a sodium salt of the compound of Formula **(Ia),** PEG 300, and water is about 50 mg/ml to about 300 mg/ml. In some embodiments, the concentration of the compound of Formula **(Ia)** in the solution comprising a sodium salt of the compound of Formula **(Ia),** PEG 300, and water is about 75 mg/ml to about 300 mg/ml. In some embodiments, the concentration of the compound of Formula **(Ia)** in the solution comprising a sodium salt of the compound of Formula **(Ia),** PEG 300, and water is about 50 mg/ml. In some embodiments, the concentration of the compound of Formula **(Ia)** in the solution comprising a sodium salt of the compound of Formula **(Ia),** PEG 300, and water is about 75 mg/ml. In some embodiments, the concentration of the compound of Formula **(Ia)** in the solution comprising a sodium salt of the compound of Formula **(Ia),** PEG 300, and water is about 100 mg/ml. In some embodiments, the concentration of the compound of Formula **(Ia)** in the solution comprising a sodium salt of the compound of Formula **(Ia),** PEG 300, and water is about 125 mg/ml. In some embodiments, the concentration of the compound of Formula **(Ia)** in the solution comprising a sodium salt of the compound of Formula **(Ia),** PEG 300, and water is about 150 mg/ml. In some embodiments, the concentration of the compound of Formula **(Ia)** in the solution comprising a sodium salt of the compound of Formula **(Ia),** PEG 300, and water is about 175 mg/ml. In some embodiments, the concentration of the compound of Formula **(Ia)** in the solution comprising a sodium salt of the compound of Formula **(Ia),** PEG 300, and water is about 200 mg/ml. In some embodiments, the concentration of the compound of Formula **(Ia)** in the solution comprising a sodium salt of the compound of Formula **(Ia),** PEG 300, and water is about 225 mg/ml. In some embodiments, the concentration of the compound of Formula **(Ia)** in the solution comprising a sodium salt of the compound of Formula **(Ia),** PEG 300, and water is about 250 mg/ml. In some embodiments, the concentration of the compound of Formula **(Ia)** in the solution comprising a sodium salt of the compound of Formula **(Ia),** PEG 300, and water is about 275 mg/ml. In some embodiments, the concentration of the compound of Formula **(Ia)** in the solution comprising a sodium salt of the compound of Formula **(Ia),** PEG 300, and water is about 300 mg/ml. In some embodiments, the concentration of the compound of Formula **(Ia)** in the solution comprising a sodium salt of the compound of Formula **(Ia),** PEG 300, and water is about 325 mg/ml. In some embodiments, the concentration of the compound of Formula **(Ia)** in the solution comprising a sodium salt of the compound of Formula **(Ia),** PEG 300, and water is about 350 mg/ml.

The amount of water in the solution comprising a sodium salt of the compound of Formula **(Ia),** PEG 300, and water is about 10 w/w% to about 40 w/w%. In some embodiments, the amount of water in the solution comprising a sodium salt of the compound of Formula **(Ia),** PEG 300, and water is about 15 w/w% to about 35 w/w%. In some embodiments, the amount of water in the solution comprising a sodium salt of the compound of Formula **(Ia),** PEG 300, and water is about 20 w/w% to about 30 w/w%. In some embodiments, the amount of water in the solution comprising a sodium salt of the compound of Formula **(Ia),** PEG 300, and water is about 21 w/w% to about 29 w/w%. In some embodiments, the amount of water in the solution comprising a sodium salt of the compound of Formula **(Ia),** PEG 300, and water is about 23.4 w/w% to about 27.5 w/w%. In some embodiments, the amount of water in the solution comprising a sodium salt of the compound of Formula **(Ia),** PEG 300, and water is about 23.41 w/w% to about 27.47 w/w%. In some embodiments, the amount of water in the solution comprising a sodium salt of the compound of Formula **(Ia),** PEG 300, and water is about 22.0 w/w%, about 22.1 w/w%, about 22.2 w/w%, about 22.3 w/w%, about 22.4 w/w%, about 22.5 w/w%, about 22.6 w/w%, about 22.7 w/w%, about 22.8 w/w%, about 22.9 w/w%, about 23.0 w/w%, about 23.1 w/w%, about 23.2 w/w%, about 23.3 w/w%, about 23.4 w/w%, about 23.5 w/w%, about 23.6 w/w%, about 23.7 w/w%, about 23.8 w/w%, about 23.9 w/w%, about 24.0 w/w%, about 24.1 w/w%, about 24.2 w/w%, about 24.3 w/w%, about 24.4 w/w%, about 24.5 w/w%, about 24.6 w/w%, about 24.7 w/w%, about 24.8 w/w%, about 24.9 w/w%, about 25.0 w/w%, about 25.1 w/w%, about 25.2 w/w%, about 25.3 w/w%, about 25.4 w/w%, about 25.5 w/w%, about 25.6 w/w%, about 25.7 w/w%, about 25.8 w/w%, about 25.9 w/w%, about 26.0 w/w%, about 26.1 w/w%, about 26.2 w/w%, about 26.3 w/w%, about 26.4 w/w%, about 26.5 w/w%, about 26.6 w/w%, about 26.7 w/w%, about 26.8 w/w%, about 26.9 w/w%, about 27.0 w/w%, about 27.1 w/w%, about 27.2 w/w%, about 27.3 w/w%, about 27.4 w/w%, about 27.5 w/w%, about 27.6 w/w%, about 27.7 w/w%, about 27.8 w/w%, about 27.9 w/w%, about 28.0 w/w%, about 28.1 w/w%, about 28.2 w/w%, about 28.3 w/w%, about 28.4 w/w%, about 28.5 w/w%, about 28.6 w/w%, about 28.7 w/w%, about 28.8 w/w%, about 28.9 w/w%, or about 29.0 w/w%. In some embodiments, the amount of water in the solution comprising a sodium salt of the compound of Formula **(Ia),** PEG 300, and water is about 23.4 w/w%. In some embodiments, the amount of water in the solution comprising a sodium salt of the compound of Formula **(Ia),** PEG 300, and water is about 23.41 w/w%. In some embodiments, the amount of water in the solution comprising a sodium salt of the compound of Formula **(Ia),** PEG 300, and water is about 27.47 w/w%. In some embodiments, the amount of water in the solution comprising a sodium salt of the compound of Formula **(Ia),** PEG 300, and water is about 27.5 w/w%.

The amount of PEG 300 in the solution comprising a sodium salt of the compound of Formula **(Ia),** PEG 300, and water is about 35 w/w% to about 75 w/w%. In some embodiments, the amount of PEG 300 in the solution comprising a sodium salt of the compound of Formula **(Ia),** PEG 300, and water is about 45 w/w% to about 65 w/w%. In some embodiments, the amount of PEG 300 in the solution comprising a sodium salt of the compound of Formula **(Ia),** PEG 300, and water is about 48 w/w% to about 60 w/w%. In some embodiments, the amount of PEG 300 in the solution comprising a sodium salt of the compound of Formula **(Ia),** PEG 300, and water is about 50 w/w% to about 59 w/w%. In some embodiments, the amount of PEG 300 in the solution comprising a sodium salt of the compound of Formula **(Ia),** PEG 300, and water is about 50.1 w/w% to about 58.8 w/w%. In some embodiments, the amount of PEG 300 in the solution comprising a sodium salt of the compound of Formula **(Ia),** PEG 300, and water is about 50.13 w/w% to about 58.84 w/w%. In some embodiments, the amount of PEG 300 in the solution comprising a sodium salt of the compound of Formula **(Ia),** PEG 300, and water is about 45 w/w%, about 46 w/w%, about 47 w/w%, about 48 w/w%, about 49 w/w%, about 50 w/w%, about 51 w/w%, about 52 w/w%, about 53 w/w%, about 54 w/w%, about 55 w/w%, about 56 w/w%, about 57 w/w%, about 58 w/w%, about 59 w/w%, about 60 w/w%, about 61 w/w%, about 62 w/w%, about 63 w/w%, about 64 w/w%, or about 65 w/w%. In some embodiments, the amount of PEG 300 in the solution comprising a sodium salt of the compound of Formula **(Ia),** PEG 300, and water is about 50.1 w/w%. In some embodiments, the amount of PEG 300 in the solution comprising a sodium salt of the compound of Formula **(Ia),** PEG 300, and water is about 50.13 w/w%. In some embodiments, the amount of PEG 300 in the solution comprising a sodium salt of the compound of Formula **(Ia),** PEG 300, and water is about 58.8 w/w%. In some embodiments, the amount of PEG 300 in the solution comprising a sodium salt of the compound of Formula **(Ia),** PEG 300, and water is about 58.84 w/w%.

The amount of the sodium salt of the compound of Formula **(Ia)** in the solution comprising a sodium salt of the compound of Formula **(Ia),** PEG 300, and water is about 5 w/w% to about 35 w/w%. In some embodiments, the amount of the sodium salt of the compound of Formula **(Ia)** in the solution comprising a sodium salt of the compound of Formula **(Ia),** PEG 300, and water is about 10 w/w% to about 30 w/w%. In some embodiments, the amount of the sodium salt of the compound of Formula **(Ia)** in the solution comprising a sodium salt of the compound of Formula **(Ia),** PEG 300, and water is about 11 w/w% to about 28 w/w%. In some embodiments, the amount of the sodium salt of the compound of Formula **(Ia)** in the solution comprising a sodium salt of the compound of Formula **(Ia),** PEG 300, and water is about 13 w/w% to about 27 w/w%. In some embodiments, the amount of the sodium salt of the compound of Formula **(Ia)** in the solution comprising a sodium salt of the compound of Formula **(Ia),** PEG 300, and water is about 13.69 w/w% to about 26.46 w/w%. In some embodiments, the amount of the sodium salt of the compound of Formula **(Ia)** in the solution comprising a sodium salt of the compound of Formula **(Ia),** PEG 300, and water is about 13.7 w/w% to about 26.5 w/w%. In some embodiments, the amount of the sodium salt of the compound of Formula **(Ia)** in the solution comprising a sodium salt of the compound of Formula **(Ia),** PEG 300, and water is about 13.0 w/w%, about 13.1 w/w%, about 13.2 w/w%, about 13.3 w/w%, about 13.4 w/w%, about 13.5 w/w%, about 13.6 w/w%, about 13.7 w/w%, about 13.8 w/w%, about 13.9 w/w%, about 14.0 w/w%, about 14.1 w/w%, about 14.2 w/w%, about 14.3 w/w%, about 14.4 w/w%, about 14.5 w/w%, about 14.6 w/w%, about 14.7 w/w%, about 14.8 w/w%, about 14.9 w/w%, about 15.0 w/w%, about 15.1 w/w%, about 15.2 w/w%, about 15.3 w/w%, about 15.4 w/w%, about 15.5 w/w%, about 15.6 w/w%, about 15.7 w/w%, about 15.8 w/w%, about 15.9 w/w%, about 16.0 w/w%, about 16.1 w/w%, about 16.2 w/w%, about 16.3 w/w%, about 16.4 w/w%, about 16.5 w/w%, about 16.6 w/w%, about 16.7 w/w%, about 16.8 w/w%, about 16.9 w/w%, about 17.0 w/w%, about 17.1 w/w%, about 17.2 w/w%, about 17.3 w/w%, about 17.4 w/w%, about 17.5 w/w%, about 17.6 w/w%, about 17.7 w/w%, about 17.8 w/w%, about 17.9 w/w%, about 18.0 w/w%, about 18.1 w/w%, about 18.2 w/w%, about 18.3 w/w%, about 18.4 w/w%, about 18.5 w/w%, about 18.6 w/w%, about 18.7 w/w%, about 18.8 w/w%, about 18.9 w/w%, about 19.0 w/w%, about 19.1 w/w%, about 19.2 w/w%, about 19.3 w/w%, about 19.4 w/w%, about 19.5 w/w%, about 19.6 w/w%, about 19.7 w/w%, about 19.8 w/w%, about 19.9 w/w%, about 20.0 w/w%, about 21.1 w/w%, about 21.2 w/w%, about 21.3 w/w%, about 21.4 w/w%, about 21.5 w/w%, about 21.6 w/w%, about 21.7 w/w%, about 21.8 w/w%, about 21.9 w/w%, about 22.0 w/w%, about 22.1 w/w%, about 22.2 w/w%, about 22.3 w/w%, about 22.4 w/w%, about 22.5 w/w%, about 22.6 w/w%, about 22.7 w/w%, about 22.8 w/w%, about 22.9 w/w%, about 23.0 w/w%, about 23.1 w/w%, about 23.2 w/w%, about 23.3 w/w%, about 23.4 w/w%, about 23.5 w/w%, about 23.6 w/w%, about 23.7 w/w%, about 23.8 w/w%, about 23.9 w/w%, about 24.0 w/w%, about 24.1 w/w%, about 24.2 w/w%, about 24.3 w/w%, about 24.4 w/w%, about 24.5 w/w%, about 24.6 w/w%, about 24.7 w/w%, about 24.8 w/w%, about 24.9 w/w%, about 25.0 w/w%, about 25.1 w/w%, about 25.2 w/w%, about 25.3 w/w%, about 25.4 w/w%, about 25.5 w/w%, about 25.6 w/w%, about 25.7 w/w%, about 25.8 w/w%, about 25.9 w/w%, about 26.0 w/w%, about 26.1 w/w%, about 26.2 w/w%, about 26.3 w/w%, about 26.4 w/w%, about 26.5 w/w%, about 26.6 w/w%, about 26.7 w/w%, about 26.8 w/w%, about 26.9 w/w%, about 27.0 w/w%, about 27.1 w/w%, about 27.2 w/w%, about 27.3 w/w%, about 27.4 w/w%, about 27.5 w/w%, about 27.6 w/w%, about 27.7 w/w%, about 27.8 w/w%, about 27.9 w/w%, about 28.0 w/w%, about 28.1 w/w%, about 28.2 w/w%, about 28.3 w/w%, about 28.4 w/w%, about 28.5 w/w%, about 28.6 w/w%, about 28.7 w/w%, about 28.8 w/w%, about 28.9 w/w%, or about 29.0 w/w%. In some embodiments, the amount of the sodium salt of the compound of Formula **(Ia)** in the solution comprising a sodium salt of the compound of Formula **(Ia),** PEG 300, and water is about 13.69 w/w%. In some embodiments, the amount of the sodium salt of the compound of Formula **(Ia)** in the solution comprising a sodium salt of the compound of Formula **(Ia),** PEG 300, and water is about 13.7 w/w%. In some embodiments, the amount of the sodium salt of the compound of Formula **(Ia)** in the solution comprising a sodium salt of the compound of Formula **(Ia),** PEG 300, and water is about 26.46 w/w%. In some embodiments, the amount of the sodium salt of the compound of Formula **(Ia)** in the solution comprising a sodium salt of the compound of Formula **(Ia),** PEG 300, and water is about 26.5 w/w%.

The solution according to the present invention comprises about 10 w/w% to about 40 w/w% water, about 35 w/w% to about 75 w/w% PEG 300, and about 5 w/w% to about 35 w/w% of a sodium salt of the compound of Formula **(Ia).** In some embodiments, the solution comprises about 15 w/w% to about 35 w/w% water, about 45 w/w% to about 65 w/w% PEG 300, and about 10 w/w% to about 30 w/w% of a sodium salt of the compound of Formula **(Ia).** In some embodiments, the solution comprises about 20 w/w% to about 30 w/w% water, about 48 w/w% to about 60 w/w% PEG 300, and about 11 w/w% to about 28 w/w% of a sodium salt of the compound of Formula **(Ia).** In some embodiments, the solution comprises about 21 w/w% to about 29 w/w% water, about 50 w/w% to about 59 w/w% PEG 300, and about 13 w/w% to about 27 w/w% of a sodium salt of the compound of Formula **(Ia).** In some embodiments, the solution comprises about 23.4 w/w% to about 27.5 w/w% water, about 50.1 w/w% to about 58.8 w/w% PEG 300, and about 13.7 w/w% to about 26.5 w/w% of a sodium salt of the compound of Formula **(Ia).** In some embodiments, the solution comprises about 23.41 w/w% to about 27.47 w/w% water, about 50.13 w/w% to about 58.84 w/w% PEG 300, and about 13.69 w/w% to about 26.46 w/w% of a sodium salt of the compound of Formula **(Ia).** In some embodiments, the solution comprises about 27.5 w/w% water, about 58.8 w/w% PEG 300, and about 13.7 w/w% of a sodium salt of the compound of Formula **(Ia).** In some embodiments, the solution comprises about 27.47 w/w% water, about 58.84 w/w% PEG 300, and about 13.69 w/w% of a sodium salt of the compound of Formula **(Ia).** In some embodiments, the solution comprises about 23.4 w/w% water, about 50.1 w/w% PEG 300, and about 26.5 w/w% of a sodium salt of the compound of Formula **(Ia).** In some embodiments, the solution comprises about 23.41 w/w% water, about 50.13 w/w% PEG 300, and about 26.46 w/w% of a sodium salt of the compound of Formula **(Ia).**

In some embodiments, the solution disclosed herein comprises a sodium salt of the compound of Formula **(Ia),** PEG 300, poloxamer 188, and water. In some embodiments, the concentration of the compound of Formula **(Ia)** in the solution comprising a sodium salt of the compound of Formula **(Ia),** PEG 300, poloxamer 188, and water is about 50 mg/ml to about 300 mg/ml. In some embodiments, the concentration of the compound of Formula **(Ia)** in the solution comprising a sodium salt of the compound of Formula **(Ia),** PEG 300, poloxamer 188, and water is about 75 mg/ml to about 300 mg/ml. In some embodiments, the concentration of the compound of Formula **(Ia)** in the solution comprising a sodium salt of the compound of Formula **(Ia),** PEG 300, poloxamer 188, and water is about 50 mg/ml. In some embodiments, the concentration of the compound of Formula **(Ia)** in the solution comprising a sodium salt of the compound of Formula **(Ia),** PEG 300, poloxamer 188, and water is about 75 mg/ml. In some embodiments, the concentration of the compound of Formula **(Ia)** in the solution comprising a sodium salt of the compound of Formula **(Ia),** PEG 300, poloxamer 188, and water is about 100 mg/ml. In some embodiments, the concentration of the compound of Formula **(Ia)** in the solution comprising a sodium salt of the compound of Formula **(Ia),** PEG 300, poloxamer 188, and water is about 125 mg/ml. In some embodiments, the concentration of the compound of Formula **(Ia)** in the solution comprising a sodium salt of the compound of Formula **(Ia),** PEG 300, poloxamer 188, and water is about 150 mg/ml. In some embodiments, the concentration of the compound of Formula **(Ia)** in the solution comprising a sodium salt of the compound of Formula **(Ia),** PEG 300, poloxamer 188, and water is about 175 mg/ml. In some embodiments, the concentration of the compound of Formula **(Ia)** in the solution comprising a sodium salt of the compound of Formula **(Ia),** PEG 300, poloxamer 188, and water is about 200 mg/ml. In some embodiments, the concentration of the compound of Formula **(Ia)** in the solution comprising a sodium salt of the compound of Formula **(Ia),** PEG 300, poloxamer 188, and water is about 225 mg/ml. In some embodiments, the concentration of the compound of Formula **(Ia)** in the solution comprising a sodium salt of the compound of Formula **(Ia),** PEG 300, poloxamer 188, and water is about 250 mg/ml. In some embodiments, the concentration of the compound of Formula **(Ia)** in the solution comprising a sodium salt of the compound of Formula **(Ia),** PEG 300, poloxamer 188, and water is about 275 mg/ml. In some embodiments, the concentration of the compound of Formula **(Ia)** in the solution comprising a sodium salt of the compound of Formula **(Ia),** PEG 300, poloxamer 188, and water is about 300 mg/ml. In some embodiments, the concentration of the compound of Formula **(Ia)** in the solution comprising a sodium salt of the compound of Formula **(Ia),** PEG 300, poloxamer 188, and water is about 325 mg/ml. In some embodiments, the concentration of the compound of Formula **(Ia)** in the solution comprising a sodium salt of the compound of Formula **(Ia),** PEG 300, poloxamer 188, and water is about 350 mg/ml.

In some embodiments, the amount of water in the solution comprising a sodium salt of the compound of Formula **(Ia),** PEG 300, poloxamer 188, and water is about 15 w/w% to about 35 w/w%. In some embodiments, the amount of water in the solution comprising a sodium salt of the compound of Formula **(Ia),** PEG 300, poloxamer 188, and water is about 20 w/w% to about 35 w/w%. In some embodiments, the amount of water in the solution comprising a sodium salt of the compound of Formula **(Ia),** PEG 300, poloxamer 188, and water is about 20 w/w% to about 31 w/w%. In some embodiments, the amount of water in the solution comprising a sodium salt of the compound of Formula **(Ia),** PEG 300, poloxamer 188, and water is about 21.9 w/w% to about 30.1 w/w%. In some embodiments, the amount of water in the solution comprising a sodium salt of the compound of Formula **(Ia),** PEG 300, poloxamer 188, and water is about 21.87 w/w% to about 30.07 w/w%. In some embodiments, the amount of water in the solution comprising a sodium salt of the compound of Formula **(Ia),** PEG 300, poloxamer 188, and water is about 19.0 w/w%, about 19.1 w/w%, about 19.2 w/w%, about 19.3 w/w%, about 19.4 w/w%, about 19.5 w/w%, about 19.6 w/w%, about 19.7 w/w%, about 19.8 w/w%, about 19.9 w/w%, about 20.0 w/w%, about 20.1 w/w%, about 20.2 w/w%, about 20.3 w/w%, about 20.4 w/w%, about 20.5 w/w%, about 20.6 w/w%, about 20.7 w/w%, about 20.8 w/w%, about 20.9 w/w%, about 21.0 w/w%, about 21.1 w/w%, about 21.2 w/w%, about 21.3 w/w%, about 21.4 w/w%, about 21.5 w/w%, about 21.6 w/w%, about 21.7 w/w%, about 21.8 w/w%, about 21.9 w/w%, about 22.0 w/w%, about 22.1 w/w%, about 22.2 w/w%, about 22.3 w/w%, about 22.4 w/w%, about 22.5 w/w%, about 22.6 w/w%, about 22.7 w/w%, about 22.8 w/w%, about 22.9 w/w%, about 23.0 w/w%, about 23.1 w/w%, about 23.2 w/w%, about 23.3 w/w%, about 23.4 w/w%, about 23.5 w/w%, about 23.6 w/w%, about 23.7 w/w%, about 23.8 w/w%, about 23.9 w/w%, about 24.0 w/w%, about 24.1 w/w%, about 24.2 w/w%, about 24.3 w/w%, about 24.4 w/w%, about 24.5 w/w%, about 24.6 w/w%, about 24.7 w/w%, about 24.8 w/w%, about 24.9 w/w%, about 25.0 w/w%, about 25.1 w/w%, about 25.2 w/w%, about 25.3 w/w%, about 25.4 w/w%, about 25.5 w/w%, about 25.6 w/w%, about 25.7 w/w%, about 25.8 w/w%, about 25.9 w/w%, about 26.0 w/w%, about 26.1 w/w%, about 26.2 w/w%, about 26.3 w/w%, about 26.4 w/w%, about 26.5 w/w%, about 26.6 w/w%, about 26.7 w/w%, about 26.8 w/w%, about 26.9 w/w%, about 27.0 w/w%, about 27.1 w/w%, about 27.2 w/w%, about 27.3 w/w%, about 27.4 w/w%, about 27.5 w/w%, about 27.6 w/w%, about 27.7 w/w%, about 27.8 w/w%, about 27.9 w/w%, about 28.0 w/w%, about 28.1 w/w%, about 28.2 w/w%, about 28.3 w/w%, about 28.4 w/w%, about 28.5 w/w%, about 28.6 w/w%, about 28.7 w/w%, about 28.8 w/w%, about 28.9 w/w%, about 29.0 w/w%, about 29.1 w/w%, about 29.2 w/w%, about 29.3 w/w%, about 29.4 w/w%, about 29.5 w/w%, about 29.6 w/w%, about 29.7 w/w%, about 29.8 w/w%, about 29.9 w/w%, about 30.0 w/w%, about 30.1 w/w%, about 30.2 w/w%, about 30.3 w/w%, about 30.4 w/w%, about 30.5 w/w%, about 30.6 w/w%, about 30.7 w/w%, about 30.8 w/w%, about 30.9 w/w%, about 31.0 w/w%, about 31.1 w/w%, about 31.2 w/w%, about 31.3 w/w%, about 31.4 w/w%, about 31.5 w/w%, about 31.6 w/w%, about 31.7 w/w%, about 31.8 w/w%, about 31.9 w/w%, about 32.0 w/w%, about 32.1 w/w%, about 32.2 w/w%, about 32.3 w/w%, about 32.4 w/w%, about 32.5 w/w%, about 32.6 w/w%, about 32.7 w/w%, about 32.8 w/w%, about 32.9 w/w%, or about 33.0 w/w%. In some embodiments, the amount of water in the solution comprising a sodium salt of the compound of Formula **(Ia),** PEG 300, poloxamer 188, and water is about 21.87 w/w%. In some embodiments, the amount of water in the solution comprising a sodium salt of the compound of Formula **(Ia),** PEG 300, poloxamer 188, and water is about 21.9 w/w%. In some embodiments, the amount of water in the solution comprising a sodium salt of the compound of Formula **(Ia),** PEG 300, poloxamer 188, and water is about 26.68 w/w%. In some embodiments, the amount of water in the solution comprising a sodium salt of the compound of Formula **(Ia),** PEG 300, poloxamer 188, and water is about 26.7 w/w%. In some embodiments, the amount of water in the solution comprising a sodium salt of the compound of Formula **(Ia),** PEG 300, and poloxamer 188, and water is about 27.5 w/w%. In some embodiments, the amount of water in the solution comprising a sodium salt of the compound of Formula **(Ia),** PEG 300, poloxamer 188, and water is about 27.51 w/w%. In some embodiments, the amount of water in the solution comprising a sodium salt of the compound of Formula **(Ia),** PEG 300, poloxamer 188, and water is about 28.36 w/w%. In some embodiments, the amount of water in the solution comprising a sodium salt of the compound of Formula **(Ia),** PEG 300, poloxamer 188, and water is about 28.4 w/w%. In some embodiments, the amount of water in the solution comprising a sodium salt of the compound of Formula **(Ia),** PEG 300, poloxamer 188, and water is about 29.2 w/w%. In some embodiments, the amount of water in the solution comprising a sodium salt of the compound of Formula **(Ia),** PEG 300, poloxamer 188, and water is about 29.21 w/w%. In some embodiments, the amount of water in the solution comprising a sodium salt of the compound of Formula **(Ia),** PEG 300, poloxamer 188, and water is about 30.07 w/w%. In some embodiments, the amount of water in the solution comprising a sodium salt of the compound of Formula **(Ia),** PEG 300, poloxamer 188, and water is about 30.1 w/w%.

In some embodiments, the amount of PEG 300 in the solution comprising a sodium salt of the compound of Formula **(Ia),** PEG 300, poloxamer 188, and water is about 35 w/w% to about 75 w/w%. In some embodiments, the amount of PEG 300 in the solution comprising a sodium salt of the compound of Formula **(Ia),** PEG 300, poloxamer 188, and water is about 40 w/w% to about 70 w/w%. In some embodiments, the amount of PEG 300 in the solution comprising a sodium salt of the compound of Formula **(Ia),** PEG 300, poloxamer 188, and water is about 45 w/w% to about 68 w/w%. In some embodiments, the amount of PEG 300 in the solution comprising a sodium salt of the compound of Formula **(Ia),** PEG 300, poloxamer 188, and water is about 46.8 w/w% to about 64.4 w/w%. In some embodiments, the amount of PEG 300 in the solution comprising a sodium salt of the compound of Formula **(Ia),** PEG 300, poloxamer 188, and water is about 46.84 w/w% to about 64.40 w/w%. In some embodiments, the amount of PEG 300 in the solution comprising a sodium salt of the compound of Formula **(Ia),** PEG 300, poloxamer 188, and water is about 40 w/w%, about 41 w/w%, about 42 w/w%, about 43 w/w%, about 44 w/w%, about 45 w/w%, about 46 w/w%, about 47 w/w%, about 48 w/w%, about 49 w/w%, about 50 w/w%, about 51 w/w%, about 52 w/w%, about 53 w/w%, about 54 w/w%, about 55 w/w%, about 56 w/w%, about 57 w/w%, about 58 w/w%, about 59 w/w%, about 60 w/w%, about 61 w/w%, about 62 w/w%, about 63 w/w%, about 64 w/w%, about 65 w/w%, about 66 w/w%, about 67 w/w%, about 68 w/w%, about 69 w/w%, or about 70 w/w%. In some embodiments, the amount of PEG 300 in the solution comprising a sodium salt of the compound of Formula **(Ia),** PEG 300, poloxamer 188, and water is about 46.8 w/w%. In some embodiments, the amount of PEG 300 in the solution comprising a sodium salt of the compound of Formula **(Ia),** PEG 300, poloxamer 188, and water is about 46.84 w/w%. In some embodiments, the amount of PEG 300 in the solution comprising a sodium salt of the compound of Formula **(Ia),** PEG 300, poloxamer 188, and water is about 57.1 w/w%. In some embodiments, the amount of PEG 300 in the solution comprising a sodium salt of the compound of Formula **(Ia),** PEG 300, poloxamer 188, and water is about 57.13 w/w%. In some embodiments, the amount of PEG 300 in the solution comprising a sodium salt of the compound of Formula **(Ia),** PEG 300, poloxamer 188, and water is about 58.9 w/w%. In some embodiments, the amount of PEG 300 in the solution comprising a sodium salt of the compound of Formula **(Ia),** PEG 300, poloxamer 188, and water is about 58.92 w/w%. In some embodiments, the amount of PEG 300 in the solution comprising a sodium salt of the compound of Formula **(Ia),** PEG 300, poloxamer 188, and water is about 60.7 w/w%. In some embodiments, the amount of PEG 300 in the solution comprising a sodium salt of the compound of Formula **(Ia),** PEG 300, poloxamer 188, and water is about 60.73 w/w%. In some embodiments, the amount of PEG 300 in the solution comprising a sodium salt of the compound of Formula **(Ia),** PEG 300, poloxamer 188, and water is about 62.55 w/w%. In some embodiments, the amount of PEG 300 in the solution comprising a sodium salt of the compound of Formula **(Ia),** PEG 300, poloxamer 188, and water is about 62.6 w/w%. In some embodiments, the amount of PEG 300 in the solution comprising a sodium salt of the compound of Formula **(Ia),** PEG 300, poloxamer 188, and water is about 64.4 w/w%. In some embodiments, the amount of PEG 300 in the solution comprising a sodium salt of the compound of Formula **(Ia),** PEG 300, poloxamer 188, and water is about 64.40 w/w%.

In some embodiments, the amount of the sodium salt of the compound of Formula **(Ia)** in the solution comprising a sodium salt of the compound of Formula **(Ia),** PEG 300, poloxamer 188, and water is about 5.0 w/w%, about 5.1 w/w%, about 5.2 w/w%, about 5.3 w/w%, about 5.4 w/w%, about 5.5 w/w%, about 5.6 w/w%, about 5.7 w/w%, about 5.8 w/w%, about 5.9 w/w%, about 6.0 w/w%, about 6.1 w/w%, about 6.2 w/w%, about 6.3 w/w%, about 6.4 w/w%, about 6.5 w/w%, about 6.6 w/w%, about 6.7 w/w%, about 6.8 w/w%, about 6.9 w/w%, about 7.0 w/w%, about 7.1 w/w%, about 7.2 w/w%, about 7.3 w/w%, about 7.4 w/w%, about 7.5 w/w%, about 7.6 w/w%, about 7.7 w/w%, about 7.8 w/w%, about 7.9 w/w%, about 8.0 w/w%, about 8.1 w/w%, about 8.2 w/w%, about 8.3 w/w%, about 8.4 w/w%, about 8.5 w/w%, about 8.6 w/w%, about 8.7 w/w%, about 8.8 w/w%, about 8.9 w/w%, about 9.0 w/w%, about 9.1 w/w%, about 9.2 w/w%, about 9.3 w/w%, about 9.4 w/w%, about 9.5 w/w%, about 9.6 w/w%, about 9.7 w/w%, about 9.8 w/w%, about 9.9 w/w%, about 10.0 w/w%, about 10.1 w/w%, about 10.2 w/w%, about 10.3 w/w%, about 10.4 w/w%, about 10.5 w/w%, about 10.6 w/w%, about 10.7 w/w%, about 10.8 w/w%, about 10.9 w/w%, about 11.0 w/w%, about 11.1 w/w%, about 11.2 w/w%, about 11.3 w/w%, about 11.4 w/w%, about 11.5 w/w%, about 11.6 w/w%, about 11.7 w/w%, about 11.8 w/w%, about 11.9 w/w%, about 12.0 w/w%, about 12.1 w/w%, about 12.2 w/w%, about 12.3 w/w%, about 12.4 w/w%, about 12.5 w/w%, about 12.6 w/w%, about 12.7 w/w%, about 12.8 w/w%, about 12.9 w/w%, about 13.0 w/w%, about 13.1 w/w%, about 13.2 w/w%, about 13.3 w/w%, about 13.4 w/w%, about 13.5 w/w%, about 13.6 w/w%, about 13.7 w/w%, about 13.8 w/w%, about 13.9 w/w%, about 14.0 w/w%, about 14.1 w/w%, about 14.2 w/w%, about 14.3 w/w%, about 14.4 w/w%, about 14.5 w/w%, about 14.6 w/w%, about 14.7 w/w%, about 14.8 w/w%, about 14.9 w/w%, about 15.0 w/w%, about 15.1 w/w%, about 15.2 w/w%, about 15.3 w/w%, about 15.4 w/w%, about 15.5 w/w%, about 15.6 w/w%, about 15.7 w/w%, about 15.8 w/w%, about 15.9 w/w%, about 16.0 w/w%, about 16.1 w/w%, about 16.2 w/w%, about 16.3 w/w%, about 16.4 w/w%, about 16.5 w/w%, about 16.6 w/w%, about 16.7 w/w%, about 16.8 w/w%, about 16.9 w/w%, about 17.0 w/w%, about 17.1 w/w%, about 17.2 w/w%, about 17.3 w/w%, about 17.4 w/w%, about 17.5 w/w%, about 17.6 w/w%, about 17.7 w/w%, about 17.8 w/w%, about 17.9 w/w%, about 18.0 w/w%, about 18.1 w/w%, about 18.2 w/w%, about 18.3 w/w%, about 18.4 w/w%, about 18.5 w/w%, about 18.6 w/w%, about 18.7 w/w%, about 18.8 w/w%, about 18.9 w/w%, about 19.0 w/w%, about 19.1 w/w%, about 19.2 w/w%, about 19.3 w/w%, about 19.4 w/w%, about 19.5 w/w%, about 19.6 w/w%, about 19.7 w/w%, about 19.8 w/w%, about 19.9 w/w%, about 20.0 w/w%, about 20.1 w/w%, about 20.2 w/w%, about 20.3 w/w%, about 20.4 w/w%, about 20.5 w/w%, about 20.6 w/w%, about 20.7 w/w%, about 20.8 w/w%, about 20.9 w/w%, about 21.0 w/w%, about 21.1 w/w%, about 21.2 w/w%, about 21.3 w/w%, about 21.4 w/w%, about 21.5 w/w%, about 21.6 w/w%, about 21.7 w/w%, about 21.8 w/w%, about 21.9 w/w%, about 22.0 w/w%, about 22.1 w/w%, about 22.2 w/w%, about 22.3 w/w%, about 22.4 w/w%, about 22.5 w/w%, about 22.6 w/w%, about 22.7 w/w%, about 22.8 w/w%, about 22.9 w/w%, about 23.0 w/w%, about 23.1 w/w%, about 23.2 w/w%, about 23.3 w/w%, about 23.4 w/w%, about 23.5 w/w%, about 23.6 w/w%, about 23.7 w/w%, about 23.8 w/w%, about 23.9 w/w%, about 24.0 w/w%, about 24.1 w/w%, about 24.2 w/w%, about 24.3 w/w%, about 24.4 w/w%, about 24.5 w/w%, about 24.6 w/w%, about 24.7 w/w%, about 24.8 w/w%, about 24.9 w/w%, about 25.0 w/w%, about 25.1 w/w%, about 25.2 w/w%, about 25.3 w/w%, about 25.4 w/w%, about 25.5 w/w%, about 25.6 w/w%, about 25.7 w/w%, about 25.8 w/w%, about 25.9 w/w%, about 26.0 w/w%, about 26.1 w/w%, about 26.2 w/w%, about 26.3 w/w%, about 26.4 w/w%, about 26.5 w/w%, about 26.6 w/w%, about 26.7 w/w%, about 26.8 w/w%, about 26.9 w/w%, about 27.0 w/w%, about 27.1 w/w%, about 27.2 w/w%, about 27.3 w/w%, about 27.4 w/w%, about 27.5 w/w%, about 27.6 w/w%, about 27.7 w/w%, about 27.8 w/w%, about 27.9 w/w%, about 28.0 w/w%, about 28.1 w/w%, about 28.2 w/w%, about 28.3 w/w%, about 28.4 w/w%, about 28.5 w/w%, about 28.6 w/w%, about 28.7 w/w%, about 28.8 w/w%, about 28.9 w/w%, or about 29.0 w/w%. In some embodiments, the amount of the sodium salt of the compound of Formula **(Ia)** in the solution comprising a sodium salt of the compound of Formula **(Ia),** PEG 300, poloxamer 188, and water is about 6.97 w/w%. In some embodiments, the amount of the sodium salt of the compound of Formula **(Ia)** in the solution comprising a sodium salt of the compound of Formula **(Ia),** PEG 300, poloxamer 188, and water is about 7 w/w%. In some embodiments, the amount of the sodium salt of the compound of Formula **(Ia)** in the solution comprising a sodium salt of the compound of Formula **(Ia),** PEG 300, poloxamer 188, and water is about 9.2 w/w%. In some embodiments, the amount of the sodium salt of the compound of Formula **(Ia)** in the solution comprising a sodium salt of the compound of Formula **(Ia),** PEG 300, poloxamer 188, and water is about 9.23 w/w%. In some embodiments, the amount of the sodium salt of the compound of Formula **(Ia)** in the solution comprising a sodium salt of the compound of Formula **(Ia),** PEG 300, poloxamer 188, and water is about 11.48 w/w%. In some embodiments, the amount of the sodium salt of the compound of Formula **(Ia)** in the solution comprising a sodium salt of the compound of Formula **(Ia),** PEG 300, poloxamer 188, and water is about 11.5 w/w%. In some embodiments, the amount of the sodium salt of the compound of Formula **(Ia)** in the solution comprising a sodium salt of the compound of Formula **(Ia),** PEG 300, poloxamer 188, and water is about 13.7 w/w%. In some embodiments, the amount of the sodium salt of the compound of Formula **(Ia)** in the solution comprising a sodium salt of the compound of Formula **(Ia),** PEG 300, poloxamer 188, and water is about 13.70 w/w%. In some embodiments, the amount of the sodium salt of the compound of Formula **(Ia)** in the solution comprising a sodium salt of the compound of Formula **(Ia),** PEG 300, poloxamer 188, and water is about 26.47 w/w%. In some embodiments, the amount of the sodium salt of the compound of Formula **(Ia)** in the solution comprising a sodium salt of the compound of Formula **(Ia),** PEG 300, poloxamer 188, and water is about 26.5 w/w%.

In some embodiments, the amount of poloxamer 188 in the solution comprising a sodium salt of the compound of Formula **(Ia),** PEG 300, poloxamer 188, and water is about 0.1 w/w% to about 10 w/w%. In some embodiments, the amount of poloxamer 188 in the solution comprising a sodium salt of the compound of Formula **(Ia),** PEG 300, poloxamer 188, and water is about 0.3 w/w% to about 8 w/w%. In some embodiments, the amount of poloxamer 188 in the solution comprising a sodium salt of the compound of Formula **(Ia),** PEG 300, poloxamer 188, and water is about 0.5 w/w% to about 7 w/w%. In some embodiments, the amount of poloxamer 188 in the solution comprising a sodium salt of the compound of Formula **(Ia),** PEG 300, poloxamer 188, and water is about 0.6 w/w% to about 7 w/w%. In some embodiments, the amount of poloxamer 188 in the solution comprising a sodium salt of the compound of Formula **(Ia),** PEG 300, poloxamer 188, and water is about 0.85 w/w% to about 4.82 w/w%. In some embodiments, the amount of poloxamer 188 in the solution comprising a sodium salt of the compound of Formula **(Ia),** PEG 300, poloxamer 188, and water is about 0.9 w/w% to about 4.8 w/w%. In some embodiments, the amount of poloxamer 188 in the solution comprising a sodium salt of the compound of Formula **(Ia),** PEG 300, poloxamer 188, and water is about 0.5 w/w%, about 0.6 w/w%, about 0.7 w/w%, about 0.8 w/w%, about 0.9 w/w%, about 1.0 w/w%, about 1.1 w/w%, about 1.2 w/w%, about 1.3 w/w%, about 1.4 w/w%, about 1.5 w/w%, about 1.6 w/w%, about 1.7 w/w%, about 1.8 w/w%, about 1.9 w/w%, about 2.0 w/w%, about 2.1 w/w%, about 2.2 w/w%, about 2.3 w/w%, about 2.4 w/w%, about 2.5 w/w%, about 2.6 w/w%, about 2.7 w/w%, about 2.8 w/w%, about 2.9 w/w%, about 3.0 w/w%, about 3.1 w/w%, about 3.2 w/w%, about 3.3 w/w%, about 3.4 w/w%, about 3.5 w/w%, about 3.6 w/w%, about 3.7 w/w%, about 3.8 w/w%, about 3.9 w/w%, about 4.0 w/w%, about 4.1 w/w%, about 4.2 w/w%, about 4.3 w/w%, about 4.4 w/w%, about 4.5 w/w%, about 4.6 w/w%, about 4.7 w/w%, about 4.8 w/w%, about 4.9 w/w%, about 5.0 w/w%, about 5.1 w/w%, about 5.2 w/w%, about 5.3 w/w%, about 5.4 w/w%, about 5.5 w/w%, about 5.6 w/w%, about 5.7 w/w%, about 5.8 w/w%, about 5.9 w/w%, about 6.0 w/w%, about 6.1 w/w%, about 6.2 w/w%, about 6.3 w/w%, about 6.4 w/w%, about 6.5 w/w%, about 6.6 w/w%, about 6.7 w/w%, about 6.8 w/w%, about 6.9 w/w%, or about 7.0 w/w%. In some embodiments, the amount of poloxamer 188 in the solution comprising a sodium salt of the compound of Formula **(Ia),** PEG 300, poloxamer 188, and water is about 0.85 w/w%. In some embodiments, the amount of poloxamer 188 in the solution comprising a sodium salt of the compound of Formula **(Ia),** PEG 300, poloxamer 188, and water is about 0.9 w/w%. In some embodiments, the amount of poloxamer 188 in the solution comprising a sodium salt of the compound of Formula **(Ia),** PEG 300, poloxamer 188, and water is about 1.27 w/w%. In some embodiments, the amount of poloxamer 188 in the solution comprising a sodium salt of the compound of Formula **(Ia),** PEG 300, poloxamer 188, and water is about 1.3 w/w%. In some embodiments, the amount of poloxamer 188 in the solution comprising a sodium salt of the compound of Formula **(Ia),** PEG 300, poloxamer 188, and water is about 1.68 w/w%. In some embodiments, the amount of poloxamer 188 in the solution comprising a sodium salt of the compound of Formula **(Ia),** PEG 300, poloxamer 188, and water is about 1.7 w/w%. In some embodiments, the amount of poloxamer 188 in the solution comprising a sodium salt of the compound of Formula **(Ia),** PEG 300, poloxamer 188, and water is about 2.09 w/w%. In some embodiments, the amount of poloxamer 188 in the solution comprising a sodium salt of the compound of Formula **(Ia),** PEG 300, poloxamer 188, and water is about 2.1 w/w%. In some embodiments, the amount of poloxamer 188 in the solution comprising a sodium salt of the compound of Formula **(Ia),** PEG 300, poloxamer 188, and water is about 2.49 w/w%. In some embodiments, the amount of poloxamer 188 in the solution comprising a sodium salt of the compound of Formula **(Ia),** PEG 300, poloxamer 188, and water is about 2.5 w/w%. In some embodiments, the amount of poloxamer 188 in the solution comprising a sodium salt of the compound of Formula **(Ia),** PEG 300, poloxamer 188, and water is about 4.8 w/w%. In some embodiments, the amount of poloxamer 188 in the solution comprising a sodium salt of the compound of Formula **(Ia),** PEG 300, poloxamer 188, and water is about 4.82 w/w%.

In some embodiments, the solution comprises about 29.2 w/w% water, about 62.6 w/w% PEG 300, about 7 w/w% of a sodium salt of a compound of Formula **(Ia),** and about 1.3 w/w% of poloxamer 188. In some embodiments, the solution comprises about 29.21 w/w% water, about 62.55 w/w% PEG 300, about 6.97 w/w% of a sodium salt of a compound of Formula **(Ia),** and about 1.27 w/w% of poloxamer 188. In some embodiments, the solution comprises about 28.4 w/w% water, about 60.7 w/w% PEG 300, about 9.2 w/w% of a sodium salt of a compound of Formula **(Ia),** and about 1.7 w/w% of poloxamer 188. In some embodiments, the solution comprises about 28.36 w/w% water, about 60.73 w/w% PEG 300, about 9.23 w/w% of a sodium salt of a compound of Formula **(Ia),** and about 1.68 w/w% of poloxamer 188. In some embodiments, the solution comprises about 27.5 w/w% water, about 58.9 w/w% PEG 300, about 11.5 w/w% of a sodium salt of a compound of Formula **(Ia),** and about 2.1 w/w% of poloxamer 188. In some embodiments, the solution comprises about 27.51 w/w% water, about 58.92 w/w% PEG 300, about 11.48 w/w% of a sodium salt of a compound of Formula **(Ia),** and about 2.09 w/w% of poloxamer 188. In some embodiments, the solution comprises about 26.7 w/w% water, about 57.1 w/w% PEG 300, about 13.7 w/w% of a sodium salt of a compound of Formula **(Ia),** and about 2.5 w/w% of poloxamer 188. In some embodiments, the solution comprises about 26.68 w/w% water, about 57.13 w/w% PEG 300, about 13.70 w/w% of a sodium salt of a compound of Formula **(Ia),** and about 2.49 w/w% of poloxamer 188. In some embodiments, the solution comprises about 21.9 w/w% water, about 46.8 w/w% PEG 300, about 26.5 w/w% of a sodium salt of a compound of Formula **(Ia),** and about 4.8 w/w% of poloxamer 188. In some embodiments, the solution comprises about 21.87 w/w% water, about 46.84 w/w% PEG 300, about 26.47 w/w% of a sodium salt of a compound of Formula **(Ia),** and about 4.82 w/w% of poloxamer 188.

In certain embodiments, the pharmaceutical composition disclosed herein is a parenteral formulation. In certain embodiments, the formulation is administered subcutaneously to a patient in need thereof. In certain embodiments, the formulation is administered intramuscularly to a patient in need thereof.

In certain embodiments, the parenteral formulation comprises N-methyl-2-pyrrolidone (NMP). In certain embodiments, the parenteral formulation comprises dimethyl sulfoxide (DMSO). In some embodiments, the parenteral formulation comprises polyethylene glycol (PEG) or glycofurol. In some embodiments, the solution comprises poloxamer in saline. In some embodiments, the solution comprises 2% poloxamer 188 in normal saline.

In certain embodiments, the parenteral formulation further contains an alcohol. In certain embodiments, the alcohol is ethanol. In certain embodiments, the parenteral formulation further contains an inorganic base. In certain embodiments, the inorganic base is sodium hydroxide (NaOH). In certain embodiments, the inorganic base is sodium ethoxide (NaOEt). In certain embodiments, the formulation comprises from about 0.1 molar equivalents to about 1.5 molar equivalents of the inorganic base. In certain embodiments, the formulation comprises from about 0.5 molar equivalents to about 1.5 molar equivalents of the inorganic base. In certain embodiments, the formulation comprises from about 0.75 molar equivalents to about 1.2 molar equivalents of the inorganic base. In certain embodiments, the formulation comprises about 1.0 molar equivalents inorganic base. In certain embodiments, the formulation comprises about 1.2 molar equivalents inorganic base. In some embodiments, the inorganic base is NaOH or NaOEt.

The parenteral formulation comprises the sodium salt of the compound of Formula **(Ia)** water and polyethylene glycol PEG 300.

In certain embodiments, the parenteral formulation comprises the sodium salt of the compound of Formula **(Ia)** water, polyethylene glycol PEG 300 (polyethylene glycol with an average molecular weight of 300 g/mol), and NaOH. In certain embodiments, the parenteral formulation comprises the sodium salt of the compound of Formula **(Ia)** water, polyethylene glycol (PEG) 300, and NaOEt. In certain embodiments, the formulation includes from about 0.1 molar equivalents to about 1.5 molar equivalents of NaOH or NaOEt. In certain embodiments, the formulation includes from about 0.5 molar equivalents to about 1.5 molar equivalents of NaOH or NaOEt. In certain embodiments, the formulation includes from about 0.75 molar equivalents to about 1.2 molar equivalents of NaOH or NaOEt. In certain embodiments, the formulation comprises about 1.0 molar equivalents of NaOH or NaOEt. In certain embodiments, the formulation includes about 1.2 molar equivalents of NaOH or NaOEt.

In some embodiments, the solution formulation further includes an inorganic base. In certain embodiments, the formulation includes from about 0.1 molar equivalents to about 1.5 molar equivalents of the inorganic base. In certain embodiments, the formulation comprises from about 0.5 molar equivalents to about 1.5 molar equivalents of the inorganic base. In certain embodiments, the formulation comprises from about 0.75 molar equivalents to about 1.2 molar equivalents of the inorganic base. In certain embodiments, the formulation comprises about 1.0 molar equivalents inorganic base. In certain embodiments, the formulation comprises about 1.2 molar equivalents inorganic base. In certain embodiments, the inorganic base is sodium hydroxide or sodium ethoxide. In certain embodiments, the inorganic base is sodium hydroxide.

The solution according to the present invention comprises a sodium salt of the compound of Formula **(Ia),** PEG 300, sodium hydroxide, and water.

In some embodiments, the concentration of the compound of Formula **(Ia)** in the solution comprising a sodium salt of the compound of Formula **(Ia),** PEG 300, sodium hydroxide, and water is about 50 mg/ml to about 300 mg/ml. In some embodiments, the concentration of the compound of Formula **(Ia)** in the solution comprising a sodium salt of the compound of Formula **(Ia),** PEG 300, sodium hydroxide, and water is about 75 mg/ml to about 300 mg/ml. In some embodiments, the concentration of the compound of Formula **(Ia)** in the solution comprising a sodium salt of the compound of Formula **(Ia),** PEG 300, sodium hydroxide, and water is about 50 mg/ml. In some embodiments, the concentration of the compound of Formula **(Ia)** in the solution comprising a sodium salt of the compound of Formula **(Ia),** PEG 300, sodium hydroxide, and water is about 75 mg/ml. In some embodiments, the concentration of the compound of Formula **(Ia)** in the solution comprising a sodium salt of the compound of Formula **(Ia),** PEG 300, sodium hydroxide, and water is about 100 mg/ml. In some embodiments, the concentration of the compound of Formula **(Ia)** in the solution comprising a sodium salt of the compound of Formula **(Ia),** PEG 300, sodium hydroxide, and water is about 125 mg/ml. In some embodiments, the concentration of the compound of Formula **(Ia)** in the solution comprising a sodium salt of the compound of Formula **(Ia),** PEG 300, sodium hydroxide, and water is about 150 mg/ml. In some embodiments, the concentration of the compound of Formula **(Ia)** in the solution comprising a sodium salt of the compound of Formula **(Ia),** PEG 300, sodium hydroxide, and water is about 175 mg/ml. In some embodiments, the concentration of the compound of Formula **(Ia)** in the solution comprising a sodium salt of the compound of Formula **(Ia),** PEG 300, sodium hydroxide, and water is about 200 mg/ml. In some embodiments, the concentration of the compound of Formula **(Ia)** in the solution comprising a sodium salt of the compound of Formula **(Ia),** PEG 300, sodium hydroxide, and water is about 225 mg/ml. In some embodiments, the concentration of the compound of Formula **(Ia)** in the solution comprising a sodium salt of the compound of Formula **(Ia),** PEG 300, sodium hydroxide, and water is about 250 mg/ml. In some embodiments, the concentration of the compound of Formula **(Ia)** in the solution comprising a sodium salt of the compound of Formula **(Ia),** PEG 300, sodium hydroxide, and water is about 275 mg/ml. In some embodiments, the concentration of the compound of Formula **(Ia)** in the solution comprising a sodium salt of the compound of Formula **(Ia),** PEG 300, sodium hydroxide, and water is about 300 mg/ml. In some embodiments, the concentration of the compound of Formula **(Ia)** in the solution comprising a sodium salt of the compound of Formula **(Ia),** PEG 300, sodium hydroxide, and water is about 325 mg/ml. In some embodiments, the concentration of the compound of Formula **(Ia)** in the solution comprising a sodium salt of the compound of Formula **(Ia),** PEG 300, sodium hydroxide, and water is about 350 mg/ml.

The amount of water in the solution comprising the sodium salt of the compound of Formula **(Ia),** PEG 300, sodium hydroxide, and water is about 10 w/w% to about 40 w/w%. In some embodiments, the amount of water in the solution comprising the sodium salt of the compound of Formula **(Ia),** PEG 300, sodium hydroxide, and water is about 15 w/w% to about 35 w/w%. In some embodiments, the amount of water in the solution comprising the sodium salt of the compound of Formula **(Ia),** PEG 300, sodium hydroxide, and water is about 20 w/w% to about 30 w/w%. In some embodiments, the amount of water in the solution comprising the sodium salt of the compound of Formula **(Ia),** PEG 300, sodium hydroxide, and water is about 21 w/w% to about 29 w/w%. In some embodiments, the amount of water in the solution comprising the sodium salt of the compound of Formula **(Ia),** PEG 300, sodium hydroxide, and water is about 23.2 w/w% to about 27.9 w/w%. In some embodiments, the amount of water in the solution comprising the sodium salt of the compound of Formula **(Ia),** PEG 300, sodium hydroxide, and water is about 23.2 w/w% to about 27.92 w/w%. In some embodiments, the amount of water in the solution comprising the sodium salt of the compound of Formula **(Ia),** PEG 300, sodium hydroxide, and water is about 22.0 w/w%, about 22.1 w/w%, about 22.2 w/w%, about 22.3 w/w%, about 22.4 w/w%, about 22.5 w/w%, about 22.6 w/w%, about 22.7 w/w%, about 22.8 w/w%, about 22.9 w/w%, about 23.0 w/w%, about 23.1 w/w%, about 23.2 w/w%, about 23.3 w/w%, about 23.4 w/w%, about 23.5 w/w%, about 23.6 w/w%, about 23.7 w/w%, about 23.8 w/w%, about 23.9 w/w%, about 24.0 w/w%, about 24.1 w/w%, about 24.2 w/w%, about 24.3 w/w%, about 24.4 w/w%, about 24.5 w/w%, about 24.6 w/w%, about 24.7 w/w%, about 24.8 w/w%, about 24.9 w/w%, about 25.0 w/w%, about 25.1 w/w%, about 25.2 w/w%, about 25.3 w/w%, about 25.4 w/w%, about 25.5 w/w%, about 25.6 w/w%, about 25.7 w/w%, about 25.8 w/w%, about 25.9 w/w%, about 26.0 w/w%, about 26.1 w/w%, about 26.2 w/w%, about 26.3 w/w%, about 26.4 w/w%, about 26.5 w/w%, about 26.6 w/w%, about 26.7 w/w%, about 26.8 w/w%, about 26.9 w/w%, about 27.0 w/w%, about 27.1 w/w%, about 27.2 w/w%, about 27.3 w/w%, about 27.4 w/w%, about 27.5 w/w%, about 27.6 w/w%, about 27.7 w/w%, about 27.8 w/w%, about 27.9 w/w%, about 28.0 w/w%, about 28.1 w/w%, about 28.2 w/w%, about 28.3 w/w%, about 28.4 w/w%, about 28.5 w/w%, about 28.6 w/w%, about 28.7 w/w%, about 28.8 w/w%, about 28.9 w/w%, or about 29.0 w/w%. In some embodiments, the amount of water in the solution comprising the sodium salt of the compound of Formula **(Ia),** PEG 300, sodium hydroxide, and water is about 23.2 w/w%. In some embodiments, the amount of water in the solution comprising the sodium salt of the compound of Formula **(Ia),** PEG 300, sodium hydroxide, and water is about 27.9 w/w%. In some embodiments, the amount of water in the solution comprising the sodium salt of the compound of Formula **(Ia),** PEG 300, sodium hydroxide, and water is about 27.92 w/w%.

The amount of PEG 300 in the solution comprising the sodium salt of the compound of Formula **(Ia),** PEG 300, sodium hydroxide, and water is about 35 w/w% to about 75 w/w%. In some embodiments, the amount of PEG 300 in the solution comprising the sodium salt of the compound of Formula **(Ia),** PEG 300, sodium hydroxide, and water is about 45 w/w% to about 65 w/w%. In some embodiments, the amount of PEG 300 in the solution comprising the sodium salt of the compound of Formula **(Ia),** PEG 300, sodium hydroxide, and water is about 48 w/w% to about 60 w/w%. In some embodiments, the amount of PEG 300 in the solution comprising the sodium salt of the compound of Formula **(Ia),** PEG 300, sodium hydroxide, and water is about 49 w/w% to about 59 w/w%. In some embodiments, the amount of PEG 300 in the solution comprising the sodium salt of the compound of Formula **(Ia),** PEG 300, sodium hydroxide, and water is about 50.0 w/w% to about 58.0 w/w%. In some embodiments, the amount of PEG 300 in the solution comprising the sodium salt of the compound of Formula **(Ia),** PEG 300, sodium hydroxide, and water is about 50.0 w/w% to about 58.04 w/w%. In some embodiments, the amount of PEG 300 in the solution comprising the sodium salt of the compound of Formula **(Ia),** PEG 300, sodium hydroxide, and water is about 45 w/w%, about 46 w/w%, about 47 w/w%, about 48 w/w%, about 49 w/w%, about 50 w/w%, about 51 w/w%, about 52 w/w%, about 53 w/w%, about 54 w/w%, about 55 w/w%, about 56 w/w%, about 57 w/w%, about 58 w/w%, about 59 w/w%, about 60 w/w%, about 61 w/w%, about 62 w/w%, about 63 w/w%, about 64 w/w%, or about 65 w/w%. In some embodiments, the amount of PEG 300 in the solution comprising the sodium salt of the compound of Formula **(Ia),** PEG 300, sodium hydroxide, and water is about 50.0 w/w%. In some embodiments, the amount of PEG 300 in the solution comprising the sodium salt of the compound of Formula **(Ia),** PEG 300, sodium hydroxide, and water is about 58.0 w/w%. In some embodiments, the amount of PEG 300 in the solution comprising the sodium salt of the compound of Formula **(Ia),** PEG 300, sodium hydroxide, and water is about 58.04 w/w%.

The amount of the compound of Formula **(Ia)** in the solution comprising a compound of Formula **(Ia),** PEG 300, sodium hydroxide, and water is about 5 w/w% to about 35 w/w%. In some embodiments, the amount of the compound of Formula **(Ia)** in the solution comprising a compound of Formula **(Ia),** PEG 300, sodium hydroxide, and water is about 10 w/w% to about 30 w/w%. In some embodiments, the amount of the compound of Formula **(Ia)** in the solution comprising a compound of Formula **(Ia),** PEG 300, sodium hydroxide, and water is about 11 w/w% to about 28 w/w%. In some embodiments, the amount of the compound of Formula **(Ia)** in the solution comprising a compound of Formula **(Ia),** PEG 300, sodium hydroxide, and water is about 13 w/w% to about 27 w/w%. In some embodiments, the amount of the compound of Formula **(Ia)** in the solution comprising a compound of Formula **(Ia),** PEG 300, sodium hydroxide, and water is about 13.5 w/w% to about 25.7 w/w%. In some embodiments, the amount of the compound of Formula **(Ia)** in the solution comprising a compound of Formula **(Ia),** PEG 300, sodium hydroxide, and water is about 13.47 w/w% to about 25.7 w/w%. In some embodiments, the amount of the compound of Formula **(Ia)** in the solution comprising a compound of Formula **(Ia),** PEG 300, sodium hydroxide, and water is about 13.0 w/w%, about 13.1 w/w%, about 13.2 w/w%, about 13.3 w/w%, about 13.4 w/w%, about 13.5 w/w%, about 13.6 w/w%, about 13.7 w/w%, about 13.8 w/w%, about 13.9 w/w%, about 14.0 w/w%, about 14.1 w/w%, about 14.2 w/w%, about 14.3 w/w%, about 14.4 w/w%, about 14.5 w/w%, about 14.6 w/w%, about 14.7 w/w%, about 14.8 w/w%, about 14.9 w/w%, about 15.0 w/w%, about 15.1 w/w%, about 15.2 w/w%, about 15.3 w/w%, about 15.4 w/w%, about 15.5 w/w%, about 15.6 w/w%, about 15.7 w/w%, about 15.8 w/w%, about 15.9 w/w%, about 16.0 w/w%, about 16.1 w/w%, about 16.2 w/w%, about 16.3 w/w%, about 16.4 w/w%, about 16.5 w/w%, about 16.6 w/w%, about 16.7 w/w%, about 16.8 w/w%, about 16.9 w/w%, about 17.0 w/w%, about 17.1 w/w%, about 17.2 w/w%, about 17.3 w/w%, about 17.4 w/w%, about 17.5 w/w%, about 17.6 w/w%, about 17.7 w/w%, about 17.8 w/w%, about 17.9 w/w%, about 18.0 w/w%, about 18.1 w/w%, about 18.2 w/w%, about 18.3 w/w%, about 18.4 w/w%, about 18.5 w/w%, about 18.6 w/w%, about 18.7 w/w%, about 18.8 w/w%, about 18.9 w/w%, about 19.0 w/w%, about 19.1 w/w%, about 19.2 w/w%, about 19.3 w/w%, about 19.4 w/w%, about 19.5 w/w%, about 19.6 w/w%, about 19.7 w/w%, about 19.8 w/w%, about 19.9 w/w%, about 20.0 w/w%, about 21.1 w/w%, about 21.2 w/w%, about 21.3 w/w%, about 21.4 w/w%, about 21.5 w/w%, about 21.6 w/w%, about 21.7 w/w%, about 21.8 w/w%, about 21.9 w/w%, about 22.0 w/w%, about 22.1 w/w%, about 22.2 w/w%, about 22.3 w/w%, about 22.4 w/w%, about 22.5 w/w%, about 22.6 w/w%, about 22.7 w/w%, about 22.8 w/w%, about 22.9 w/w%, about 23.0 w/w%, about 23.1 w/w%, about 23.2 w/w%, about 23.3 w/w%, about 23.4 w/w%, about 23.5 w/w%, about 23.6 w/w%, about 23.7 w/w%, about 23.8 w/w%, about 23.9 w/w%, about 24.0 w/w%, about 24.1 w/w%, about 24.2 w/w%, about 24.3 w/w%, about 24.4 w/w%, about 24.5 w/w%, about 24.6 w/w%, about 24.7 w/w%, about 24.8 w/w%, about 24.9 w/w%, about 25.0 w/w%, about 25.1 w/w%, about 25.2 w/w%, about 25.3 w/w%, about 25.4 w/w%, about 25.5 w/w%, about 25.6 w/w%, about 25.7 w/w%, about 25.8 w/w%, about 25.9 w/w%, about 26.0 w/w%, about 26.1 w/w%, about 26.2 w/w%, about 26.3 w/w%, about 26.4 w/w%, about 26.5 w/w%, about 26.6 w/w%, about 26.7 w/w%, about 26.8 w/w%, about 26.9 w/w%, about 27.0 w/w%, about 27.1 w/w%, about 27.2 w/w%, about 27.3 w/w%, about 27.4 w/w%, about 27.5 w/w%, about 27.6 w/w%, about 27.7 w/w%, about 27.8 w/w%, about 27.9 w/w%, about 28.0 w/w%, about 28.1 w/w%, about 28.2 w/w%, about 28.3 w/w%, about 28.4 w/w%, about 28.5 w/w%, about 28.6 w/w%, about 28.7 w/w%, about 28.8 w/w%, about 28.9 w/w%, or about 29.0 w/w%. In some embodiments, the amount of the compound of Formula **(Ia)** in the solution comprising a compound of Formula **(Ia),** PEG 300, sodium hydroxide, and water is about 13.47 w/w%. In some embodiments, the amount of the compound of Formula **(Ia)** in the solution comprising a compound of Formula **(Ia),** PEG 300, sodium hydroxide, and water is about 13.5 w/w%. In some embodiments, the amount of the compound of Formula **(Ia)** in the solution comprising a compound of Formula **(Ia),** PEG 300, sodium hydroxide, and water is about 25.7 w/w%.

In some embodiments, the amount of sodium hydroxide in the solution comprising a compound of Formula **(Ia),** PEG 300, sodium hydroxide, and water is about 0.05 w/w% to about 2 w/w%. In some embodiments, the amount of sodium hydroxide in the solution comprising a compound of Formula **(Ia),** PEG 300, sodium hydroxide, and water is about 0.1 w/w% to about 1.5 w/w%. In some embodiments, the amount of sodium hydroxide in the solution comprising a compound of Formula **(Ia),** PEG 300, sodium hydroxide, and water is about 0.3 w/w% to about 1.3 w/w%. In some embodiments, the amount of sodium hydroxide in the solution comprising a compound of Formula **(Ia),** PEG 300, sodium hydroxide, and water is about 0.5 w/w% to about 1.2 w/w%. In some embodiments, the amount of sodium hydroxide in the solution comprising a compound of Formula **(Ia),** PEG 300, sodium hydroxide, and water is about 0.6 w/w% to about 1.1 w/w%. In some embodiments, the amount of sodium hydroxide in the solution comprising a compound of Formula **(Ia),** PEG 300, sodium hydroxide, and water is about 0.58 w/w% to about 1.1 w/w%. In some embodiments, the amount of sodium hydroxide in the solution comprising a compound of Formula **(Ia),** PEG 300, sodium hydroxide, and water is about 0.1 w/w%, about 0.2 w/w%, about 0.3 w/w%, about 0.4 w/w%, about 0.5 w/w%, about 0.6 w/w%, about 0.7 w/w%, about 0.8 w/w%, about 0.9 w/w%, about 1.0 w/w%, about 1.1 w/w%, about 1.2 w/w%, about 1.3 w/w%, about 1.4 w/w%, about 1.5 w/w%, about 1.6 w/w%, about 1.7 w/w%, about 1.8 w/w%, about 1.9 w/w%, or about 2.0 w/w%. In some embodiments, the amount of sodium hydroxide in the solution comprising a compound of Formula **(Ia),** PEG 300, sodium hydroxide, and water is about 0.58 w/w%. In some embodiments, the amount of sodium hydroxide in the solution comprising a compound of Formula **(Ia),** PEG 300, sodium hydroxide, and water is about 0.6 w/w%. In some embodiments, the amount of sodium hydroxide in the solution comprising a compound of Formula **(Ia),** PEG 300, sodium hydroxide, and water is about 1.1 w/w%. In some embodiments, the solution disclosed herein comprises about 10 w/w% to about 40 w/w% water, about 35 w/w% to about 75 w/w% PEG 300, about 5 w/w% to about 35 w/w% of a compound of Formula **(Ia),** and about 0.05 w/w% to about 2 w/w% of sodium hydroxide. In some embodiments, the solution comprises about 15 w/w% to about 35 w/w% water, about 45 w/w% to about 65 w/w% PEG 300, about 10 w/w% to about 30 w/w% of a compound of Formula **(Ia),** and about 0.1 w/w% to about 1.5 w/w% of sodium hydroxide. In some embodiments, the solution comprises about 20 w/w% to about 30 w/w% water, about 48 w/w% to about 60 w/w% PEG 300, about 11 w/w% to about 28 w/w% of a compound of Formula **(Ia),** and about 0.3 w/w% to about 1.3 w/w% of sodium hydroxide. In some embodiments, the solution comprises about 21 w/w% to about 29 w/w% water, about 49 w/w% to about 59 w/w% PEG 300, about 13 w/w% to about 27 w/w% of a compound of Formula **(Ia),** and about 0.5 w/w% to about 1.2 w/w% of sodium hydroxide. In some embodiments, the solution comprises about 23.2 w/w% to about 27.9 w/w% water, about 50 w/w% to about 58 w/w% PEG 300, about 13.5 w/w% to about 25.7 w/w% of a compound of Formula **(Ia),** and about 0.6 w/w% to about 1.1 w/w% of sodium hydroxide. In some embodiments, the solution comprises about 23.2 w/w% to about 27.92 w/w% water, about 50.0 w/w% to about 58.04 w/w% PEG 300, about 13.47 w/w% to about 25.7 w/w% of a compound of Formula **(Ia),** and about 0.58 w/w% to about 1.1 w/w% of sodium hydroxide. In some embodiments, the solution comprises about 27.9 w/w% water, about 58 w/w% PEG 300, about 13.5 w/w% of a compound of Formula **(Ia),** and about 0.6 w/w% of sodium hydroxide. In some embodiments, the solution comprises about 27.92 w/w% water, about 58.04 w/w% PEG 300, about 13.47 w/w% of a compound of Formula **(Ia),** and about 0.58 w/w% of sodium hydroxide. In some embodiments, the solution comprises about 23.2 w/w% water, about 50.0 w/w% PEG 300, about 25.7 w/w% of a compound of Formula **(Ia),** and about 1.1 w/w% of sodium hydroxide.

In some embodiments, the solution disclosed herein comprises a sodium salt of the compound of Formula **(Ia),** PEG 300, sodium hydroxide, poloxamer 188, and water.

In some embodiments, the concentration of the compound of Formula **(Ia)** in the solution comprising a sodium salt of the compound of Formula **(Ia),** PEG 300, sodium hydroxide, poloxamer 188, and water is about 50 mg/ml to about 300 mg/ml. In some embodiments, the concentration of the compound of Formula **(Ia)** in the solution comprising a sodium salt of the compound of Formula **(Ia),** PEG 300, sodium hydroxide, poloxamer 188, and water is about 75 mg/ml to about 300 mg/ml. In some embodiments, the concentration of the compound of Formula **(Ia)** in the solution comprising a sodium salt of the compound of Formula **(Ia),** PEG 300, sodium hydroxide, poloxamer 188, and water is about 50 mg/ml. In some embodiments, the concentration of the compound of Formula **(Ia)** in the solution comprising a sodium salt of the compound of Formula **(Ia),** PEG 300, sodium hydroxide, poloxamer 188, and water is about 75 mg/ml. In some embodiments, the concentration of the compound of Formula **(Ia)** in the solution comprising a sodium salt of the compound of Formula **(Ia),** PEG 300, sodium hydroxide, poloxamer 188, and water is about 100 mg/ml. In some embodiments, the concentration of the compound of Formula **(Ia)** in the solution comprising a sodium salt of the compound of Formula **(Ia),** PEG 300, sodium hydroxide, poloxamer 188, and water is about 125 mg/ml. In some embodiments, the concentration of the compound of Formula **(Ia)** in the solution comprising a sodium salt of the compound of Formula **(Ia),** PEG 300, sodium hydroxide, poloxamer 188, and water is about 150 mg/ml. In some embodiments, the concentration of the compound of Formula **(Ia)** in the solution comprising a sodium salt of the compound of Formula **(Ia),** PEG 300, sodium hydroxide, poloxamer 188, and water is about 175 mg/ml. In some embodiments, the concentration of the compound of Formula **(Ia)** in the solution comprising a sodium salt of the compound of Formula **(Ia),** PEG 300, sodium hydroxide, poloxamer 188, and water is about 200 mg/ml. In some embodiments, the concentration of the compound of Formula **(Ia)** in the solution comprising a sodium salt of the compound of Formula **(Ia),** PEG 300, sodium hydroxide, poloxamer 188, and water is about 225 mg/ml. In some embodiments, the concentration of the compound of Formula **(Ia)** in the solution comprising a sodium salt of the compound of Formula **(Ia),** PEG 300, sodium hydroxide, poloxamer 188, and water is about 250 mg/ml. In some embodiments, the concentration of the compound of Formula **(Ia)** in the solution comprising a sodium salt of the compound of Formula **(Ia),** PEG 300, sodium hydroxide, poloxamer 188, and water is about 275 mg/ml. In some embodiments, the concentration of the compound of Formula **(Ia)** in the solution comprising a sodium salt of the compound of Formula **(Ia),** PEG 300, sodium hydroxide, poloxamer 188, and water is about 300 mg/ml. In some embodiments, the concentration of the compound of Formula **(Ia)** in the solution comprising a sodium salt of the compound of Formula **(Ia),** PEG 300, sodium hydroxide, poloxamer 188, and water is about 325 mg/ml. In some embodiments, the concentration of the compound of Formula **(Ia)** in the solution comprising a sodium salt of the compound of Formula **(Ia),** PEG 300, sodium hydroxide, poloxamer 188, and water is about 350 mg/ml.

In some embodiments, the amount of water in the solution comprising the sodium salt of the compoundof Formula **(Ia),** PEG 300, sodium hydroxide, poloxamer 188, and water is about 15 w/w% to about 35 w/w%. In some embodiments, the amount of water in the solution comprising the sodium salt of the compound of Formula **(Ia),** PEG 300, sodium hydroxide, poloxamer 188, and water is about 20 w/w% to about 35 w/w%. In some embodiments, the amount of water in the solution comprising the sodium salt of the compound of Formula **(Ia),** PEG 300, sodium hydroxide, poloxamer 188, and water is about 20 w/w% to about 31 w/w%. In some embodiments, the amount of water in the solution comprising the sodium salt of the compound of Formula **(Ia),** PEG 300, sodium hydroxide, poloxamer 188, and water is about 22 w/w% to about 30.1 w/w%. In some embodiments, the amount of water in the solution comprising the sodium salt of the compound of Formula **(Ia),** PEG 300, sodium hydroxide, poloxamer 188, and water is about 21.97 w/w% to about 30.07 w/w%. In some embodiments, the amount of water in the solution comprising the sodium salt of the compound of Formula **(Ia),** PEG 300, sodium hydroxide, poloxamer 188, and water is about 19.0 w/w%, about 19.1 w/w%, about 19.2 w/w%, about 19.3 w/w%, about 19.4 w/w%, about 19.5 w/w%, about 19.6 w/w%, about 19.7 w/w%, about 19.8 w/w%, about 19.9 w/w%, about 20.0 w/w%, about 20.1 w/w%, about 20.2 w/w%, about 20.3 w/w%, about 20.4 w/w%, about 20.5 w/w%, about 20.6 w/w%, about 20.7 w/w%, about 20.8 w/w%, about 20.9 w/w%, about 21.0 w/w%, about 21.1 w/w%, about 21.2 w/w%, about 21.3 w/w%, about 21.4 w/w%, about 21.5 w/w%, about 21.6 w/w%, about 21.7 w/w%, about 21.8 w/w%, about 21.9 w/w%, about 22.0 w/w%, about 22.1 w/w%, about 22.2 w/w%, about 22.3 w/w%, about 22.4 w/w%, about 22.5 w/w%, about 22.6 w/w%, about 22.7 w/w%, about 22.8 w/w%, about 22.9 w/w%, about 23.0 w/w%, about 23.1 w/w%, about 23.2 w/w%, about 23.3 w/w%, about 23.4 w/w%, about 23.5 w/w%, about 23.6 w/w%, about 23.7 w/w%, about 23.8 w/w%, about 23.9 w/w%, about 24.0 w/w%, about 24.1 w/w%, about 24.2 w/w%, about 24.3 w/w%, about 24.4 w/w%, about 24.5 w/w%, about 24.6 w/w%, about 24.7 w/w%, about 24.8 w/w%, about 24.9 w/w%, about 25.0 w/w%, about 25.1 w/w%, about 25.2 w/w%, about 25.3 w/w%, about 25.4 w/w%, about 25.5 w/w%, about 25.6 w/w%, about 25.7 w/w%, about 25.8 w/w%, about 25.9 w/w%, about 26.0 w/w%, about 26.1 w/w%, about 26.2 w/w%, about 26.3 w/w%, about 26.4 w/w%, about 26.5 w/w%, about 26.6 w/w%, about 26.7 w/w%, about 26.8 w/w%, about 26.9 w/w%, about 27.0 w/w%, about 27.1 w/w%, about 27.2 w/w%, about 27.3 w/w%, about 27.4 w/w%, about 27.5 w/w%, about 27.6 w/w%, about 27.7 w/w%, about 27.8 w/w%, about 27.9 w/w%, about 28.0 w/w%, about 28.1 w/w%, about 28.2 w/w%, about 28.3 w/w%, about 28.4 w/w%, about 28.5 w/w%, about 28.6 w/w%, about 28.7 w/w%, about 28.8 w/w%, about 28.9 w/w%, about 29.0 w/w%, about 29.1 w/w%, about 29.2 w/w%, about 29.3 w/w%, about 29.4 w/w%, about 29.5 w/w%, about 29.6 w/w%, about 29.7 w/w%, about 29.8 w/w%, about 29.9 w/w%, about 30.0 w/w%, about 30.1 w/w%, about 30.2 w/w%, about 30.3 w/w%, about 30.4 w/w%, about 30.5 w/w%, about 30.6 w/w%, about 30.7 w/w%, about 30.8 w/w%, about 30.9 w/w%, about 31.0 w/w%, about 31.1 w/w%, about 31.2 w/w%, about 31.3 w/w%, about 31.4 w/w%, about 31.5 w/w%, about 31.6 w/w%, about 31.7 w/w%, about 31.8 w/w%, about 31.9 w/w%, about 32.0 w/w%, about 32.1 w/w%, about 32.2 w/w%, about 32.3 w/w%, about 32.4 w/w%, about 32.5 w/w%, about 32.6 w/w%, about 32.7 w/w%, about 32.8 w/w%, about 32.9 w/w%, or about 33.0 w/w%. In some embodiments, the amount of water in the solution comprising the sodium salt of the compound of Formula **(Ia),** PEG 300, sodium hydroxide, poloxamer 188, and water is about 21.97 w/w%. In some embodiments, the amount of water in the solution comprising the sodium salt of the compound of Formula **(Ia),** PEG 300, sodium hydroxide, poloxamer 188, and water is about 22 w/w%. In some embodiments, the amount of water in the solution comprising the sodium salt of the compound of Formula **(Ia),** PEG 300, sodium hydroxide, poloxamer 188, and water is about 30.07 w/w%. In some embodiments, the amount of water in the solution comprising the sodium salt of the compound of Formula **(Ia),** PEG 300, sodium hydroxide, poloxamer 188, and water is about 30.1 w/w%.

In some embodiments, the amount of PEG 300 in the solution comprising the sodium salt of the compound of Formula **(Ia),** PEG 300, sodium hydroxide, poloxamer 188, and water is about 40 w/w% to about 70 w/w%. In some embodiments, the amount of PEG 300 in the solution comprising the sodium salt of the compound of Formula **(Ia),** PEG 300, sodium hydroxide, poloxamer 188, and water is about 45 w/w% to about 68 w/w%. In some embodiments, the amount of PEG 300 in the solution comprising the sodium salt of the compound of Formula **(Ia),** PEG 300, sodium hydroxide, poloxamer 188, and water is about 47.1 w/w% to about 64.4 w/w%. In some embodiments, the amount of PEG 300 in the solution comprising the sodium salt of the compound of Formula **(Ia),** PEG 300, sodium hydroxide, poloxamer 188, and water is about 47.05 w/w% to about 64.41 w/w%. In some embodiments, the amount of PEG 300 in the solution comprising the sodium salt of the compound of Formula **(Ia),** PEG 300, sodium hydroxide, poloxamer 188, and water is about 40 w/w%, about 41 w/w%, about 42 w/w%, about 43 w/w%, about 44 w/w%, about 45 w/w%, about 46 w/w%, about 47 w/w%, about 48 w/w%, about 49 w/w%, about 50 w/w%, about 51 w/w%, about 52 w/w%, about 53 w/w%, about 54 w/w%, about 55 w/w%, about 56 w/w%, about 57 w/w%, about 58 w/w%, about 59 w/w%, about 60 w/w%, about 61 w/w%, about 62 w/w%, about 63 w/w%, about 64 w/w%, about 65 w/w%, about 66 w/w%, about 67 w/w%, about 68 w/w%, about 69 w/w%, or about 70 w/w%. In some embodiments, the amount of PEG 300 in the solution comprising the sodium salt of the compound of Formula **(Ia),** PEG 300, sodium hydroxide, poloxamer 188, and water is about 47.05 w/w%. In some embodiments, the amount of PEG 300 in the solution comprising the sodium salt of the compound of Formula **(Ia),** PEG 300, sodium hydroxide, poloxamer 188, and water is about 47.1 w/w%. In some embodiments, the amount of PEG 300 in the solution comprising the sodium salt of the compound of Formula **(Ia),** PEG 300, sodium hydroxide, poloxamer 188, and water is about 64.4 w/w%. In some embodiments, the amount of PEG 300 in the solution comprising the sodium salt of the compound of Formula **(Ia),** PEG 300, sodium hydroxide, poloxamer 188, and water is about 64.41 w/w%.

In some embodiments, the amount of the sodium salt of the compound of Formula **(Ia)** in the solution comprising the sodium salt of the compound of Formula **(Ia),** PEG 300, sodium hydroxide, poloxamer 188, and water is about 5.0 w/w%, about 5.1 w/w%, about 5.2 w/w%, about 5.3 w/w%, about 5.4 w/w%, about 5.5 w/w%, about 5.6 w/w%, about 5.7 w/w%, about 5.8 w/w%, about 5.9 w/w%, about 6.0 w/w%, about 6.1 w/w%, about 6.2 w/w%, about 6.3 w/w%, about 6.4 w/w%, about 6.5 w/w%, about 6.6 w/w%, about 6.7 w/w%, about 6.8 w/w%, about 6.9 w/w%, about 7.0 w/w%, about 7.1 w/w%, about 7.2 w/w%, about 7.3 w/w%, about 7.4 w/w%, about 7.5 w/w%, about 7.6 w/w%, about 7.7 w/w%, about 7.8 w/w%, about 7.9 w/w%, about 8.0 w/w%, about 8.1 w/w%, about 8.2 w/w%, about 8.3 w/w%, about 8.4 w/w%, about 8.5 w/w%, about 8.6 w/w%, about 8.7 w/w%, about 8.8 w/w%, about 8.9 w/w%, about 9.0 w/w%, about 9.1 w/w%, about 9.2 w/w%, about 9.3 w/w%, about 9.4 w/w%, about 9.5 w/w%, about 9.6 w/w%, about 9.7 w/w%, about 9.8 w/w%, about 9.9 w/w%, about 10.0 w/w%, about 10.1 w/w%, about 10.2 w/w%, about 10.3 w/w%, about 10.4 w/w%, about 10.5 w/w%, about 10.6 w/w%, about 10.7 w/w%, about 10.8 w/w%, about 10.9 w/w%, about 11.0 w/w%, about 11.1 w/w%, about 11.2 w/w%, about 11.3 w/w%, about 11.4 w/w%, about 11.5 w/w%, about 11.6 w/w%, about 11.7 w/w%, about 11.8 w/w%, about 11.9 w/w%, about 12.0 w/w%, about 12.1 w/w%, about 12.2 w/w%, about 12.3 w/w%, about 12.4 w/w%, about 12.5 w/w%, about 12.6 w/w%, about 12.7 w/w%, about 12.8 w/w%, about 12.9 w/w%, about 13.0 w/w%, about 13.1 w/w%, about 13.2 w/w%, about 13.3 w/w%, about 13.4 w/w%, about 13.5 w/w%, about 13.6 w/w%, about 13.7 w/w%, about 13.8 w/w%, about 13.9 w/w%, about 14.0 w/w%, about 14.1 w/w%, about 14.2 w/w%, about 14.3 w/w%, about 14.4 w/w%, about 14.5 w/w%, about 14.6 w/w%, about 14.7 w/w%, about 14.8 w/w%, about 14.9 w/w%, about 15.0 w/w%, about 15.1 w/w%, about 15.2 w/w%, about 15.3 w/w%, about 15.4 w/w%, about 15.5 w/w%, about 15.6 w/w%, about 15.7 w/w%, about 15.8 w/w%, about 15.9 w/w%, about 16.0 w/w%, about 16.1 w/w%, about 16.2 w/w%, about 16.3 w/w%, about 16.4 w/w%, about 16.5 w/w%, about 16.6 w/w%, about 16.7 w/w%, about 16.8 w/w%, about 16.9 w/w%, about 17.0 w/w%, about 17.1 w/w%, about 17.2 w/w%, about 17.3 w/w%, about 17.4 w/w%, about 17.5 w/w%, about 17.6 w/w%, about 17.7 w/w%, about 17.8 w/w%, about 17.9 w/w%, about 18.0 w/w%, about 18.1 w/w%, about 18.2 w/w%, about 18.3 w/w%, about 18.4 w/w%, about 18.5 w/w%, about 18.6 w/w%, about 18.7 w/w%, about 18.8 w/w%, about 18.9 w/w%, about 19.0 w/w%, about 19.1 w/w%, about 19.2 w/w%, about 19.3 w/w%, about 19.4 w/w%, about 19.5 w/w%, about 19.6 w/w%, about 19.7 w/w%, about 19.8 w/w%, about 19.9 w/w%, about 20.0 w/w%, about 21.1 w/w%, about 21.2 w/w%, about 21.3 w/w%, about 21.4 w/w%, about 21.5 w/w%, about 21.6 w/w%, about 21.7 w/w%, about 21.8 w/w%, about 21.9 w/w%, about 22.0 w/w%, about 22.1 w/w%, about 22.2 w/w%, about 22.3 w/w%, about 22.4 w/w%, about 22.5 w/w%, about 22.6 w/w%, about 22.7 w/w%, about 22.8 w/w%, about 22.9 w/w%, about 23.0 w/w%, about 23.1 w/w%, about 23.2 w/w%, about 23.3 w/w%, about 23.4 w/w%, about 23.5 w/w%, about 23.6 w/w%, about 23.7 w/w%, about 23.8 w/w%, about 23.9 w/w%, about 24.0 w/w%, about 24.1 w/w%, about 24.2 w/w%, about 24.3 w/w%, about 24.4 w/w%, about 24.5 w/w%, about 24.6 w/w%, about 24.7 w/w%, about 24.8 w/w%, about 24.9 w/w%, about 25.0 w/w%, about 25.1 w/w%, about 25.2 w/w%, about 25.3 w/w%, about 25.4 w/w%, about 25.5 w/w%, about 25.6 w/w%, about 25.7 w/w%, about 25.8 w/w%, about 25.9 w/w%, about 26.0 w/w%, about 26.1 w/w%, about 26.2 w/w%, about 26.3 w/w%, about 26.4 w/w%, about 26.5 w/w%, about 26.6 w/w%, about 26.7 w/w%, about 26.8 w/w%, about 26.9 w/w%, about 27.0 w/w%, about 27.1 w/w%, about 27.2 w/w%, about 27.3 w/w%, about 27.4 w/w%, about 27.5 w/w%, about 27.6 w/w%, about 27.7 w/w%, about 27.8 w/w%, about 27.9 w/w%, about 28.0 w/w%, about 28.1 w/w%, about 28.2 w/w%, about 28.3 w/w%, about 28.4 w/w%, about 28.5 w/w%, about 28.6 w/w%, about 28.7 w/w%, about 28.8 w/w%, about 28.9 w/w%, or about 29.0 w/w%. In some embodiments, the amount of the compound of Formula **(Ia)** in the solution comprising a compound of Formula **(Ia),** PEG 300, sodium hydroxide, poloxamer 188, and water is about 25.2 w/w%. In some embodiments, the amount of the compound of Formula **(Ia)** in the solution comprising a compound of Formula **(Ia),** PEG 300, sodium hydroxide, poloxamer 188, and water is about 25.21 w/w%.

In some embodiments, the amount of sodium hydroxide in the solution comprising the sodium salt of the compound of Formula **(Ia),** PEG 300, sodium hydroxide, poloxamer 188, and water is about 0.01 w/w% to about 3.0 w/w%. In some embodiments, the amount of sodium hydroxide in the solution comprising the sodium salt of the compound of Formula **(Ia),** PEG 300, sodium hydroxide, poloxamer 188, and water is about 0.01 w/w% to about 2.0 w/w%. In some embodiments, the amount of sodium hydroxide in the solution comprising the sodium salt of the compound of Formula **(Ia),** PEG 300, sodium hydroxide, poloxamer 188, and water is about 0.05 w/w% to about 1.5 w/w%. In some embodiments, the amount of sodium hydroxide in the solution comprising the sodium salt of the compound of Formula **(Ia),** PEG 300, sodium hydroxide, poloxamer 188, and water is about 0.05 w/w% to about 1.2 w/w%. In some embodiments, the amount of sodium hydroxide in the solution comprising the sodium salt of the compound of Formula **(Ia),** PEG 300, sodium hydroxide, poloxamer 188, and water is about 0.1 w/w% to about 1.1 w/w%. In some embodiments, the amount of sodium hydroxide in the solution comprising the sodium salt of the compound of Formula **(Ia),** PEG 300, sodium hydroxide, poloxamer 188, and water is about 0.10 w/w% to about 1.08 w/w%. In some embodiments, the amount of sodium hydroxide in the solution comprising the sodium salt of the compound of Formula **(Ia),** PEG 300, sodium hydroxide, poloxamer 188, and water is about 0.01 w/w%, about 0.02 w/w%, about 0.03 w/w%, about 0.04 w/w%, about 0.05 w/w%, about 0.06 w/w%, about 0.07 w/w%, about 0.08 w/w%, about 0.09 w/w%, about 0.1 w/w%, about 0.2 w/w%, about 0.3 w/w%, about 0.4 w/w%, about 0.5 w/w%, about 0.6 w/w%, about 0.7 w/w%, about 0.8 w/w%, about 0.9 w/w%, about 1.0 w/w%, about 1.1 w/w%, about 1.2 w/w%, about 1.3 w/w%, about 1.4 w/w%, about 1.5 w/w%, about 1.6 w/w%, about 1.7 w/w%, about 1.8 w/w%, about 1.9 w/w%, or about 2.0 w/w%. In some embodiments, the amount of sodium hydroxide in the solution comprising the sodium salt of the compound of Formula **(Ia),** PEG 300, sodium hydroxide, poloxamer 188, and water is about 0.1 w/w%. In some embodiments, the amount of sodium hydroxide in the solution comprising the sodium salt of the compound of Formula **(Ia),** PEG 300, sodium hydroxide, poloxamer 188, and water is about 0.10 w/w%. In some embodiments, the amount of sodium hydroxide in the solution comprising the sodium salt of the compound of Formula **(Ia),** PEG 300, sodium hydroxide, poloxamer 188, and water is about 1.08 w/w%. In some embodiments, the amount of sodium hydroxide in the solution comprising the sodium salt of the compound of Formula **(Ia),** PEG 300, sodium hydroxide, poloxamer 188, and water is about 1.1 w/w%.

In some embodiments, the amount of poloxamer 188 in the solution comprising the sodium salt of the compound of Formula **(Ia),** PEG 300, sodium hydroxide, poloxamer 188, and water is about 0.1 w/w% to about 10 w/w%. In some embodiments, the amount of poloxamer 188 in the solution comprising the sodium salt of the compound of Formula **(Ia),** PEG 300, sodium hydroxide, poloxamer 188, and water is about 0.3 w/w% to about 8 w/w%. In some embodiments, the amount of poloxamer 188 in the solution comprising the sodium salt of the compound of Formula **(Ia),** PEG 300, sodium hydroxide, poloxamer 188, and water is about 0.5 w/w% to about 7 w/w%. In some embodiments, the amount of poloxamer 188 in the solution comprising the sodium salt of the compound of Formula **(Ia),** PEG 300, sodium hydroxide, poloxamer 188, and water is about 0.6 w/w% to about 7 w/w%. In some embodiments, the amount of poloxamer 188 in the solution comprising the sodium salt of the compound of Formula **(Ia),** PEG 300, sodium hydroxide, poloxamer 188, and water is about 0.85 w/w% to about 4.69 w/w%. In some embodiments, the amount of poloxamer 188 in the solution comprising the sodium salt of the compound of Formula **(Ia),** PEG 300, sodium hydroxide, poloxamer 188, and water is about 0.9 w/w% to about 4.7 w/w%. In some embodiments, the amount of poloxamer 188 in the solution comprising the sodium salt of the compound of Formula **(Ia),** PEG 300, sodium hydroxide, poloxamer 188, and water is about 0.5 w/w%, about 0.6 w/w%, about 0.7 w/w%, about 0.8 w/w%, about 0.9 w/w%, about 1.0 w/w%, about 1.1 w/w%, about 1.2 w/w%, about 1.3 w/w%, about 1.4 w/w%, about 1.5 w/w%, about 1.6 w/w%, about 1.7 w/w%, about 1.8 w/w%, about 1.9 w/w%, about 2.0 w/w%, about 2.1 w/w%, about 2.2 w/w%, about 2.3 w/w%, about 2.4 w/w%, about 2.5 w/w%, about 2.6 w/w%, about 2.7 w/w%, about 2.8 w/w%, about 2.9 w/w%, about 3.0 w/w%, about 3.1 w/w%, about 3.2 w/w%, about 3.3 w/w%, about 3.4 w/w%, about 3.5 w/w%, about 3.6 w/w%, about 3.7 w/w%, about 3.8 w/w%, about 3.9 w/w%, about 4.0 w/w%, about 4.1 w/w%, about 4.2 w/w%, about 4.3 w/w%, about 4.4 w/w%, about 4.5 w/w%, about 4.6 w/w%, about 4.7 w/w%, about 4.8 w/w%, about 4.9 w/w%, about 5.0 w/w%, about 5.1 w/w%, about 5.2 w/w%, about 5.3 w/w%, about 5.4 w/w%, about 5.5 w/w%, about 5.6 w/w%, about 5.7 w/w%, about 5.8 w/w%, about 5.9 w/w%, about 6.0 w/w%, about 6.1 w/w%, about 6.2 w/w%, about 6.3 w/w%, about 6.4 w/w%, about 6.5 w/w%, about 6.6 w/w%, about 6.7 w/w%, about 6.8 w/w%, about 6.9 w/w%, or about 7.0 w/w%. In some embodiments, the amount of poloxamer 188 in the solution comprising the sodium salt of the compound of Formula **(Ia),** PEG 300, sodium hydroxide, poloxamer 188, and water is about 0.85 w/w%. In some embodiments, the amount of poloxamer 188 in the solution comprising the sodium salt of the compound of Formula **(Ia),** PEG 300, sodium hydroxide, poloxamer 188, and water is about 0.9 w/w%. In some embodiments, the amount of poloxamer 188 in the solution comprising the sodium salt of the compound of Formula **(Ia),** PEG 300, sodium hydroxide, poloxamer 188, and water is about 4.69 w/w%. In some embodiments, the amount of poloxamer 188 in the solution comprising the sodium salt of the compound of Formula **(Ia),** PEG 300, sodium hydroxide, poloxamer 188, and water is about 4.7 w/w%.

In some embodiments, the solution comprises about 22 w/w% water, about 47.1 w/w% PEG 300, about 25.2 w/w% of the sodium salt of the compound of Formula **(Ia),** about 1.1 w/w% of sodium hydroxide, and about 4.7 w/w% of poloxamer 188. In some embodiments, the solution comprises about 21.97 w/w% water, about 47.05 w/w% PEG 300, about 25.21 w/w% of the sodium salt of the compound of Formula **(Ia),** about 1.08 w/w% of sodium hydroxide, and about 4.69 w/w% of poloxamer 188.

In some embodiments, the solutions disclosed herein are administered through subcutaneous injection. In some embodiments, the solutions disclosed herein are administered through intramuscular injection.

### Routes of Administration

The pharmaceutical composition comprising the sodium salt of the compound of the Formula **(Ia)** can be administered parenterally (including subcutaneous, intramuscular, intravenous, intradermal, intrathecal and epidural routes). It will be appreciated that the preferred route can vary with, for example, the condition of the recipient. In certain embodiments, the pharmaceutical compositions disclosed are dosed parenterally. In certain embodiments, the pharmaceutical compositions disclosed are dosed intravenously, subcutaneously, or intramuscularly. In some embodiments, the pharmaceutical composition comprising the sodium salt of the compound of Formula **(Ia)** is administered subcutaneously. In some embodiments, the pharmaceutical composition comprising the sodium salt of the compound of Formula **(Ia)** is administered intravenously. In some embodiments, the pharmaceutical composition comprising the sodium salt of the compound of Formula **(Ia)** is administered intramuscularly.

In some embodiments, the pharmaceutical composition comprising the sodium salt of the compound of the Formula **(Ia)** is administered via injection, using an injection device. In some embodiments, the injection device is or includes a syringe, which can be employed manually, or as part of a syringe-containing injection device. A wide variety of injection devices can be used, including, but not limited to, a handheld or wearable autoinjector, a handheld or wearable manual injector, an on-body injector, a syrette, a jet injector, or a pen injector, each of which can be reusable or disposable.

In some embodiments, the pharmaceutical composition comprising the sodium salt of the compound of Formula **(Ia)** can be administered with a syringe suitable for administration of the compound. In some embodiments, the syringe is disposable. In some embodiments, the syringe is reusable. In some embodiments, the syringe is pre-filled with the pharmaceutical composition comprising the sodium salt of the compound of Formula **(Ia).**

In some embodiments, the pharmaceutical composition comprising the sodium salt of the compound of Formula **(Ia)** can be administered with an auto-injector comprising a syringe. In some embodiments, the syringe is disposable. In some embodiments, the syringe is reusable. In some embodiments, the syringe is pre-filled with the compound of Formula **(Ia).**

In some embodiments, the pharmaceutical composition comprising the sodium salt of the compound of Formula **(Ia)** is formulated for subcutaneous administration. In some embodiments, the pharmaceutical composition comprising the sodium salt of the compound of Formula **(Ia)** is formulated as a solution for subcutaneous administration. In some embodiments, the compound of Formula **(Ia)** is formulated at a concentration of about 50 mg/mL to about 300 mg/mL, about 50 mg/mL to about 200 mg/mL, about 50 mg/mL to about 150 mg/mL, about 50 mg/mL to about 100 mg/mL, about 100 mg/mL to about 300 mg/mL, about 100 mg/mL to about 200 mg/mL, about 200 mg/mL to about 300 mg/mL, or about 50 mg/mL, about 100 mg/mL, about 150 mg/mL, about 200 mg/mL, or about 300 mg/mL. In some embodiments, the compound of Formula **(Ia)** is formulated at a concentration of about 100 mg/mL. In some embodiments, the compound of Formula **(Ia)** is formulated at a concentration of about 150 mg/mL. In some embodiments, the compound of Formula **(Ia)** is formulated at a concentration of about 300 mg/mL.

In some embodiments, the compound of Formula **(Ia)** is formulated at a concentration of about 50 mg/mL. In some embodiments, the compound of Formula **(Ia)** is formulated at a concentration of about 75 mg/mL. In some embodiments, the compound of Formula **(Ia)** is formulated at a concentration of about 125 mg/mL. In some embodiments, the compound of Formula **(Ia)** is formulated at a concentration of about 175 mg/mL. In some embodiments, the compound of Formula **(Ia)** is formulated at a concentration of about 200 mg/mL. In some embodiments, the compound of Formula **(Ia)** is formulated at a concentration of about 225 mg/mL. In some embodiments, the compound of Formula **(Ia)** is formulated at a concentration of about 250 mg/mL. In some embodiments, the compound of Formula **(Ia)** is formulated at a concentration of about 275 mg/mL. In some embodiments, the compound of Formula **(Ia)** is formulated at a concentration of about 325 mg/mL. In some embodiments, the compound of Formula **(Ia)** is formulated at a concentration of about 350 mg/mL.

In some embodiments, the compound of Formula **(Ia)** is formulated at a concentration of about 50 mg/mL to about 100 mg/mL, about 50 mg/mL to about 75 mg/mL, or about 75 mg/mL to about 100 mg/mL, or 50 mg/mL, about 60 mg/mL, about 70 mg/mL, about 80 mg/mL, about 90 mg/mL, or about 100 mg/mL.

### Dosing Regimen

In some embodiments, the pharmaceutical composition comprising the sodium salt of the compound of Formula **(Ia)** is administered to a patient in accordance with an effective dosing regimen for a desired period of time or duration. In some embodiments, the dosing regimen includes administration of the compound of Formula **(Ia)** for at least about 1 week, about 2 weeks, about 4 weeks, about 8 weeks, about 12 weeks, about 16 weeks, about 20 weeks, about 24 weeks, or about 48 weeks, or longer.

In some embodiments, the dosing regimen includes administration of the pharmaceutical composition comprising the sodium salt of the compound of Formula **(Ia)** for at least about 1 year, 2 years, 3 years, 4 years, 5 years, 10 years, or longer. In some embodiments, the dosing regimen includes a permanent administration of the pharmaceutical composition comprising the sodium salt of the compound of Formula **(Ia).**

In some embodiments, the pharmaceutical composition comprising the sodium salt of the compound of Formula **(Ia)** is administered on a daily or intermittent schedule. In some embodiments, the pharmaceutical composition comprising the sodium salt of the compound of Formula **(Ia)** is administered once daily. In some embodiments, the pharmaceutical composition comprising the sodium salt of the compound of Formula **(Ia)** is administered on a monthly schedule. In some embodiments, the pharmaceutical composition comprising the sodium salt of the compound of Formula **(Ia)** is administered once about every 1 week, about every 2 weeks, about every 4 weeks, about every 8 weeks, about every 12 weeks, about every 16 weeks, about every 20 weeks, about every 24 weeks, or once about every 48 weeks. In some embodiments, the pharmaceutical composition comprising the sodium salt of the compound of Formula **(Ia)** is administered once every 4 weeks (or monthly). In some embodiments, the pharmaceutical composition comprising the sodium salt of the compound of Formula **(Ia)** is administered once every 8 weeks (or 2 months). In some embodiments, the pharmaceutical composition comprising the sodium salt of the compound of Formula **(Ia)** is administered once every 12 weeks (or three months). In some embodiments, the pharmaceutical composition comprising the sodium salt of the compound of Formula **(Ia)** is administered once every 16 weeks (or four months). In some embodiments, the pharmaceutical composition comprising the sodium salt of the compound of Formula **(Ia)** is administered once every 20 weeks (or five months). In some embodiments, the pharmaceutical composition comprising the sodium salt of the compound of Formula **(Ia)** is administered every 24 weeks (or 6 months). In some embodiments, the pharmaceutical composition comprising the sodium salt of the compound of Formula **(Ia)** is administered every 52 weeks (or yearly).

In some embodiments, the pharmaceutical composition comprising the sodium salt of the compound of Formula **(Ia)** is subcutaneously administered to a patient for at least about one month. In some embodiments, the pharmaceutical composition comprising the sodium salt of the compound of Formula **(Ia)** is subcutaneously or intramuscularly administered to a patient for at least about 2 months, at least about 3 months, at least about 4 months, or at least about 6 months. In some embodiments, the pharmaceutical composition comprising the sodium salt of the compound of Formula **(Ia)** is subcutaneously administered to a patient once about every month. In some embodiments, the pharmaceutical composition comprising the sodium salt of the compound of Formula **(Ia)** is subcutaneously or intramuscularly administered to a patient once about every 3 months. In some embodiments, the pharmaceutical composition comprising the sodium salt of the compound of Formula **(Ia)** is subcutaneously or intramuscularly administered to a patient once about every 6 months.

In some embodiments, the pharmaceutical composition comprising the sodium salt of the compound of Formula **(Ia)** is intramuscularly administered to a patient once about every month. In some embodiments, the pharmaceutical composition comprising the sodium salt of the compound of Formula **(Ia)** is subcutaneously administered to a patient once about every 3 months. In some embodiments, the pharmaceutical composition comprising the sodium salt of the compound of Formula **(Ia)** is intramuscularly administered to a patient once about every 3 months. In some embodiments, the pharmaceutical composition comprising the sodium salt of the compound of Formula **(Ia)** is subcutaneously administered to a patient once about every 6 months. In some embodiments, the pharmaceutical composition comprising the sodium salt of the compound of Formula **(Ia)** is intramuscularly administered to a patient once about every 6 months. In some embodiments, the pharmaceutical composition comprising the sodium salt of the compound of Formula **(Ia)** is subcutaneously administered to a patient once about every year. In some embodiments, the pharmaceutical composition comprising the sodium salt of the compound of Formula **(Ia)** is intramuscularly administered to a patient once about every year.

The dosage or dosing frequency of the pharmaceutical composition comprising the sodium salt of the compound of Formula **(Ia)** can be adjusted over the course of the treatment, based on the judgment of the administering physician.

In some embodiments, the pharmaceutical composition comprising the sodium salt of the compound of Formula **(Ia)** is administered to a patient (for example, a human) in a therapeutically effective amount. In some embodiments, the pharmaceutical composition comprising the sodium salt of the compound of Formula **(Ia)** is administered once daily. In some embodiments, the pharmaceutical composition comprising the sodium salt of the compound of Formula **(Ia)** is administered monthly. In some embodiments, the pharmaceutical composition comprising the sodium salt of the compound of Formula **(Ia)** is administered every two months. In some embodiments, the pharmaceutical composition comprising the sodium salt of the compound of Formula **(Ia)** is administered every three months. In some embodiments, the pharmaceutical composition comprising the sodium salt of the compound of Formula **(Ia)** is administered every four months. In some embodiments, the pharmaceutical composition comprising the sodium salt of the compound of Formula **(Ia)** is administered every six months. In some embodiments, the pharmaceutical composition comprising the sodium salt of the compound of Formula **(Ia)** is administered yearly.

In some embodiments, the pharmaceutical composition comprising the sodium salt of the compound of Formula **(Ia)** is administered in a dosage amount that is effective. In some embodiments, the dosage is from about 1 mg to about 1000 mg of the compound of Formula **(Ia).** In certain embodiments, the dosage amount is about 1 mg, about 10 mg, about 20 mg, about 30 mg, about 40 mg, about 50 mg, about 60 mg, about 70 mg, about 80 mg, about 90 mg, about 95 mg, about 100 mg, about 105 mg, about 110 mg, about 120 mg, about 130 mg, about 140 mg, or about 150 mg of the compound of Formula **(Ia).** In certain embodiments, the dosage amount is about 100 mg, about 150 mg, about 200 mg, about 250 mg, about 300 mg, about 350 mg, about 400 mg, about 450 mg, about 500 mg, about 550 mg, about 600 mg, about 650 mg, about 700 mg, about 750 mg, about 800 mg, about 850 mg, about 900 mg, about 950 mg, or about 1000 mg of the compound of Formula **(Ia).**

In certain embodiments, the dosage amount is about 1 mg, about 5 mg, about 10 mg, about 20 mg, about 30 mg, about 40 mg, about 50 mg, about 60 mg, about 70 mg, about 80 mg, about 90 mg, about 100 mg, about 110 mg, about 120 mg, about 130 mg, about 140 mg, about 150 mg, about 200 mg, about 250 mg, about 300 mg, about 350 mg, about 400 mg, about 450 mg, about 500 mg, about 550 mg, about 600 mg, about 650 mg, about 700 mg, about 750 mg, about 800 mg, about 850 mg, about 900 mg, about 950 mg, about 1000 mg, about 1050 mg, about 1100 mg, about 1150 mg, about 1200 mg, about 1250 mg, about 1300 mg, about 1350 mg, about 1400 mg, about 1450 mg, about 1500 mg, about 1550 mg, about 2000 mg, about 2050 mg, or about 3000 mg of the compound of Formula **(Ia).**

In some embodiments, the dosage amount of the compound of Formula **(Ia)** is about 1 mg to about 2500 mg. In some embodiments, the dosage amount of the compound of Formula **(Ia)** is about 5 mg to about 2400 mg. In some embodiments, the dosage amount of the compound of Formula **(Ia)** is about 5 mg to about 2000 mg. In some embodiments, the dosage amount of the compound of Formula **(Ia)** is about 5 mg to about 1500 mg. In some embodiments, the dosage amount of the compound of Formula **(Ia)** is about 5 mg to about 1200 mg. In some embodiments, the dosage amount of the compound of Formula **(Ia)** is about 5 mg to about 1000 mg. In some embodiments, the dosage amount of the compound of Formula **(Ia)** is about 5 mg to about 500 mg. In some embodiments, the dosage amount of the compound of Formula **(Ia)** is about 5 mg to about 300 mg. In some embodiments, the dosage amount of the compound of Formula **(Ia)** is about 5 mg to about 200 mg. In some embodiments, the dosage amount of the compound of Formula **(Ia)** is about 5 mg to about 100 mg. In some embodiments, the dosage amount of the compound of Formula **(Ia)** is about 5 mg to about 50 mg.

In some embodiments, the compound of Formula **(Ia)** is administered in a once daily dose of about 1 mg to about 1500 mg. In some embodiments, the compound of Formula **(Ia)** is administered in a once daily dose of about 5 mg to about 1200 mg. In some embodiments, the compound of Formula **(Ia)** is administered in a once daily dose of about 100 mg to about 1200 mg. In some embodiments, the compound of Formula **(Ia)** is administered in a once daily dose of about 200 mg to about 1200 mg. In some embodiments, the compound of Formula **(Ia)** is administered in a once daily dose of about 300 mg to about 1200 mg. In some embodiments, the compound of Formula **(Ia)** is administered in a once daily dose of about 500 mg to about 1200 mg.

In some embodiments, the compound of Formula **(Ia)** is administered in a once daily dose of about 1 mg to about 200 mg. In some embodiments, the compound of Formula **(Ia)** is administered in a once daily dose of about 5 mg to about 200 mg. In some embodiments, the compound of Formula **(Ia)** is administered in a once daily dose of about 10 mg to about 200 mg. In some embodiments, the compound of Formula **(Ia)** is administered in a once daily dose of about 50 mg to about 200 mg. In some embodiments, the compound of Formula **(Ia)** is administered in a once daily dose of about 100 mg to about 200 mg.

In some embodiments, the compound of Formula **(Ia)** is administered in a once daily dose of about 1 mg, about 5 mg, about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, about 100 mg, about 105 mg, about 110 mg, about 115 mg, about 120 mg, about 125 mg, about 130 mg, about 135 mg, about 140 mg, about 145 mg, about 150 mg, about 155 mg, about 160 mg, about 165 mg, about 170 mg, about 175 mg, about 180 mg, about 185 mg, about 190 mg, about 195 mg, about 200 mg, about 250 mg, about 300 mg, about 350 mg, about 400 mg, about 450 mg, about 500 mg, about 550 mg, about 600 mg, about 650 mg, about 700 mg, about 750 mg, about 800 mg, about 850 mg, about 900 mg, about 950 mg, about 1000 mg, about 1050 mg, about 1100 mg, about 1150 mg, about 1200 mg, about 1250 mg, about 1300 mg, about 1350 mg, about 1400 mg, about 1450 mg, or about 1500 mg.

In some embodiments, the compound of Formula **(Ia)** is administered in a once daily dose of about 1 mg.

In some embodiments, the compound of Formula **(Ia)** is administered in a once daily dose of about 5 mg. In some embodiments, the compound of Formula **(Ia)** is administered in a once daily dose of about 10 mg. In some embodiments, the compound of Formula **(Ia)** is administered in a once daily dose of about 15 mg. In some embodiments, the compound of Formula **(Ia)** is administered in a once daily dose of about 20 mg. In some embodiments, the compound of Formula **(Ia)** is administered in a once daily dose of about 25 mg. In some embodiments, the compound of Formula **(Ia)** is administered in a once daily dose of about 50 mg. In some embodiments, the compound of Formula **(Ia)** is administered in a once daily dose of about 75 mg. In some embodiments, the compound of Formula **(Ia)** is administered in a once daily dose of about 100 mg. In some embodiments, the compound of Formula **(Ia)** is administered in a once daily dose of about 125 mg. In some embodiments, the compound of Formula **(Ia)** is administered in a once daily dose of about 150 mg. In some embodiments, the compound of Formula **(Ia)** is administered in a once daily dose of about 175 mg. In some embodiments, the compound of Formula **(Ia)** is administered in a once daily dose of about 200 mg. In some embodiments, the compound of Formula **(Ia)** is administered in a once daily dose of about 300 mg. In some embodiments, the compound of Formula **(Ia)** is administered in a once daily dose of about 400 mg. In some embodiments, the compound of Formula **(Ia)** is administered in a once daily dose of about 500 mg. In some embodiments, the compound of Formula **(Ia)** is administered in a once daily dose of about 600 mg. In some embodiments, the compound of Formula **(Ia)** is administered in a once daily dose of about 700 mg. In some embodiments, the compound of Formula **(Ia)** is administered in a once daily dose of about 800 mg. In some embodiments, the compound of Formula **(Ia)** is administered in a once daily dose of about 900 mg. In some embodiments, the compound of Formula **(Ia)** is administered in a once daily dose of about 1000 mg. In some embodiments, the compound of Formula **(Ia)** is administered in a once daily dose of about 1100 mg. In some embodiments, the compound of Formula **(Ia)** is administered in a once daily dose of about 1200 mg. In some embodiments, the compound of Formula **(Ia)** is administered in a once daily dose of about 1300 mg. In some embodiments, the compound of Formula **(Ia)** is administered in a once daily dose of about 1400 mg. In some embodiments, the compound of Formula **(Ia)** is administered in a once daily dose of about 1500 mg.

In some embodiments, the compound of Formula **(Ia)** is administered weekly at a dose of about 100 mg to about 2500 mg. In some embodiments, the compound of Formula **(Ia)** is administered weekly at a dose of about 100 mg to about 2400 mg. In some embodiments, the compound of Formula **(Ia)** is administered weekly at a dose of about 100 mg to about 2000 mg. In some embodiments, the compound of Formula **(Ia)** is administered weekly at a dose of about 300 mg to about 2500 mg. In some embodiments, the compound of Formula **(Ia)** is administered weekly at a dose of about 500 mg to about 2500 mg. In some embodiments, the compound of Formula **(Ia)** is administered weekly at a dose of about 800 mg to about 2500 mg. In some embodiments, the compound of Formula **(Ia)** is administered weekly at a dose of about 1000 mg to about 2500 mg. In some embodiments, the compound of Formula **(Ia)** is administered weekly at a dose of about 100 mg to about 1200 mg. In some embodiments, the compound of Formula **(Ia)** is administered weekly at a dose of about 150 mg to about 1200 mg. In some embodiments, the compound of Formula **(Ia)** is administered weekly at a dose of about 200 mg to about 1200 mg. In some embodiments, the compound of Formula **(Ia)** is administered weekly at a dose of about 300 mg to about 1200 mg. In some embodiments, the compound of Formula **(Ia)** is administered weekly at a dose of about 500 mg to about 1200 mg. In some embodiments, the compound of Formula **(Ia)** is administered weekly at a dose of about 600 mg to about 1200 mg. In some embodiments, the compound of Formula **(Ia)** is administered weekly at a dose of about 800 mg to about 1200 mg. In some embodiments, the compound of Formula **(Ia)** is administered weekly at a dose of about 1000 mg to about 1200 mg. In some embodiments, the compound of Formula **(Ia)** is administered weekly at a dose of about 100 mg, about 125 mg, about 150 mg, about 175 mg, about 200 mg, about 225 mg, about 250 mg, about 275 mg, about 300 mg, about 325 mg, about 350 mg, about 375 mg, about 400 mg, about 425 mg, about 450 mg, about 475 mg, about 500 mg, about 525 mg, about 550 mg, about 575 mg, about 600 mg, about 625 mg, about 650 mg, about 675 mg, about 700 mg, about 725 mg, about 750 mg, about 775 mg, about 800 mg, about 825 mg, about 850 mg, about 875 mg, about 900 mg, about 925 mg, about 950 mg, about 975 mg, about 1000 mg, about 1025 mg, about 1050 mg, about 1075 mg, about 1100 mg, about 1125 mg, about 1150 mg, about 1175 mg, about 1200 mg, about 1250 mg, about 1300 mg, about 1350 mg, about 1400 mg, about 1450 mg, about 1500 mg, about 1550 mg, about 1600 mg, about 1650 mg, about 1700 mg, about 1750 mg, about 1800 mg, about 1850 mg, about 1900 mg, about 1950 mg, about 2000 mg, about 2050 mg, about 2100 mg, about 2150 mg, about 2200 mg, about 2250 mg, about 2300 mg, about 2350 mg, about 2400 mg, about 2450 mg, or about 2500 mg.

In some embodiments, the compound of Formula **(Ia)** is administered weekly at a dose of about 100 mg. In some embodiments, the compound of Formula **(Ia)** is administered weekly at a dose of about 150 mg. In some embodiments, the compound of Formula **(Ia)** is administered weekly at a dose of about 175 mg. In some embodiments, the compound of Formula **(Ia)** is administered weekly at a dose of about 200 mg. In some embodiments, the compound of Formula **(Ia)** is administered weekly at a dose of about 300 mg.

In some embodiments, the compound of Formula **(Ia)** is administered weekly at a dose of about 400 mg. In some embodiments, the compound of Formula **(Ia)** is administered weekly at a dose of about 500 mg.

In some embodiments, the compound of Formula **(Ia)** is administered weekly at a dose of about 600 mg.

In some embodiments, the compound of Formula **(Ia)** is administered weekly at a dose of about 700 mg.

In some embodiments, the compound of Formula **(Ia)** is administered weekly at a dose of about 800 mg.

In some embodiments, the compound of Formula **(Ia)** is administered weekly at a dose of about 900 mg.

In some embodiments, the compound of Formula **(Ia)** is administered weekly at a dose of about 1000 mg. In some embodiments, the compound of Formula **(Ia)** is administered weekly at a dose of about 1100 mg. In some embodiments, the compound of Formula **(Ia)** is administered weekly at a dose of about 1200 mg. In some embodiments, the compound of Formula **(Ia)** is administered weekly at a dose of about 1300 mg. In some embodiments, the compound of Formula **(Ia)** is administered weekly at a dose of about 1400 mg. In some embodiments, the pharmaceutical composition comprising the sodium salt of the compound of Formula **(Ia)** is administered weekly at a dose of about 1500 mg.

In some embodiments, the pharmaceutical composition comprising the sodium salt of the compound of Formula **(Ia)** is administered weekly at a dose of about 1600 mg. In some embodiments, the compound of Formula **(Ia)** is administered weekly at a dose of about 1700 mg. In some embodiments, the compound of Formula **(Ia)** is administered weekly at a dose of about 1800 mg. In some embodiments, the compound of Formula **(Ia)** is administered weekly at a dose of about 1900 mg. In some embodiments, the compound of Formula **(Ia)** is administered weekly at a dose of about 2000 mg. In some embodiments, the compound of Formula **(Ia)** is administered weekly at a dose of about 2100 mg.

In some embodiments, the compound of Formula **(Ia)** is administered weekly at a dose of about 2200 mg. In some embodiments, the compound of Formula **(Ia)** is administered weekly at a dose of about 2300 mg. In some embodiments, the compound of Formula **(Ia)** is administered weekly at a dose of about 2400 mg. In some embodiments, the compound of Formula **(Ia)** is administered weekly at a dose of about 2500 mg.

In some embodiments, the compound of Formula **(Ia)** is administered monthly at a dose of about 100 mg to about 3000 mg. In some embodiments, the compound of Formula **(Ia)** is administered monthly at a dose of about 100 mg to about 2500 mg. In some embodiments, the compound of Formula **(Ia)** is administered monthly at a dose of about 100 mg to about 2400 mg. In some embodiments, the compound of Formula **(Ia)** is administered monthly at a dose of about 100 mg to about 2000 mg. In some embodiments, the compound of Formula **(Ia)** is administered monthly at a dose of about 300 mg to about 2500 mg. In some embodiments, the compound of Formula **(Ia)** is administered monthly at a dose of about 500 mg to about 2500 mg. In some embodiments, the compound of Formula **(Ia)** is administered monthly at a dose of about 800 mg to about 2500 mg. In some embodiments, the compound of Formula **(Ia)** is administered monthly at a dose of about 1000 mg to about 2500 mg.

In some embodiments, the compound of Formula **(Ia)** is administered monthly at a dose of about 200 mg to about 2000 mg. In some embodiments, the compound of Formula **(Ia)** is administered monthly at a dose of about 300 mg to about 2000 mg. In some embodiments, the compound of Formula **(Ia)** is administered monthly at a dose of about 400 mg to about 2000 mg. In some embodiments, the compound of Formula **(Ia)** is administered monthly at a dose of about 500 mg to about 2000 mg. In some embodiments, the compound of Formula **(Ia)** is administered monthly at a dose of about 800 mg to about 2000 mg. In some embodiments, the compound of Formula **(Ia)** is administered monthly at a dose of about 1000 mg to about 2000 mg. In some embodiments, the compound of Formula **(Ia)** is administered monthly at a dose of about 100 mg, about 125 mg, about 150 mg, about 175 mg, about 200 mg, about 225 mg, about 250 mg, about 275 mg, about 300 mg, about 325 mg, about 350 mg, about 375 mg, about 400 mg, about 425 mg, about 450 mg, about 475 mg, about 500 mg, about 525 mg, about 550 mg, about 575 mg, about 600 mg, about 625 mg, about 650 mg, about 675 mg, about 700 mg, about 725 mg, about 750 mg, about 775 mg, about 800 mg, about 825 mg, about 850 mg, about 875 mg, about 900 mg, about 925 mg, about 950 mg, about 975 mg, about 1000 mg, about 1025 mg, about 1050 mg, about 1075 mg, about 1100 mg, about 1125 mg, about 1150 mg, about 1175 mg, about 1200 mg, about 1250 mg, about 1300 mg, about 1350 mg, about 1400 mg, about 1450 mg, about 1500 mg, about 1550 mg, about 1600 mg, about 1650 mg, about 1700 mg, about 1750 mg, about 1800 mg, about 1850 mg, about 1900 mg, about 1950 mg, about 2000 mg, about 2050 mg, about 2100 mg, about 2150 mg, about 2200 mg, about 2250 mg, about 2300 mg, about 2350 mg, about 2400 mg, about 2450 mg, about 2500 mg, about 2550 mg, about 2600 mg, about 2650 mg, about 2700 mg, about 2750 mg, about 2800 mg, about 2850 mg, about 2900 mg, about 2950 mg, or about 3000 mg.

In some embodiments, the compound of Formula **(Ia)** is administered monthly at a dose of about 100 mg.

In some embodiments, the compound of Formula **(Ia)** is administered monthly at a dose of about 150 mg.

In some embodiments, the compound of Formula **(Ia)** is administered monthly at a dose of about 200 mg.

In some embodiments, the compound of Formula **(Ia)** is administered monthly at a dose of about 300 mg.

In some embodiments, the compound of Formula **(Ia)** is administered monthly at a dose of about 400 mg. In some embodiments, the compound of Formula **(Ia)** is administered monthly at a dose of about 500 mg. In some embodiments, the compound of Formula **(Ia)** is administered monthly at a dose of about 800 mg.

In some embodiments, the compound of Formula **(Ia)** is administered monthly at a dose of about 1000 mg. In some embodiments, the compound of Formula **(Ia)** is administered monthly at a dose of about 1100 mg. In some embodiments, the compound of Formula **(Ia)** is administered monthly at a dose of about 1200 mg. In some embodiments, the compound of Formula **(Ia)** is administered monthly at a dose of about 1500 mg. In some embodiments, the compound of Formula **(Ia)** is administered monthly at a dose of about 1800 mg. In some embodiments, the compound of Formula **(Ia)** is administered monthly at a dose of about 2000 mg. In some embodiments, the compound of Formula **(Ia)** is administered monthly at a dose of about 2100 mg. In some embodiments, the compound of Formula **(Ia)** is administered monthly at a dose of about 2200 mg. In some embodiments, the compound of Formula **(Ia)** is administered monthly at a dose of about 2300 mg. In some embodiments, the compound of Formula **(Ia)** is administered monthly at a dose of about 2400 mg. In some embodiments, the compound of Formula **(Ia)** is administered monthly at a dose of about 2500 mg. In some embodiments, the compound of Formula **(Ia)** is administered monthly at a dose of about 2600 mg. In some embodiments, the compound of Formula **(Ia)** is administered monthly at a dose of about 2700 mg. In some embodiments, the compound of Formula **(Ia)** is administered monthly at a dose of about 2800 mg. In some embodiments, the compound of Formula **(Ia)** is administered monthly at a dose of about 2900 mg. In some embodiments, the compound of Formula **(Ia)** is administered monthly at a dose of about 3000 mg.

In some embodiments, the compound of Formula **(Ia)** is administered every 6 months at a dose of about 600 mg.

In some embodiments, the dosage and/or dose amounts disclosed herein of the compound of Formula **(Ia)** is administered parenterally.

In some embodiments, the dosage and/or dose amounts disclosed herein of the compound of Formula **(Ia)** is administered parenterally. In some embodiments, the dosage and/or dose amounts disclosed herein of the compound of Formula **(Ia)** is administered through an injection. In some embodiments, the dosage and/or dose amounts disclosed herein of the compound of Formula **(Ia)** is administered through a subcutaneous injection.

### Combination Therapy

In certain embodiments, a pharmaceutical composition according to the claims for use in a method for treating or preventing an HIV infection in a human having or at risk of having the infection is provided, comprising administering to the human a therapeutically effective amount of the pharmaceutical composition comprising the sodium salt of the compound of Formula **(Ia)** in combination with a therapeutically effective amount of one or more (for example, one, two, three, or four; or one or two; or one to three; or one to four) additional therapeutic agents. In one embodiment, the pharmaceutical composition comprising the sodium salt of the compound of Formula **(Ia)** for use in a method for treating an HIV infection in a human having or at risk of having the infection is provided, comprising administering to the human a therapeutically effective amount of the pharmaceutical composition comprising the sodium salt of the compound of Formula **(Ia)** in combination with a therapeutically effective amount of one or more (for example, one, two, three, or four; or one or two; or one to three; or one to four) additional therapeutic agents.

In some embodiments, a pharmaceutical composition according to the claims for use in a method for treating or preventing an HIV infection in a human having or at risk of having the infection is provided, comprising administering to the human a therapeutically effective amount of the pharmaceutical composition comprising the sodium salt of the compound of Formula **(Ia)** in combination with a therapeutically effective amount of one or more (for example, one, two, three, or four; or one or two; or one to three; or one to four) additional therapeutic agents. In one embodiment, a pharmaceutical composition comprising the sodium salt of the compound of Formula **(Ia)** for use in method for treating an HIV infection in a human having or at risk of having the infection is provided, comprising administering to the human a therapeutically effective amount of the pharmaceutical composition comprising the sodium salt of the compound of Formula **(Ia)** in combination with a therapeutically effective amount of one or more (for example, one, two, three, or four; or one or two; or one to three; or one to four) additional therapeutic agents.

In one embodiment, the pharmaceutical composition comprising the sodium salt of the compound of Formula **(Ia)** in combination with one or more (for example, one, two, three, or four; or one or two; or one to three; or one to four) additional therapeutic agents, and a pharmaceutically acceptable excipient is provided.

In some embodiments, the pharmaceutical composition comprising the sodium salt of a compound of Formula **(Ia)** in combination with one or more (for example, one, two, three, or four; or one or two; or one to three; or one to four) additional therapeutic agents, and a pharmaceutically acceptable excipient is provided.

In certain embodiments, the present disclosure provides a pharmaceutical composition comprising the sodium salt of a compound of Formula **(Ia)** for use in a method for treating an HIV infection, comprising administering to a patient in need thereof a therapeutically effective amount of the pharmaceutical composition comprising the sodium salt of the compound of Formula **(Ia)** in combination with a therapeutically effective amount of one or more additional therapeutic agents which are suitable for treating an HIV infection.

In certain embodiments, the present disclosure provides a pharmaceutical composition comprising the sodium salt of a compound of Formula **(Ia)** for use in a method for treating an HIV infection, comprising administering to a heavily treatment-experienced patient in need thereof a therapeutically effective amount of the pharmaceutical composition comprising the sodium salt of the compound of Formula **(Ia)** in combination with a therapeutically effective amount of one or more additional therapeutic agents which are suitable for treating an HIV infection in a heavily treatment-experienced patient. In some embodiments, the heavily treatment-experienced patients are patients with multidrug resistant HIV infection.

In certain embodiments, the present disclosure provides a pharmaceutical composition comprising the sodium salt of a compound of Formula **(Ia)** for use in a method for treating an HIV infection in a heavily treatment-experienced patient, comprising administering to a patient in need thereof a therapeutically effective amount of the pharmaceutical composition comprising the sodium salt of the compound of Formula **(Ia).**

In certain embodiments, the pharmaceutical composition comprising the sodium salt of a compound of Formula **(Ia)** is combined with one, two, three, four, or more additional therapeutic agents. In certain embodiments, the pharmaceutical composition comprising the sodium salt of a compound of Formula **(Ia)** is combined with one additional therapeutic agent. In certain embodiments, the pharmaceutical composition comprising the sodium salt of a compound of Formula **(Ia)** is combined with two additional therapeutic agents. In other embodiments, the pharmaceutical composition comprising the sodium salt of a compound of Formula **(Ia)** is combined with three additional therapeutic agents. In further embodiments, the pharmaceutical composition comprising the sodium salt of a compound of Formula **(Ia)** is combined with four additional therapeutic agents. The one, two, three, four, or more additional therapeutic agents can be different therapeutic agents selected from the same class of therapeutic agents, and/or they can be selected from different classes of therapeutic agents.

In some embodiments, the pharmaceutical composition comprising the sodium salt of a compound of Formula **(Ia)** is combined with one, two, three, four, or more additional therapeutic agents. In certain embodiments, the pharmaceutical composition comprising the sodium salt of a compound of Formula **(Ia)** is combined with one additional therapeutic agent. In certain embodiments, the pharmaceutical composition comprising the sodium salt of a compound of Formula **(Ia)** is combined with two additional therapeutic agents. In other embodiments, the pharmaceutical composition comprising the sodium salt of a compound of Formula **(Ia)** is combined with three additional therapeutic agents. In further embodiments, the pharmaceutical composition comprising the sodium salt of a compound of Formula **(Ia)** is combined with four additional therapeutic agents. The one, two, three, four, or more additional therapeutic agents can be different therapeutic agents selected from the same class of therapeutic agents, and/or they can be selected from different classes of therapeutic agents.

### Administration of HIV Combination Therapy

In certain embodiments, the pharmaceutical composition comprising the sodium salt of Formula **(Ia),** is administered with one or more additional therapeutic agents. Co-administration of the pharmaceutical composition comprising the sodium salt of Formula **(Ia),** with one or more additional therapeutic agents generally refers to simultaneous or sequential administration of the pharmaceutical composition comprising the sodium salt of Formula **(Ia)** and one or more additional therapeutic agents, such that therapeutically effective amounts of the pharmaceutical composition comprising the sodium salt of Formula **(Ia),** and the one or more additional therapeutic agents are both present in the body of the patient. When administered sequentially, the combination may be administered in two or more administrations.

In some embodiments, the pharmaceutical composition comprising the sodium salt of Formula **(Ia)** is administered with one or more additional therapeutic agents. Co-administration of the pharmaceutical composition comprising the sodium salt of Formula **(Ia),** with one or more additional therapeutic agents generally refers to simultaneous or sequential administration of the pharmaceutical composition comprising the sodium salt of Formula **(Ia)** and one or more additional therapeutic agents, such that therapeutically effective amounts of the pharmaceutical composition comprising the sodium salt of Formula **(Ia),** and the one or more additional therapeutic agents are both present in the body of the patient. When administered sequentially, the combination may be administered in two or more administrations.

Co-administration includes administration of unit dosages of the pharmaceutical composition comprising the sodium salt of the compound of Formula **(Ia)** before or after administration of unit dosages of one or more additional therapeutic agents. For example, the pharmaceutical composition comprising the sodium salt of Formula **(Ia)** may be administered within seconds, minutes, or hours of the administration of the one or more additional therapeutic agents. In some embodiments, a unit dose of the pharmaceutical composition comprising the sodium salt of Formula **(Ia)** is administered first, followed within seconds or minutes by administration of a unit dose of one or more additional therapeutic agents. Alternatively, a unit dose of one or more additional therapeutic agents is administered first, followed by administration of a unit dose of the pharmaceutical composition comprising the sodium salt of Formula **(Ia)** within seconds or minutes. In other embodiments, a unit dose of the pharmaceutical composition comprising the sodium salt of Formula **(Ia)** is administered first, followed, after a period of hours (for example, 1-12 hours), by administration of a unit dose of one or more additional therapeutic agents. In yet other embodiments, a unit dose of one or more additional therapeutic agents is administered first, followed, after a period of hours (for example, 1-12 hours), by administration of a unit dose of the pharmaceutical composition comprising the sodium salt of Formula **(Ia).**

In some embodiments, co-administration includes administration of unit dosages of the pharmaceutical composition comprising the sodium salt of Formula **(Ia)** before or after administration of unit dosages of one or more additional therapeutic agents. For example, the pharmaceutical composition comprising the sodium salt of Formula **(Ia)** may be administered within seconds, minutes, or hours of the administration of the one or more additional therapeutic agents. In some embodiments, a unit dose of the pharmaceutical composition comprising the sodium salt of Formula **(Ia)** is administered first, followed within seconds or minutes by administration of a unit dose of one or more additional therapeutic agents. Alternatively, a unit dose of one or more additional therapeutic agents is administered first, followed by administration of a unit dose of the pharmaceutical composition comprising the sodium salt of Formula **(Ia)** within seconds or minutes. In other embodiments, a unit dose of the pharmaceutical composition comprising the sodium salt of Formula **(Ia)** is administered first, followed, after a period of hours (for example, 1-12 hours), by administration of a unit dose of one or more additional therapeutic agents. In yet other embodiments, a unit dose of one or more additional therapeutic agents is administered first, followed, after a period of hours (for example, 1-12 hours), by administration of a unit dose of the pharmaceutical composition comprising the sodium salt of Formula **(Ia).**

For the avoidance of doubt, co-administration of the pharmaceutical composition comprising the sodium salt of Formula **(Ia)** with one or more additional therapeutic agents, may refer to co-administration with one or more of the therapeutic agents described herein.

In certain embodiments, the sodium salt of the compound of Formula **(Ia)** is combined with one or more additional therapeutic agents in a unitary dosage form for simultaneous administration to a patient. In certain embodiments, such a unitary dosage form can be administered parenterally (including subcutaneous, intramuscular, intravenous, intradermal, intrathecal and epidural). In certain embodiments, the compounds disclosed can be dosed parenterally. In certain embodiments, the unitary dosage form can be dosed intravenous, subcutaneous, or intramuscular.

In some embodiments, the sodium salt of the compound of Formula **(Ia)** is combined with one or more additional therapeutic agents in a unitary dosage form for simultaneous administration to a patient. In certain embodiments, such a unitary dosage form can be administered parenterally (including subcutaneous, intramuscular, intravenous, intradermal, intrathecal and epidural). In certain embodiments, the compounds disclosed can be dosed parenterally. In certain embodiments, the unitary dosage form can be dosed intravenous, subcutaneous, or intramuscular.

The pharmaceutical composition comprising the sodium salt of Formula **(Ia)** in combination with one or more additional therapeutic agents is administered parenterally (including subcutaneously, intramuscularly, intravenously, intradermally, intrathecally and epidurally). In certain embodiments, the compounds disclosed can be dosed parenterally. In certain embodiments, the pharmaceutical composition disclosed can be dosed intravenous, subcutaneous, or intramuscular.

The pharmaceutical composition comprising the sodium salt of the compound of Formula **(Ia)** is formulated as a solution formulation, which may optionally contain one or more other compounds useful for treating HIV. In certain embodiments, the solution can contain one or more other compounds useful for treating HIV, such as HIV protease inhibitors, HIV non-nucleoside or non-nucleotide inhibitors of reverse transcriptase, HIV nucleoside or nucleotide inhibitors of reverse transcriptase, HIV integrase inhibitors, HIV non-catalytic site (or allosteric) integrase inhibitors, HIV nucleoside reverse transcriptase translocation inhibitors, pharmacokinetic enhancers, and combinations thereof.

### HIV Combination Therapy

In some embodiments, the sodium salt of the compound of Formula **(Ia)** is administered with a therapeutically effective amount of at least one additional therapeutic agent.

In the above embodiments, the additional therapeutic agent can be an anti-HIV agent selected from the group consisting of combination drugs for treating HIV, other drugs for treating HIV, HIV nucleoside reverse transcriptase translocation inhibitors, HIV protease inhibitors, HIV non-nucleoside or non-nucleotide inhibitors of reverse transcriptase, HIV nucleoside or nucleotide inhibitors of reverse transcriptase, HIV integrase inhibitors, HIV non-catalytic site (or allosteric) integrase inhibitors, HIV entry inhibitors, HIV maturation inhibitors, latency reversing agents, compounds that target the HIV capsid, immune-based therapies, phosphatidylinositol 3-kinase (PI3K) inhibitors, HIV antibodies, bispecific antibodies and "antibody-like" therapeutic proteins, HIV p17 matrix protein inhibitors, IL-13 antagonists, peptidyl-prolyl cis-trans isomerase A modulators, protein disulfide isomerase inhibitors, complement C5a receptor antagonists, DNA methyltransferase inhibitor, HIV vif gene modulators, Vif dimerization antagonists, HIV-1 viral infectivity factor inhibitors, TAT protein inhibitors, HIV-1 Nef modulators, Hck tyrosine kinase modulators, mixed lineage kinase-3 (MLK-3) inhibitors, HIV-1 splicing inhibitors, Rev protein inhibitors, integrin antagonists, nucleoprotein inhibitors, splicing factor modulators, COMM domain containing protein 1 modulators, HIV ribonuclease H inhibitors, retrocyclin modulators, CDK-9 inhibitors, dendritic ICAM-3 grabbing nonintegrin 1 inhibitors, HIV GAG protein inhibitors, HIV POL protein inhibitors, Complement Factor H modulators, ubiquitin ligase inhibitors, deoxycytidine kinase inhibitors, cyclin dependent kinase inhibitors, proprotein convertase PC9 stimulators, ATP dependent RNA helicase DDX3X inhibitors, reverse transcriptase priming complex inhibitors, G6PD and NADH-oxidase inhibitors, pharmacokinetic enhancers, HIV gene therapy, HIV vaccines, and combinations thereof.

In some embodiments, the additional therapeutic agent is selected from immunomodulators, immunotherapeutic agents, antibody-drug conjugates, gene modifiers, gene editors (such as CRISPR/Cas9, zinc finger nucleases, homing nucleases, synthetic nucleases, TALENs), and cell therapies such as chimeric antigen receptor T-cell, CAR-T (e.g., YESCARTA^{®} (axicabtagene ciloleucel)), and engineered T cell receptors, TCR-T.

In some embodiments, the additional therapeutic agent is selected from the group consisting of combination drugs for HIV, other drugs for treating HIV, HIV nucleoside reverse transcriptase translocation inhibitors, HIV protease inhibitors, HIV reverse transcriptase inhibitors, HIV integrase inhibitors, HIV non-catalytic site (or allosteric) integrase inhibitors, HIV entry (fusion) inhibitors, HIV maturation inhibitors, latency reversing agents, capsid inhibitors, immune-based therapies, PI3K inhibitors, HIV antibodies, and bispecific antibodies, and "antibody-like" therapeutic proteins, and combinations thereof.

### HIV Combination Drugs

Examples of combination drugs include ATRIPLA^{®} (efavirenz, tenofovir disoproxil fumarate, and emtricitabine); COMPLERA^{®} (EVIPLERA^{®}; rilpivirine, tenofovir disoproxil fumarate, and emtricitabine); STRIBILD^{®} (elvitegravir, cobicistat, tenofovir disoproxil fumarate, and emtricitabine); TRUVADA^{®} (tenofovir disoproxil fumarate and emtricitabine; TDF+FTC); DESCOVY^{®} (tenofovir alafenamide and emtricitabine); ODEFSEY^{®} (tenofovir alafenamide, emtricitabine, and rilpivirine); GENVOYA^{®} (tenofovir alafenamide, emtricitabine, cobicistat, and elvitegravir); BIKTARVY^{®} (bictegravir, emtricitabine, and tenofovir alafenamide); darunavir, tenofovir alafenamide hemifumarate, emtricitabine, and cobicistat; efavirenz, lamivudine, and tenofovir disoproxil fumarate; lamivudine and tenofovir disoproxil fumarate; tenofovir and lamivudine; tenofovir alafenamide and emtricitabine; tenofovir alafenamide hemifumarate and emtricitabine; tenofovir alafenamide hemifumarate, emtricitabine, and rilpivirine; tenofovir alafenamide hemifumarate, emtricitabine, cobicistat, and elvitegravir; COMBIVIR^{®} (zidovudine and lamivudine; AZT+3TC); EPZICOM^{®} (LIVEXA^{®}; abacavir sulfate and lamivudine; ABC+3TC); KALETRA^{®} (ALUVIA^{®}; lopinavir and ritonavir); TRIUMEQ^{®} (dolutegravir, abacavir, and lamivudine); TRIZIVIR^{®} (abacavir sulfate, zidovudine, and lamivudine; ABC+AZT+3TC); atazanavir and cobicistat; atazanavir sulfate and cobicistat; atazanavir sulfate and ritonavir; darunavir and cobicistat; dolutegravir and rilpivirine; dolutegravir and rilpivirine hydrochloride; cabotegravir and rilpivirine; cabotegravir and rilpivirine hydrochloride; dolutegravir, abacavir sulfate, and lamivudine; lamivudine, nevirapine, and zidovudine; raltegravir and lamivudine; doravirine, lamivudine, and tenofovir disoproxil fumarate; doravirine, lamivudine, and tenofovir disoproxil; dolutegravir + lamivudine; lamivudine + abacavir + zidovudine; lamivudine + abacavir; lamivudine + tenofovir disoproxil fumarate; lamivudine + zidovudine + nevirapine; lopinavir + ritonavir; lopinavir + ritonavir + abacavir + lamivudine; lopinavir + ritonavir + zidovudine + lamivudine; tenofovir + lamivudine; and tenofovir disoproxil fumarate + emtricitabine + rilpivirine hydrochloride; lopinavir , ritonavir, zidovudine and lamivudine; Vacc-4x and romidepsin; and APH-0812.

### Other HIV Drugs

Examples of other drugs for treating HIV include acemannan, alisporivir, BanLec, deferiprone, Gamimune, metenkefalin, naltrexone, Prolastin, REP 9, RPI-MN, VSSP, H1viral, SB-728-T, 1,5-dicaffeoylquinic acid, rHIV7-shl-TAR-CCR5RZ, AAV-eCD4-Ig gene therapy, MazF gene therapy, BlockAide, ABX-464, AG-1105, APH-0812, BIT-225, CYT-107, HGTV-43, HPH-116, HS-10234, IMO-3100, IND-02, MK-1376, MK-8507, MK-8591, NOV-205, PA-1050040 (PA-040), PGN-007, SCY-635, SB-9200, SCB-719, TR-452, TEV-90110, TEV-90112, TEV-90111, TEV-90113, RN-18, Immuglo, and VIR-576.

### HIV nucleoside reverse transcriptase translocation inhibitors

Examples of HIV nucleoside reverse transcriptase translocation inhibitors ("NRTTIs") include 4'-ethynyl-2-fluoro-2'-deoxyadenosine triphosphate (also known as MK-8591 and EFdA).

### HIV Protease Inhibitors

Examples of HIV protease inhibitors include amprenavir, atazanavir, brecanavir, darunavir, fosamprenavir, fosamprenavir calcium, indinavir, indinavir sulfate, lopinavir, nelfinavir, nelfinavir mesylate, ritonavir, saquinavir, saquinavir mesylate, tipranavir, DG-17, TMB-657 (PPL-100), T-169, BL-008, and TMC-310911.

### HIV Reverse Transcriptase Inhibitors

Examples of HIV non-nucleoside or non-nucleotide inhibitors of reverse transcriptase include dapivirine, delavirdine, delavirdine mesylate, doravirine, efavirenz, etravirine, lentinan, nevirapine, rilpivirine, AIC-292, KM-023, and VM-1500. Further examples of non-nucleoside reverse transcriptase inhibitors are disclosed in U.S. Patent Publication No. US 2016/0250215.

Examples of HIV nucleoside or nucleotide inhibitors of reverse transcriptase include adefovir, adefovir dipivoxil, azvudine, emtricitabine, tenofovir, tenofovir alafenamide, tenofovir alafenamide fumarate, tenofovir alafenamide hemifumarate, tenofovir disoproxil, tenofovir disoproxil fumarate, tenofovir disoproxil hemifumarate, VIDEX^{®} and VIDEX EC^{®} (didanosine, ddl), abacavir, abacavir sulfate, alovudine, apricitabine, censavudine, didanosine, elvucitabine, festinavir, fosalvudine tidoxil, CMX-157, dapivirine, doravirine, etravirine, OCR-5753, tenofovir disoproxil orotate, fozivudine tidoxil, lamivudine, phosphazid, stavudine, zalcitabine, zidovudine, GS-9131, GS-9148, and KP-1461.

In some embodiments, examples of HIV nucleoside or nucleotide inhibitors of reverse transcriptase include adefovir, adefovir dipivoxil, azvudine, emtricitabine, tenofovir, tenofovir alafenamide, tenofovir alafenamide fumarate, tenofovir alafenamide hemifumarate, tenofovir disoproxil, tenofovir disoproxil fumarate, tenofovir disoproxil hemifumarate, VIDEX^{®} and VIDEX EC^{®} (didanosine, ddl), abacavir, abacavir sulfate, alovudine, apricitabine, censavudine, didanosine, elvucitabine, festinavir, fosalvudine tidoxil, CMX-157, dapivirine, doravirine, etravirine, OCR-5753, tenofovir disoproxil orotate, fozivudine tidoxil, lamivudine, phosphazid, stavudine, zalcitabine, zidovudine, GS-9131, GS-9148, KP-1461, and 4'-ethynyl-2-fluoro-2'-deoxyadenosine (EFdA).

### HIV Integrase Inhibitors

Examples of HIV integrase inhibitors include elvitegravir, curcumin, derivatives of curcumin, chicoric acid, derivatives of chicoric acid, 3,5-dicaffeoylquinic acid, derivatives of 3,5-dicaffeoylquinic acid, aurintricarboxylic acid, derivatives of aurintricarboxylic acid, caffeic acid phenethyl ester, derivatives of caffeic acid phenethyl ester, tyrphostin, derivatives of tyrphostin, quercetin, derivatives of quercetin, raltegravir, dolutegravir, JTK-351, bictegravir, AVX-15567, diketo quinolin-4-1 derivatives, integrase-LEDGF inhibitor, ledgins, M-522, M-532, NSC-310217, NSC-371056, NSC-48240, NSC-642710, NSC-699171, NSC-699172, NSC-699173, NSC-699174, stilbenedisulfonic acid, T-169 and cabotegravir.

Examples of HIV non-catalytic site, or allosteric, integrase inhibitors (NCINI) include CX-05045, CX-05168, and CX-14442.

### HIV Entry Inhibitors

Examples of HIV entry (fusion) inhibitors include cenicriviroc, CCR5 inhibitors, gp41 inhibitors, CD4 attachment inhibitors, gp120 inhibitors, and CXCR4 inhibitors.
Examples of CCR5 inhibitors include aplaviroc, vicriviroc, maraviroc, cenicriviroc, PRO-140, adaptavir (RAP-101), nifeviroc (TD-0232), anti-GP120/CD4 or CCR5 bispecific antibodies, B-07, MB-66, polypeptide C25P, TD-0680, and vMIP (Haimipu).
Examples of gp41 inhibitors include albuvirtide, enfuvirtide, BMS-986197, enfuvirtide biobetter, enfuvirtide biosimilar, HIV-1 fusion inhibitors (P26-Bapc), ITV-1, ITV-2, ITV-3, ITV-4, PIE-12 trimer and sifuvirtide.
Examples of CD4 attachment inhibitors include ibalizumab and CADA analogs
Examples of gp120 inhibitors include Radha-108 (receptol) 3B3-PE38, BanLec, bentonite-based nanomedicine, fostemsavir tromethamine, IQP-0831, and BMS-663068
Examples of CXCR4 inhibitors include plerixafor, ALT-1188, N15 peptide, and vMIP (Haimipu).

### HIV Maturation Inhibitors

Examples of HIV maturation inhibitors include BMS-955176 and GSK-2838232.

### Latency Reversing Agents

Examples of latency reversing agents include histone deacetylase (HDAC) inhibitors, proteasome inhibitors such as velcade, protein kinase C (PKC) activators, BET-bromodomain 4 (BRD4) inhibitors, ionomycin, PMA, SAHA (suberanilohydroxamic acid, or suberoyl, anilide, and hydroxamic acid), IL-15, JQ1, disulfram, amphotericin B, and ubiquitin inhibitors such as largazole analogs, and GSK-343.

Examples of HDAC inhibitors include romidepsin, vorinostat, and panobinostat.

Examples of PKC activators include indolactam, prostratin, ingenol B, and DAG-lactones.

### Capsid Inhibitors

Examples of capsid inhibitors include capsid polymerization inhibitors or capsid disrupting compounds, HIV nucleocapsid p7 (NCp7) inhibitors such as azodicarbonamide, HIV p24 capsid protein inhibitors, AVI-621, AVI-101, AVI-201, AVI-301, and AVI-CAN1-15 series.

### Immune-based Therapies

Examples of immune-based therapies include toll-like receptors modulators such as tlr1, tlr2, tlr3, tlr4, tlr5, tlr6, tlr7, tlr8, tlr9, tlr10, tlr11, tlr12, and tlr13; programmed cell death protein 1 (Pd-1) modulators; programmed death-ligand 1 (Pd-L1) modulators; IL-15 agonists; DermaVir; interleukin-7; plaquenil (hydroxychloroquine); proleukin (aldesleukin, IL-2); interferon alfa; interferon alfa-2b; interferon alfa-n3; pegylated interferon alfa; interferon gamma; hydroxyurea; mycophenolate mofetil (MPA) and its ester derivative mycophenolate mofetil (MMF); ribavirin; rintatolimod, polymer polyethyleneimine (PEI); gepon; rintatolimod; IL-12; WF-10; VGV-1; MOR-22; BMS-936559; CYT-107, interleukin-15/Fc fusion protein, normferon, peginterferon alfa-2a, peginterferon alfa-2b, recombinant interleukin-15, RPI-MN, GS-9620, and IR-103.

### Phosphatidylinositol 3-kinase (PI3K) Inhibitors

Examples of PI3K inhibitors include idelalisib, alpelisib, buparlisib, CAI orotate, copanlisib, duvelisib, gedatolisib, neratinib, panulisib, perifosine, pictilisib, pilaralisib, puquitinib mesylate, rigosertib, rigosertib sodium, sonolisib, taselisib, AMG-319, AZD-8186, BAY-1082439, CLR-1401, CLR-457, CUDC-907, DS-7423, EN-3342, GSK-2126458, GSK-2269577, GSK-2636771, INCB-040093, LY-3023414, MLN-1117, PQR-309, RG-7666, RP-6530, RV-1729, SAR-245409, SAR-260301, SF-1126, TGR-1202, UCB-5857, VS-5584, XL-765, and ZSTK-474.

### HIV Antibodies, Bispecific Antibodies, and "Antibody-like" Therapeutic Proteins

Examples of HIV antibodies, bispecific antibodies, and "antibody-like" therapeutic proteins include DARTs^{®}, DUOBODIES^{®}, BITES^{®}, XmAbs^{®}, TandAbs^{®}, Fab derivatives, bnABs (broadly neutralizing HIV-1 antibodies), BMS-936559, TMB-360, and those targeting HIV gp120 or gp41, antibody-Recruiting Molecules targeting HIV, anti-CD63 monoclonal antibodies , anti-GB virus C antibodies, anti-GP120/CD4, CCR5 bispecific antibodies, anti-nef single domain antibodies, anti-Rev antibody, camelid derived anti-CD18 antibodies, camelid-derived anti-ICAM-1 antibodies, DCVax-001, gp140 targeted antibodies, gp41-based HIV therapeutic antibodies, human recombinant mAbs (PGT-121), ibalizumab, Immuglo, MB-66

Examples of those targeting HIV in such a manner include bavituximab, UB-421, C2F5, C2G12, C4E10, C2F5+C2G12+C4E10, 3-BNC-117, PGT145, PGT121, MDX010 (ipilimumab), VRC01, A32, 7B2, 10E8, VRC-07-523, VRC-HIVMAB080-00-AB, MGD-014 and VRC07.

### Pharmacokinetic Enhancers

Examples of pharmacokinetic enhancers include cobicistat and ritonavir.

### Additional Therapeutic Agents

Examples of additional therapeutic agents include the compounds disclosed in WO 2004/096286 (Gilead Sciences), WO 2006/015261 (Gilead Sciences), WO 2006/110157 (Gilead Sciences), WO 2012/003497 (Gilead Sciences), WO 2012/003498 (Gilead Sciences), WO 2012/145728 (Gilead Sciences), WO 2013/006738 (Gilead Sciences), WO 2013/159064 (Gilead Sciences), WO 2014/100323 (Gilead Sciences), US 2013/0165489 (University of Pennsylvania), US 2014/0221378 (Japan Tobacco), US 2014/0221380 (Japan Tobacco), WO 2009/062285 (Boehringer Ingelheim), WO 2010/130034 (Boehringer Ingelheim), WO 2013/006792 (Pharma Resources), US 20140221356 (Gilead Sciences), US 20100143301 (Gilead Sciences) and WO 2013/091096 (Boehringer Ingelheim).

### HIV Vaccines

Examples of HIV vaccines include peptide vaccines, recombinant subunit protein vaccines, live vector vaccines, DNA vaccines, CD4-derived peptide vaccines, vaccine combinations, rgp120 (AIDSVAX), ALVAC HIV (vCP1521)/AIDSVAX B/E (gp120) (RV144), monomeric gp120 HIV-1 subtype C vaccine, Remune, ITV-1, Contre Vir, Ad5-ENVA-48, DCVax-001 (CDX-2401), Vacc-4x, Vacc-C5, VAC-3S, multiclade DNA recombinant adenovirus-5 (rAd5), Pennvax-G, Pennvax-GP, HIV-TriMix-mRNA vaccine, HIV-LAMP-vax, Ad35, Ad35-GRIN, NAcGM3/VSSP ISA-51, poly-ICLC adjuvanted vaccines, TatImmune, GTU-multiHIV (FIT-06), gp140[delta]V2.TV1+MF-59, rVSVIN HIV-1 gag vaccine, SeV-Gag vaccine, AT-20, DNK-4, ad35-Grin/ENV, TBC-M4, HIVAX, HIVAX-2, NYVAC-HIV-PT1, NYVAC-HIV-PT4, DNA-HIV-PT123, rAAV1-PG9DP, GOVX-B11, GOVX-B21, TVI-HIV-1, Ad-4 (Ad4-env Clade C+Ad4-mGag), EN41-UGR7C, EN41-FPA2, PreVaxTat, AE-H, MYM-V101, CombiHIVvac, ADVAX, MYM-V201, MVA-CMDR, DNA-Ad5 gag/pol/nef/nev (HVTN505), MVATG-17401, ETV-01, CDX-1401, rcAD26.MOS1.HIV-Env, Ad26.Mod.HIV vaccine, AGS-004, AVX-101, AVX-201, PEP-6409, SAV-001, ThV-01, TL-01, TUTI-16, VGX-3300, IHV-001, and virus-like particle vaccines such as pseudovirion vaccine, CombiVICHvac, LFn-p24 B/C fusion vaccine, GTU-based DNA vaccine, HIV gag/pol/nef/env DNA vaccine, anti-TAT HIV vaccine, conjugate polypeptides vaccine, dendritic-cell vaccines, gag-based DNA vaccine, GI-2010, gp41 HIV-1 vaccine, HIV vaccine (PIKA adjuvant), I i-key/MHC class II epitope hybrid peptide vaccines, ITV-2, ITV-3, ITV-4, LIPO-5, multiclade Env vaccine, MVA vaccine, Pennvax-GP, pp71-deficient HCMV vector HIV gag vaccine, recombinant peptide vaccine (HIV infection), NCI, rgp160 HIV vaccine, RNActive HIV vaccine, SCB-703, Tat Oyi vaccine, TBC-M4, therapeutic HIV vaccine, UBI HIV gp120, Vacc-4x + romidepsin, variant gp120 polypeptide vaccine, rAd5 gag-pol env A/B/C vaccine.

### HIV Combination Therapy

In a particular embodiment, the sodium salt of the compound of Formula (Ia) is combined with one, two, three, four or more additional therapeutic agents selected from ATRIPLA^{®} (efavirenz, tenofovir disoproxil fumarate, and emtricitabine); COMPLERA^{®} (EVIPLERA^{®}; rilpivirine, tenofovir disoproxil fumarate, and emtricitabine); STRIBILD^{®} (elvitegravir, cobicistat, tenofovir disoproxil fumarate, and emtricitabine); TRUVADA^{®} (tenofovir disoproxil fumarate and emtricitabine; TDF +FTC); DESCOVY^{®} (tenofovir alafenamide and emtricitabine); ODEFSEY^{®} (tenofovir alafenamide, emtricitabine, and rilpivirine); GENVOYA^{®} (tenofovir alafenamide, emtricitabine, cobicistat, and elvitegravir); adefovir; adefovir dipivoxil; cobicistat; emtricitabine; tenofovir; tenofovir disoproxil; tenofovir disoproxil fumarate; tenofovir alafenamide; tenofovir alafenamide hemifumarate; TRIUMEQ^{®} (dolutegravir, abacavir, and lamivudine); dolutegravir, abacavir sulfate, and lamivudine; raltegravir; raltegravir and lamivudine; maraviroc; enfuvirtide; ALUVIA^{®} (KALETRA^{®}; lopinavir and ritonavir); COMBIVIR^{®} (zidovudine and lamivudine; AZT+3TC); EPZICOM^{®} (LIVEXA^{®}; abacavir sulfate and lamivudine; ABC+3TC); TRIZIVIR^{®} (abacavir sulfate, zidovudine, and lamivudine; ABC+AZT+3TC); rilpivirine; rilpivirine hydrochloride; atazanavir sulfate and cobicistat; atazanavir and cobicistat; darunavir and cobicistat; atazanavir; atazanavir sulfate; dolutegravir; elvitegravir; ritonavir; atazanavir sulfate and ritonavir; darunavir; lamivudine; prolastin; fosamprenavir; fosamprenavir calcium efavirenz; etravirine; nelfinavir; nelfinavir mesylate; interferon; didanosine; stavudine; indinavir; indinavir sulfate; tenofovir and lamivudine; zidovudine; nevirapine; saquinavir; saquinavir mesylate; aldesleukin; zalcitabine; tipranavir; amprenavir; delavirdine; delavirdine mesylate; Radha-108 (receptol); lamivudine and tenofovir disoproxil fumarate; efavirenz, lamivudine, and tenofovir disoproxil fumarate; phosphazid; lamivudine, nevirapine, and zidovudine; abacavir; and abacavir sulfate.

In some embodiments, the sodium salt of the compound of Formula (Ia) is combined with one, two, three, four or more additional therapeutic agents selected from ATRIPLA^{®} (efavirenz, tenofovir disoproxil fumarate, and emtricitabine); COMPLERA^{®} (EVIPLERA^{®}; rilpivirine, tenofovir disoproxil fumarate, and emtricitabine); STRIBILD^{®} (elvitegravir, cobicistat, tenofovir disoproxil fumarate, and emtricitabine); TRUVADA^{®} (tenofovir disoproxil fumarate and emtricitabine; TDF +FTC); DESCOVY^{®} (tenofovir alafenamide and emtricitabine); ODEFSEY^{®} (tenofovir alafenamide, emtricitabine, and rilpivirine); GENVOYA^{®} (tenofovir alafenamide, emtricitabine, cobicistat, and elvitegravir); adefovir; adefovir dipivoxil; cobicistat; emtricitabine; tenofovir; tenofovir disoproxil; tenofovir disoproxil fumarate; tenofovir alafenamide; tenofovir alafenamide hemifumarate; TRIUMEQ^{®} (dolutegravir, abacavir, and lamivudine); dolutegravir, abacavir sulfate, and lamivudine; raltegravir; raltegravir and lamivudine; maraviroc; enfuvirtide; ALUVIA^{®} (KALETRA^{®}; lopinavir and ritonavir); COMBIVIR^{®} (zidovudine and lamivudine; AZT+3TC); EPZICOM^{®} (LIVEXA^{®}; abacavir sulfate and lamivudine; ABC+3TC); TRIZIVIR^{®} (abacavir sulfate, zidovudine, and lamivudine; ABC+AZT+3TC); rilpivirine; rilpivirine hydrochloride; atazanavir sulfate and cobicistat; atazanavir and cobicistat; darunavir and cobicistat; atazanavir; atazanavir sulfate; dolutegravir; elvitegravir; ritonavir; atazanavir sulfate and ritonavir; darunavir; lamivudine; prolastin; fosamprenavir; fosamprenavir calcium efavirenz; etravirine; nelfinavir; nelfinavir mesylate; interferon; didanosine; stavudine; indinavir; indinavir sulfate; tenofovir and lamivudine; zidovudine; nevirapine; saquinavir; saquinavir mesylate; aldesleukin; zalcitabine; tipranavir; amprenavir; delavirdine; delavirdine mesylate; Radha-108 (receptol); lamivudine and tenofovir disoproxil fumarate; efavirenz, lamivudine, and tenofovir disoproxil fumarate; phosphazid; lamivudine, nevirapine, and zidovudine; abacavir; abacavir sulfate; 4'-ethynyl-2-fluoro-2'-deoxyadenosine (EFdA); and bictegravir, or a pharmaceutically acceptable salt thereof.

It will be appreciated by one of skill in the art that the additional therapeutic agents listed above may be included in more than one of the classes listed above. The particular classes are not intended to limit the functionality of those compounds listed in those classes.

In some embodiments, the sodium salt of the compound of Formula **(Ia)** is combined with at least one HIV nucleoside reverse transcriptase translocation inhibitor. In some embodiments, the sodium salt of the compound of Formula **(Ia)** is combined with at least one HIV nucleoside or nucleotide inhibitor of reverse transcriptase. In a specific embodiment, the sodium salt of the compound of Formula **(Ia)** is combined with at least one HIV nucleoside or nucleotide inhibitor of reverse transcriptase and at least one HIV non-nucleoside inhibitor of reverse transcriptase. In another specific embodiment, the sodium salt of the compound of Formula **(Ia)** is combined with an HIV nucleoside or nucleotide inhibitor of reverse transcriptase, and an HIV protease inhibiting compound. In an additional embodiment, the sodium salt of the compound of Formula **(Ia)** is combined with an HIV nucleoside or nucleotide inhibitor of reverse transcriptase, an HIV non-nucleoside inhibitor of reverse transcriptase, and a pharmacokinetic enhancer. In certain embodiments, the sodium salt of the compound of Formula **(Ia)** is combined with at least one HIV nucleoside inhibitor of reverse transcriptase, an integrase inhibitor, and a pharmacokinetic enhancer. In another embodiment, the sodium salt of Formula **(Ia)** is combined with two HIV nucleoside or nucleotide inhibitors of reverse transcriptase.

In some embodiments, the sodium salt of the compound of Formula **(Ia)** is combined with one or two HIV nucleoside or nucleotide inhibitors of reverse transcriptase. In a specific embodiment, the sodium salt of the compound of Formula **(Ia)** is combined with an HIV nucleoside or nucleotide inhibitor of reverse transcriptase and an HIV non-nucleoside inhibitor of reverse transcriptase. In another specific embodiment, the sodium salt of the compound of Formula **(Ia)** is combined with an HIV nucleoside or nucleotide inhibitor of reverse transcriptase, and an HIV protease inhibiting compound. In an additional embodiment, the sodium salt of the compound of Formula **(Ia)** is combined with an HIV nucleoside or nucleotide inhibitor of reverse transcriptase, an HIV non-nucleoside inhibitor of reverse transcriptase, and a pharmacokinetic enhancer. In certain embodiments, the sodium salt of the compound of Formula **(Ia)** is combined with at least one HIV nucleoside inhibitor of reverse transcriptase, an integrase inhibitor, and a pharmacokinetic enhancer. In another embodiment, the sodium salt of the compound of Formula **(Ia)** is combined with two HIV nucleoside or nucleotide inhibitors of reverse transcriptase.

In some embodiments, the sodium salt of the compound of Formula **(Ia)** is combined with abacavir sulfate, tenofovir, tenofovir disoproxil, tenofovir disoproxil fumarate, tenofovir disoproxil hemifumarate, tenofovir alafenamide, tenofovir alafenamide fumarate, tenofovir alafenamide hemifumarate, bictegravir (or a pharmaceutically acceptable salt thereof), or 4'-ethynyl-2-fluoro-2'-deoxyadenosine (EFdA).

In a particular embodiment, the sodium salt of the compound of Formula **(Ia)** is combined with at least one HIV nucleoside reverse transcriptase translocation inhibitor. In some embodiments, the HIV nucleoside reverse transcriptase translocation inhibitor is 4'-ethynyl-2-fluoro-2'-deoxyadenosine triphosphate.

In some embodiments, the sodium salt of the compound of Formula **(Ia)** is combined with a HIV integrase inhibitor. In some embodiments, the HIV integrase inhibitor is bictegravir.

In a particular embodiment, the sodium salt of the compound of Formula **(Ia)** is combined with a nucleotide reverse transcriptase inhibitor. In some embodiments, the nucleotide reverse transcriptase inhibitor is selected from the group consisting of tenofovir, tenofovir disoproxil, tenofovir disoproxil fumarate, tenofovir alafenamide, tenofovir alafenamide fumarate, and tenofovir alafenamide hemifumarate.

In some embodiments, the sodium salt of the compound of Formula **(Ia)** is combined with tenofovir, tenofovir disoproxil, tenofovir disoproxil fumarate, tenofovir alafenamide, tenofovir alafenamide fumarate, tenofovir alafenamide hemifumarate, bictegravir (or a pharmaceutically acceptable salt thereof), or 4'-ethynyl-2-fluoro-2'-deoxyadenosine (EFdA).

In a particular embodiment, the sodium salt of the compound of Formula **(Ia)** is combined with a first additional therapeutic agent selected from the group consisting of abacavir sulfate, tenofovir, tenofovir disoproxil, tenofovir disoproxil fumarate, tenofovir alafenamide, tenofovir alafenamide fumarate and tenofovir alafenamide hemifumarate, and a second additional therapeutic agent selected from the group consisting of emtricitabine and lamivudine.

In some embodiments, the sodium salt of the compound of Formula **(Ia)** is combined with a first additional therapeutic agent selected from the group consisting of abacavir sulfate, tenofovir, tenofovir disoproxil, tenofovir disoproxil fumarate, tenofovir alafenamide, tenofovir alafenamide fumarate and tenofovir alafenamide hemifumarate, and a second additional therapeutic agent selected from the group consisting of emtricitabine and lamivudine.

In a particular embodiment, the sodium salt of the compound of Formula **(Ia)** is combined with a first additional therapeutic agent selected from the group consisting of tenofovir, tenofovir disoproxil, tenofovir disoproxil fumarate, tenofovir alafenamide, and tenofovir alafenamide hemifumarate, and a second additional therapeutic agent, wherein the second additional therapeutic agent is emtricitabine. In a particular embodiment, the sodium salt of the compound of Formula **(Ia)** is combined with a first additional therapeutic agent selected from the group consisting of tenofovir alafenamide fumarate, tenofovir alafenamide, and tenofovir alafenamide hemifumarate, and a second additional therapeutic agent, wherein the second additional therapeutic agent is emtricitabine. In a particular embodiment, the sodium salt of the compound of Formula **(Ia)** is combined with a first additional therapeutic agent selected from the group consisting of tenofovir disoproxil fumarate, tenofovir disoproxil, and tenofovir disoproxil hemifumarate, and a second additional therapeutic agent, wherein the second additional therapeutic agent is emtricitabine. In some embodiments, the sodium salt of the compound of Formula **(Ia)** and the first and second additional therapeutic agents as disclosed above are administered simultaneously. Optionally, the pharmaceutical composition comprising the sodium salt of the compound of Formula **(Ia)** and the first and second additional therapeutic agents as disclosed above are combined in a unitary dosage form for simultaneous administration to a patient. In other embodiments, the pharmaceutical composition comprising the sodium salt of compound of Formula **(Ia)** and the first and second additional therapeutic agents as disclosed above are administered sequentially.

In some embodiments, the sodium salt of the compound of Formula **(Ia)** is combined with a first additional therapeutic agent selected from the group consisting of tenofovir, tenofovir disoproxil, tenofovir disoproxil fumarate, tenofovir alafenamide, and tenofovir alafenamide hemifumarate, and a second additional therapeutic agent, wherein the second additional therapeutic agent is emtricitabine. In a particular embodiment, the sodium salt of the compound of Formula **(Ia)** is combined with a first additional therapeutic agent selected from the group consisting of tenofovir alafenamide fumarate, tenofovir alafenamide, and tenofovir alafenamide hemifumarate, and a second additional therapeutic agent, wherein the second additional therapeutic agent is emtricitabine. In a particular embodiment, the sodium salt of the compound of Formula **(Ia)** is combined with a first additional therapeutic agent selected from the group consisting of tenofovir disoproxil fumarate, tenofovir disoproxil, and tenofovir disoproxil hemifumarate, and a second additional therapeutic agent, wherein the second additional therapeutic agent is emtricitabine. In some embodiments, the sodium salt of the compound of Formula **(Ia)** and the first and second additional therapeutic agents as disclosed above are administered simultaneously. Optionally, the sodium salt of the compound of Formula **(Ia)** and the first and second additional therapeutic agents as disclosed above are combined in a unitary dosage form for simultaneous administration to a patient. In other embodiments, the pharmaceutical composition comprising the sodium salt of a compound of Formula **(Ia)** and the first and second additional therapeutic agents as disclosed above are administered sequentially.

In some embodiments, the sodium salt of the compound of Formula **(Ia)** is combined with bictegravir.

The sodium salt of the compound of Formula **(Ia)** may be combined with one or more additional therapeutic agents in any dosage amount of the compound of Formula **(Ia)** (for example, from about 1 mg to about 1000 mg of compound).

In some embodiments, a compound as disclosed herein (for example, a compound of Formula **(Ia))** may be combined with one or more additional therapeutic agents in any dosage amount of a compound of Formula **(Ia)** (for example, from about 1 mg to about 1000 mg of compound).

In certain embodiments, the sodium salt of Formula **(Ia)** is combined with about 5-30 mg tenofovir alafenamide fumarate, tenofovir alafenamide hemifumarate, or tenofovir alafenamide, and about 200 mg emtricitabine. In certain embodiments, the sodium salt of the compound of Formula **(Ia)** is combined with about 5-10 mg, about 5-15 mg, about 5-20 mg, about 5-25 mg, about 25-30 mg, about 20-30 mg, about 15-30 mg, or about 10-30 mg tenofovir alafenamide fumarate, tenofovir alafenamide hemifumarate, or tenofovir alafenamide, and about 200 mg emtricitabine. In certain embodiments, the sodium salt of the compound of Formula **(Ia)** is combined with about 10 mg tenofovir alafenamide fumarate, tenofovir alafenamide hemifumarate, or tenofovir alafenamide, and about 200 mg emtricitabine. In certain embodiments, a compound disclosed herein is combined with about 25 mg tenofovir alafenamide fumarate, tenofovir alafenamide hemifumarate, or tenofovir alafenamide, and about 200 mg emtricitabine. A compound of Formula **(Ia)** can be combined with the agents provided herein in any dosage amount of the compound (for example, from about 1 mg to about 1000 mg of compound), the same as if each combination of dosages were specifically and individually listed.

In some embodiments, the sodium salt of the compound of Formula **(Ia)** is combined with about 5-30 mg tenofovir alafenamide fumarate, tenofovir alafenamide hemifumarate, or tenofovir alafenamide, and about 200 mg emtricitabine. In certain embodiments, the sodium salt of the compound of Formula **(Ia)** is combined with about 5-10 mg, about 5-15 mg, about 5-20 mg, about 5-25 mg, about 25-30 mg, about 20-30 mg, about 15-30 mg, or about 10-30 mg tenofovir alafenamide fumarate, tenofovir alafenamide hemifumarate, or tenofovir alafenamide, and about 200 mg emtricitabine. In certain embodiments, the sodium salt of the compound of Formula **(Ia)** is combined with about 10 mg tenofovir alafenamide fumarate, tenofovir alafenamide hemifumarate, or tenofovir alafenamide, and about 200 mg emtricitabine. In certain embodiments, a compound disclosed herein is combined with about 25 mg tenofovir alafenamide fumarate, tenofovir alafenamide hemifumarate, or tenofovir alafenamide, and about 200 mg emtricitabine. The compound of Formula **(Ia)** can be combined with the agents provided herein in any dosage amount of the compound (for example, from about 1 mg to about 1000 mg of compound), the same as if each combination of dosages were specifically and individually listed.

In certain embodiments, the sodium salt of the compound of Formula **(Ia)** is combined with about 200-400 mg tenofovir disoproxil fumarate, tenofovir disoproxil hemifumarate, or tenofovir disoproxil, and about 200 mg emtricitabine. In certain embodiments, the sodium salt of the compound of Formula **(Ia)** is combined with about 200-250 mg, about 200-300 mg, about 200-350 mg, about 250-350 mg, about 250-400 mg, about 350-400 mg, about 300-400 mg, or about 250-400 mg tenofovir disoproxil fumarate, tenofovir disoproxil hemifumarate, or tenofovir disoproxil, and about 200 mg emtricitabine. In certain embodiments, the sodium salt of Formula **(Ia)** is combined with about 300 mg tenofovir disoproxil fumarate, tenofovir disoproxil hemifumarate, or tenofovir disoproxil, and about 200 mg emtricitabine. The compound of Formula **(Ia)** can be combined with the agents provided herein in any dosage amount of the compound (for example, from about 1 mg to about 1000 mg of compound), the same as if each combination of dosages were specifically and individually listed.

In some embodiments, the sodium salt of the compound of Formula **(Ia)** is combined with about 200-400 mg tenofovir disoproxil fumarate, tenofovir disoproxil hemifumarate, or tenofovir disoproxil, and about 200 mg emtricitabine. In certain embodiments, the sodium salt of the compound of Formula **(Ia)** is combined with about 200-250 mg, about 200-300 mg, about 200-350 mg, about 250-350 mg, about 250-400 mg, about 350-400 mg, about 300-400 mg, or about 250-400 mg tenofovir disoproxil fumarate, tenofovir disoproxil hemifumarate, or tenofovir disoproxil, and about 200 mg emtricitabine. In certain embodiments, the sodium salt of the compound of Formula **(Ia)** is combined with about 300 mg tenofovir disoproxil fumarate, tenofovir disoproxil hemifumarate, or tenofovir disoproxil, and about 200 mg emtricitabine. The compound of Formula **(Ia)** can be combined with the agents provided herein in any dosage amount of the compound (for example, from about 1 mg to about 1000 mg of compound), the same as if each combination of dosages were specifically and individually listed.

In some embodiments, the sodium salt of the compound of Formula **(Ia)** is combined with about 20-80 mg of bictegravir. In some embodiments, the sodium salt of Formula **(Ia)** is combined with about 50 mg of bictegravir. The compound of Formula **(Ia)** can be combined with the agents provided herein in any dosage amount of the compound (for example, from about 1 mg to about 1000 mg of compound), the same as if each combination of dosages were specifically and individually listed.

In some embodiments, the pharmaceutical composition comprising the sodium salt of the compound of Formula **(Ia)** is for use in the methods of treating human immunodeficiency virus (HIV) in a heavily treatment-experienced patient, wherein the methods further comprise administering one other therapeutic agent selected from the group consisting of tenofovir alafenamide, tenofovir alafenamide hemifumarate, and bictegravir, wherein the one other therapeutic agent is simultaneously administerd or co-administered with the pharmaceutical composition comprising the sodium salt of the compound of Formula **(Ia).** In some embodiments, the pharmaceutical composition comprising the sodium salt of the compound of Formula **(Ia)** is for use in methods of treating human immunodeficiency virus (HIV) in a heavily treatment-experienced patient, wherein the methods further comprise administering one other therapeutic agent selected from the group consisting of tenofovir alafenamide hemifumarate and bictegravir, wherein the one other therapeutic agent is simultaneously administerd or co-administered with the pharmaceutical composition comprising the sodium salt of the compound of Formula **(Ia).** In some embodiments, the pharmaceutical composition comprising the sodium salt of the compound of Formula **(Ia)** is for use in methods of treating human immunodeficiency virus (HIV) in a heavily treatment-experienced patient, wherein the methods further comprise simultaneously administering or co-administering tenofovir alafenamide with the pharmaceutical composition comprising the sodium salt of the compound of Formula **(Ia).** In some embodiments, the pharmaceutical composition comprising the sodium salt of the compound of Formula **(Ia)** is for use in methods of treating human immunodeficiency virus (HIV) in a heavily treatment-experienced patient, wherein the methods further comprise simultaneously administering or co-administering tenofovir alafenamide hemifumarate with the pharmaceutical composition comprising the sodium salt of the compound of Formula **(Ia).** In some embodiments, the pharmaceutical composition comprising the sodium salt of the compound of Formula **(Ia)** is for use in methods of treating human immunodeficiency virus (HIV) in a heavily treatment-experienced patient, wherein the methods further comprise simultaneously administering or co-administering bictegravir with the pharmaceutical composition comprising the sodium salt of the compound of Formula **(Ia).**

In one embodiment, kits comprising a pharmaceutical composition comprising the sodium salt of the compound of Formula **(Ia)** according to the claims in combination with one or more (for example, one, two, three, one or two, or one to three) additional therapeutic agents are provided.

In some embodiments, kits comprising a pharmaceutical composition comprising the sodium salt of the compound of Formula **(Ia)** according to the claims in combination with one or more (for example, one, two, three, one or two, or one to three) additional therapeutic agents are provided.

### Kits and Articles of Manufacture

In one embodiment of the disclosure, a kit comprising a pharmaceutical composition comprising the sodium salt of the compound of Formula **(Ia)** according to the claims is provided. In one embodiment of the disclosure, the kit contains one or more additional therapeutic agents as described herein. The kit can further contain instructions for use, for example, instructions for use in inhibiting an HIV reverse transcriptase, such as for use in treating an HIV infection or AIDS or as a research tool. The instructions for use are generally written instructions, although electronic storage media (for example, magnetic diskette or optical disk) containing instructions are also acceptable.

In one embodiment of the disclosure, a pharmaceutical kit containing one or more containers that contain a pharmaceutical composition comprising the sodium salt of the compound of Formula **(Ia)** according to the claims is provided. Optionally associated with such container(s) can be a notice in the form prescribed by a governmental agency regulating the manufacture, use or sale of pharmaceuticals. In some embodiments, the notice reflects approval by the agency for the manufacture, use, or sale for human administration. Each component (if there is more than one component) can be packaged in separate containers or some components can be combined in one container where cross-reactivity and shelf life permit. The kits can be in unit dosage forms, bulk packages (for example, multi-dose packages) or sub-unit doses. Kits can also include multiple unit doses of the compounds and instructions for use and be packaged in quantities sufficient for storage and use in pharmacies (for example, hospital pharmacies and compounding pharmacies).

Also in one embodiment of the disclosure are articles of manufacture comprising a unit dosage of the compound of Formula **(Ia)** according to the claims in suitable packaging for use in the methods described herein. Suitable packaging is known in the art and includes, for example, vials, vessels, ampules, bottles, jars, flexible packaging and the like. An article of manufacture can further be sterilized and/or sealed.

### EXAMPLES

### Example 1: Synthesis of the compound of Formula (Ia)

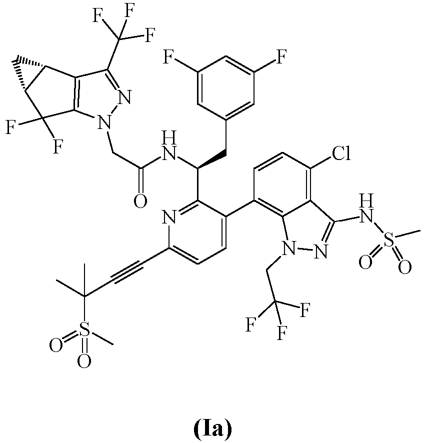

N-((S)-1-(3-(4-chloro-3-(methylsulfonamido)-1-(2,2,2-trifluoroethyl)-1H-indazol-7-yl)-6-(3-methyl-3-(methylsulfonyl)but-1-yn-1-yl)pyridin-2-yl)-2-(3,5-difluorophenyl)ethyl)-2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamide **(Ia),** was synthesized according the methods described below and is also described in WO 2018/035359. The compound structure for the compound of Formula (Ia) provided above may also be named or identified as 1H-Cyclopropa[3,4]cyclopenta[1,2]pyrazole-1-acetamide, N-[(1S)-1-[3-[4-chloro-3-[(methylsulfonyl)amino]-1-(2,2,2-trifluoroethyl)-1H-indazol-7-yl]-6-[3-methyl-3-(methylsulfonyl)-1-butyn-1-yl]-2-pyridinyl]-2-(3,5-difluorophenyl)ethyl]-5,5-difluoro-3b,4,4a,5-tetrahydro-3-(trifluoromethyl)-, (3bS,4aR)- under CAS; CAS Registry Number 2189684-44-2.

### A. Preparation of compounds (8a) and (8b)

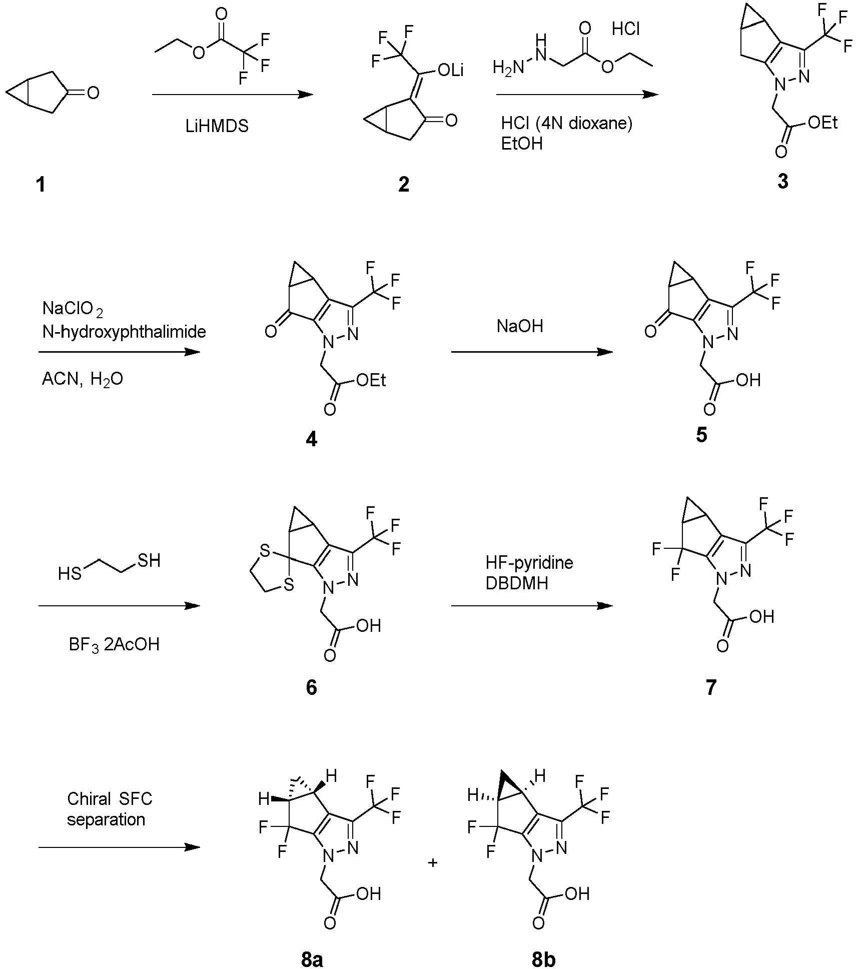

Synthesis of lithium 2,2,2-trifluoro-1-(3-oxobicyclo[3.1.0]hexan-2-ylidene)ethan-1-olate (2): A reactor was charged with bicyclo[3.1.0]hexan-3-one (1) (95.6 g, 0.99 mol), ethyl 2,2,2-trifluoroacetate (113.2 mL, 0.95 mol), and THF (50 mL). The reaction mixture was cooled to 0 °C. LiHMDS (lithium bis(trimethylsilyl)amide) (1 L of 1.0 M solution in THF, 1 mol) was added via an addition funnel at a rate to maintain an internal temperature of ≤ 1 °C. After the addition was complete, hexanes (235 mL) was added in a steady stream via an addition funnel and stirred for 15 min. The resultant solids were collected by filtration, washed with hexanes (3 x 400 mL), and dried to provide the title compound.

Synthesis of ethyl 2-(3-(trifluoromethyl)-3b,4,4a,5-tetrahydro-1H-cyclopropa[3,4]cyclopenta[1,2-c]pyrazol-1-yl)acetate **(3):** A reactor was charged with lithium 2,2,2-trifluoro-1-(3-oxobicyclo[3.1.0]hexan-2-ylidene)ethan-1-olate **(2)** (177.2 g, 0.89 mol) and EtOH (ethanol) (779 mL). The temperature was brought to and maintained at 0 °C. HCl in dioxane (4.0 N, 443 mL) was added via an addition funnel followed by the addition of solid ethyl hydrazinoacetate HCl salt (138.4 g, 0.90 mol). The reaction temperature was adjusted to 35 °C. After 1 h, the reaction volume was reduced by ~40% by distillation at reduced pressure. Water (1.3 L) was added with vigorous agitation and the temperature was adjusted to 15 °C. The resultant solids were collected by filtration, washed with water (3 x 500 mL), then hexanes (3 x 400 mL), and dried to provide the title compound. MS *(m*/*z)* 275.1 [M+H]⁺.

Synthesis of ethyl 2-(5-oxo-3-(trifluoromethyl)-3b,4,4a,5-tetrahydro-1H-cyclopropa[3,4]cyclopenta[1,2-c]pyrazol-1-yl)acetate **(4):** A reactor was charged with ethyl 2-(3-(trifluoromethyl)-3b,4,4a,5-tetrahydro-1H-cyclopropa[3,4]cyclopenta[1,2-c]pyrazol-1-yl)acetate **(3)** (291.2 g, 1.06 mol), acetonitrile (1.65 L) and water (825 mL) to which N-hydroxyphthalimide (17.4 g, 0.103 mol) and NaClO₂ (41.0 g, 0.45 mol, ~20% of total amount to be added) were added. The reaction mixture was heated to 50 °C and the remaining NaClO₂ (163.0 g, 1.80 mol) was added in five portions over 2 h. After consumption of starting material, the temperature was adjusted to 20 °C and aqueous sodium bisulfite (40% w/w, 350 mL) was added via an addition funnel. Ethyl acetate (1.75 L) was added and the layers were separated. The aqueous layer was back extracted with EtOAc (ethyl acetate) (500 mL). The organic layers were combined and washed with saturated aqueous NaHCO₃ (500 mL) and 1:1 water/brine (500 mL). The organic layer was concentrated under reduced pressure and co-evaporated with IPAc (isopropyl acetate) (300 mL). The crude solid was crystallized from a mixture of IPAc/heptane. The resultant solids were collected by filtration, washed with heptane, and dried to provide the title compound. MS *(m*/*z)* 289.0 [M+H]⁺.

Synthesis of 2-(5-oxo-3-(trifluoromethyl)-3b,4,4a,5-tetrahydro-1H-cyclopropa[3,4]cyclopenta[1,2-c]pyrazol-1-yl)acetic acid **(5)**: To a solution of ethyl 2-(5-oxo-3-(trifluoromethyl)-3b,4,4a,5-tetrahydro-1H-cyclopropa[3,4]cyclopenta[1,2-c]pyrazol-1-yl)acetate **(4)** (80.40 g, 278.95 mmol) in 2-MeTHF (2-methyltetrahydrofuran) (167 mL) was added 2M aqueous sodium hydroxide (167 mL). After 25 minutes of stirring at room temperature, the reaction mixture was diluted with 2-MeTHF and was slowly acidified by the dropwise addition of concentrated HCl. The organic layer was isolated and the aqueous layer was extracted with an additional portion of 2-MeTHF. The combined organic layers were washed with saturated aqueous sodium chloride, then dried over sodium sulfate, filtered, and concentrated. The resulting oil was taken in ethyl acetate. Hexanes was added with vigorous stirring until solid formation was observed. The solid was isolated by filtration and dried to provide the title compound. MS *(m*/*z)* 259.00 [M-H]⁻.

Synthesis of 2-(3-(trifluoromethyl)-4,4a-dihydrospiro[cyclopropa[3,4]cyclopenta[1,2-c]pyrazole-5,2'-[1,3]dithiolane]-1(3bH)-yl)acetic acid **(6):** To a solution of 2-(5-oxo-3-(trifluoromethyl)-3b,4,4a,5-tetrahydro-1H-cyclopropa[3,4]cyclopenta[1,2-c]pyrazol-1-yl)acetic acid **(5)** (3.0 g, 11.5 mmol) in DCM (dichloromethane) (25 mL) was added 1,2-ethanedithiol (1.07 mL, 12.68 mmol) followed by boron trifluoride-acetic acid complex (4.0 mL, 28.8 mmol). The reaction mixture was stirred at room temperature overnight. To the reaction mixture was added water (60 mL) and 2-MeTHF (60 mL). The organic layer was isolated, dried over sodium sulfate, filtered, and concentrated. The crude material was dissolved in ethyl acetate (2 mL) and the solution diluted with hexanes (12 mL) with vigorous stirring to provide a solid. The solid was isolated by filtration and dried to provide the title compound. MS *(m*/*z)* 337.12 [M+H]⁺.

Synthesis of 2-(5,5-difluoro-3-(trifluoromethyl)-3b,4,4a,5-tetrahydro-1H-cyclopropa[3,4]cyclopenta[1,2-c]pyrazol-1-yl)acetic acid (**7**): To a suspension of 1,3-dibromo-5,5-dimethylhydantoin (12.75 g, 44.6 mmol) in DCM (35 mL) was added pyridine hydrofluoride (5.0 mL) at 0 °C. The suspension was stirred at 0 °C for 10 minutes. To the suspension was added a solution of 2-(3-(trifluoromethyl)-4,4a-dihydrospiro[cyclopropa[3,4]cyclopenta[1,2-c]pyrazole-5,2'-[1,3]dithiolane]-1(3bH)-yl)acetic acid **(6)** (5.00 g, 14.9 mmol) dropwise. After addition was complete, the reaction mixture was stirred at 0 °C for an additional 15 minutes. The reaction mixture was poured into a saturated aqueous sodium bicarbonate solution (300 mL) with vigorous stirring. The organic layer was removed and the aqueous layer was acidified to pH ~1 with concentrated HCl. The aqueous phase was extracted with three portions of MTBE (methyl *tert*-butyl ether). The combined organic layers were dried over sodium sulfate, filtered, and concentrated. The resulting solid was taken in MTBE (16 mL) and filtered to remove any resulting solid. The solution was then extracted with 2N NaOH (16 mL). The aqueous layer was diluted with water (16 mL) with vigorous stirring and continued stirring at room temperature for 15 minutes. The resulting solid was removed by filtration. The aqueous layer was acidified by slow, dropwise addition of concentrated HCl to pH ~1 with vigorous stirring to provide a solid precipitate. The solid was isolated by filtration to provide the title compound. MS (*m*/*z*) 281.12 [M+H]⁺.

Synthesis of 2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetic acid **(8a)** and 2-((3bR,4aS)-5,5-difluoro-3-(trifluoromethyl)-3b,4,4a,5-tetrahydro-1H-cyclopropa[3,4]cyclopenta[1,2-c]pyrazol-1-yl)acetic acid **(8b):** 2-(5,5-Difluoro-3-(trifluoromethyl)-3b,4,4a,5-tetrahydro-1H-cyclopropa[3,4]cyclopenta[1,2-c]pyrazol-1-yl)acetic acid was separated to its constituent enantiomers, the title compounds, by chiral SFC under the following conditions: Instrument: Thar 350 preparative SFC; Column: ChiralPak IC-10 u, 300×50mmI.D; Mobile phase: 35% Isopropanol (0.1% NH₃·H₂O) and CO₂; Flow rate: 200 mL/min; Column temperature: 38 °C; UV detection: 220 nm; Sample preparation: Compound was dissolved in isopropanol to ~ 45 mg/mL; Injection: 6.5 mL per injection. Analytical SFC [mobile phase: A for CO₂ and B for Isopropanol (0.05% DEA); Gradient: B 20%; A; Flow rate: 2.35 mL/min; Column: Chiralpak IC-3, 150×4.6 mm, 3um; Wavelength: 254 nm] **(8a):** t= 3.39 min, **(8b):** t= 2.17 min.

Compound (8a): ¹H NMR (400 MHz, chloroform-d) δ 4.93 (s, 2H), 2.52 - 2.43 (m, 2H), 1.44 - 1.38 (m, 1H), 1.15 (m, 1H).

### B. Preparation of compound (12)

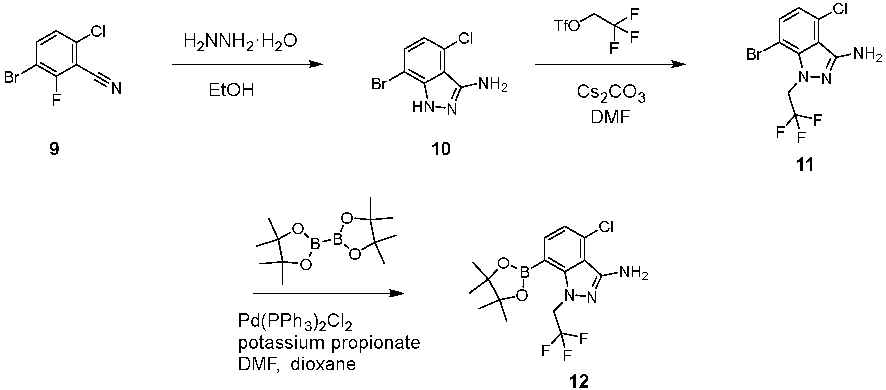

Synthesis of 7-bromo-4-chloro-1H-indazol-3-amine **(10):** To 3-bromo-6-chloro-2-fluorobenzonitrile **(9)** (13.9 g, 59.3 mmol) in EtOH (ethanol) (60 mL) was added hydrazine monohydrate (5.77 mL). The reaction mixture was heated to 80 °C for 3 h. After cooling to ambient temperature, EtOH (20 mL) was added to allow for stirring. The solids were isolated by filtration, washed with cold EtOH, and dried to provide the title compound. MS *(m*/*z)* 247.9 [M+H]⁺.

Synthesis of 7-bromo-4-chloro-1-(2,2,2-trifluoroethyl)-1H-indazol-3-amine **(11):** A reactor was charged with 7-bromo-4-chloro-1H-indazol-3-amine **(10)** (397.2 g, 1.6 mol) and Cs₂CO₃ (1052 g, 3.2 mol) then diluted with DMF (dimethylformamide) (4000 mL). To this was slowly added 2,2,2-trifluoroethyl trifluoromethanesulfonate (463.2 g, 1.9 mol) via addition funnel. Upon completion of the addition, the reaction mixture was allowed to stir for 1 hour, at which time, H₂O (16 L) was added slowly. Upon completion of the addition, the mixture was allowed to stir for 12 hours at 15 °C. The slurry was filtered and the collected solids were suspended in DMF (800 mL). To this was added H₂O (4800 mL) and the resulting solids were collected by filtration and dried to provide the title compound. MS *(m*/*z)* 330.1 [M+H]⁺.

Synthesis of 4-chloro-7-(4,4,5,5-tetramethyl-1,3,2-dioxaborolan-2-yl)-1-(2,2,2-trifluoroethyl)-1H-indazol-3-amine **(12):** A reaction vessel was charged with 7-bromo-4-chloro-1-(2,2,2-trifluoroethyl)-1H-indazol-3-amine **(11)** (15.00 g, 45.66 mmol), bis(pinacolato)diboron (17.39 g, 68.49 mmol), potassium propionate (15.36 g, 136.98 mmol), dioxane (90 mL), and DMF (dimethylformamide) (30 mL). Bis(triphenylphosphine)palladium(II) dichloride (0.64 g, 0.91 mmol) was added and the reaction solution degassed by bubbling argon for 2 min. The reaction mixture was heated to 105 °C for 4 hrs. After cooling to ambient temperature, the reaction mixture was filtered through a pad of Celite and silica gel while washing with EtOAc. The filtrate was washed with a 5% LiCl solution and brine. The organic layers were separated, dried, and concentrated under reduced pressure. The residue was treated with IPAc/heptane (1/10) at 60 °C then cooled to ambient temperature and stirred for 15 h. The solids were collected by filtration and dried to afford the title compound. MS *(m*/*z)* 376.7 [M+H]^{+ 1}H NMR (400 MHz, DMSO-d₆) δ 7.69 (d, 1H), 7.06 (d, 1H), 5.55 (s, 2H), 5.45 (q, 2H), 1.32 (s, 12H).

### C. Preparation of compound (14)

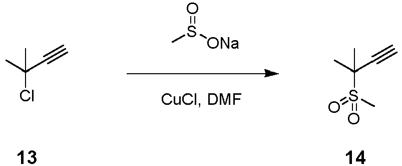

Synthesis of 3-methyl-3-(methylsulfonyl)but-1-yne **(14):** To a stirred suspension of sodium methanesulfinate (18.47 g, 175.5 mmol) and copper(I) chloride (1.45 g, 14.6 mmol) in DMF (dimethylformamide) (50 mL) was added 3-chloro-3-methylbut-1-yne **(13)** (15.00 g, 146.3 mmol, 16.4 mL) dropwise. The resulting reaction mixture was heated to 40 °C and stirred for 16 h. The reaction mixture was cooled to room temperature and diluted with EtOAc. The solution was washed with water and brine. The organic layer was collected and dried over sodium sulfate, then filtered. The solution was concentrated under vacuum and purified by silica gel chromatography to provide the title compound. Mp: 114.8-115.5 °C. ¹H NMR (400 MHz, chloroform-d) δ 3.04 (s, 3H), 2.58 (s, 1H), 1.67 (s, 6H).

### D. Preparation of compound (19)

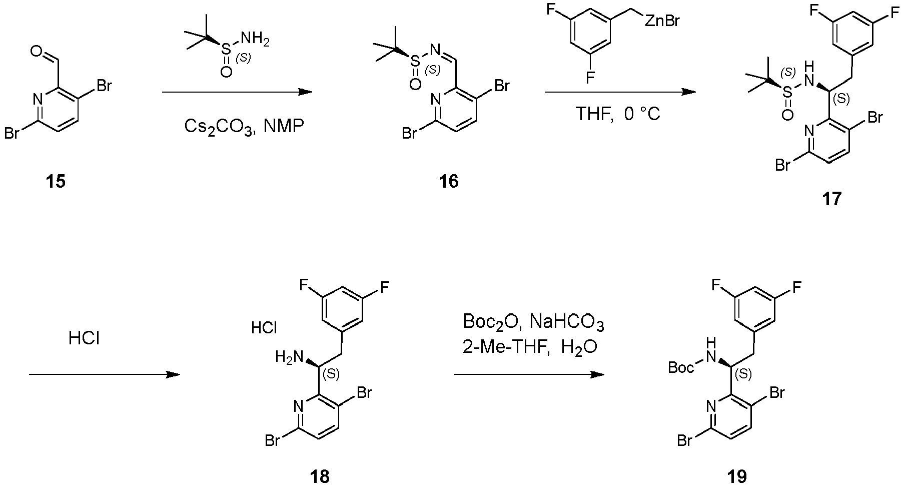

Synthesis of (S)-N-((3,6-dibromopyridin-2-yl)methylene)-2-methylpropane-2-sulfinamide **(16):** 3,6-Dibromopicolinaldehyde **(15)** (76.0 g, 0.287 mol) and (S)-2-methylpropane-2-sulfinamide (36.51 g, 0.301 mol) were combined in NMP (N-methyl-2-pyrrolidone) (200 mL). To the reaction mixture was added Cs₂CO₃ (41.94 g, 0.316 mol) as a solid in one portion. The reaction mixture was stirred for 2h then cooled to 5 °C. Water (1.3 L) was added to the reaction mixture. The resulting suspension was stirred for 1h, the solids were isolated by filtration, then washed with water (5 x 100 mL) and dried to provide the title compound. MS *(m*/*z)* 368.9 [M+H]⁺.

Synthesis of (S)-N-((S)-1-(3,6-dibromopyridin-2-yl)-2-(3,5-difluorophenyl)ethyl)-2-methylpropane-2-sulfinamide **(17):** A reaction vessel was charged with (S)-N-((3,6-dibromopyridin-2-yl)methylene)-2-methylpropane-2-sulfinamide **(16)** (65.5 g, 177.95 mmol) followed by DMF (dimethylformamide) (260 mL). The mixture was stirred for 5 min until homogeneous and the solution was cooled to 8 °C. To the reaction mixture was added (3,5-difluorobenzyl)zinc bromide (0.5 M in THF (tetrahydrofuran), 516.04 mL) dropwise over 90 mins. The mixture was stirred for an additional 2.5h. To the reaction mixture, 5% AcOH (acetic acid) in water (640 mL) was added over 10 mins followed by CPME (cyclopentyl methyl ether) (320 mL) in one portion. The mixture was stirred for 5 mins, warmed to room temperature, and the layers were separated. The organic layer was washed with 5% AcOH (320 mL) then treated with 0.5 M NaOH (330 mL) and washed with brine. The organic layer was collected, dried with Na₂SO₄, and filtered. To the crude mixture was added MeOH (methanol) (33 mL). To the stirring mixture was added dropwise 3M HCl in CPME (128 mL) over 15 mins. After stirring for 1h, the precipitate was removed by filtration. The filtrate was diluted with hexane (300 mL) and the product was extracted with water (450 mL). The aqueous layer was basified with 8 M NaOH and extracted with CPME (375 mL). The organic layer was washed with brine, dried over Na₂SO₄, and filtered to provide the title compound in solution which was used directly in the next reaction. MS *(m*/*z)* 497.0 [M+H]⁺.

Synthesis of (S)-1-(3,6-dibromopyridin-2-yl)-2-(3,5-difluorophenyl)ethan-1-amine **(18):** The resulting solution of (S)-N-((S)-1-(3,6-dibromopyridin-2-yl)-2-(3,5-difluorophenyl)ethyl)-2-methylpropane-2-sulfinamide **(17)** was diluted with CPME to a volume of 700 mL to which acetonitrile (350 mL) was added. To the stirring mixture, concentrated HCl (37%, 16.4 mL) was added dropwise over 10 mins at room temperature. The thick slurry was vigorously stirred for 4h. The solids were filtered and washed with 2:1 CPME:ACN to provide the title compound. MS *(m*/*z)* 393.3 [M+H]⁺.

Synthesis of tert-butyl (S)-(1-(3,6-dibromopyridin-2-yl)-2-(3,5-difluorophenyl)ethyl)carbamate **(19):** A reaction vessel was charged with 2-MeTHF (190 mL), water (190 mL), and (S)-1-(3,6-dibromopyridin-2-yl)-2-(3,5-difluorophenyl)ethan-1-amine **(18)** (46.9 g, 0.11 mol) followed by portionwise addition of NaHCO₃ (30.34 g, 0.36 mol). The reaction mixture was cooled to 5 °C and di-tert-butyl dicarbonate (27.47 g, 0.13 mol) was added. The reaction mixture was stirred at 0 °C for 2h and ambient temperature for 2h. The reaction mixture was diluted with water and extracted with MTBE (methyl *tert*-butyl ether). The organic layers were washed with brine, dried, and concentrated. Crude compound was purified by column chromatography on silica to provide the title compound. MS *(m*/*z)* 492.8 [M+H]⁺. ¹H NMR (400 MHz, methanol-*d*₄) δ 7.85 (d, 1H), 7.42 (d, 1H), 6.90 - 6.72 (m, 3H), 5.33 (dd, 1H), 3.10 (dd, 1H), 2.92 (dd, 1H), 1.36 (s, 9H).

### E. Preparation of the compound of Formula (Ia)

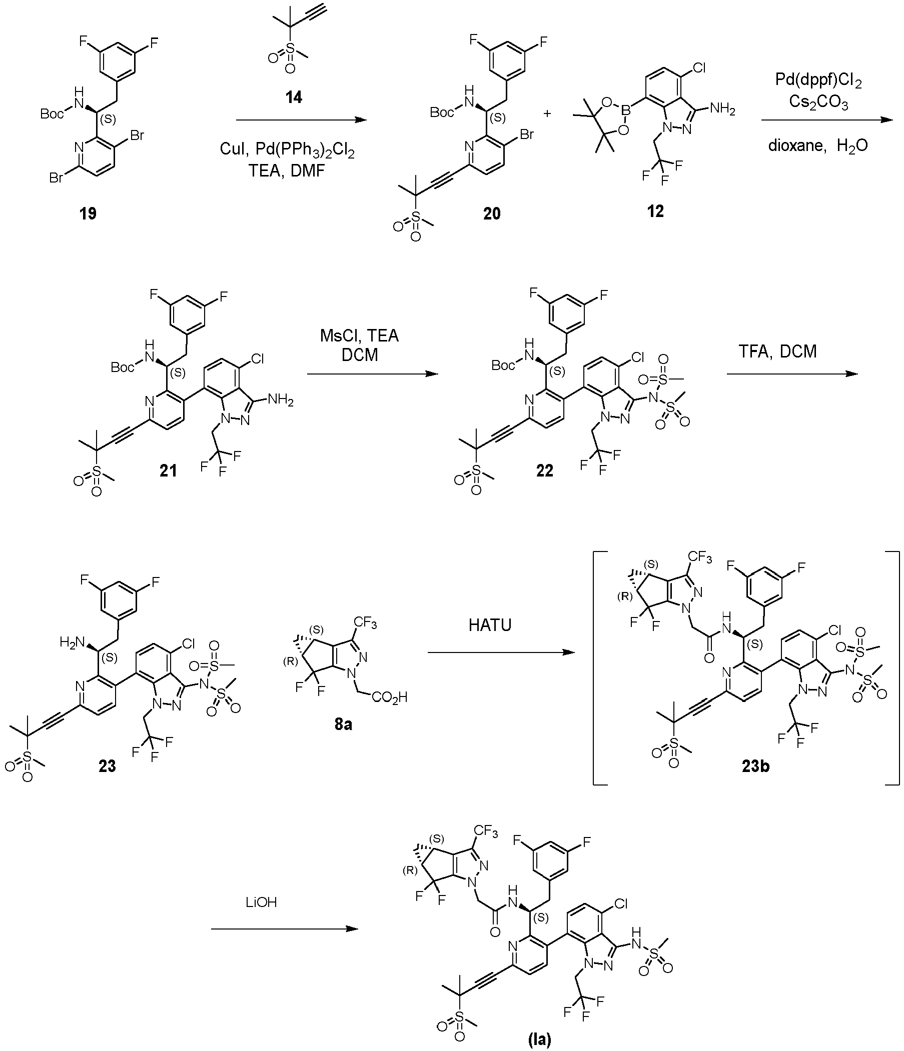

Synthesis of tert-butyl (S)-(1-(3-bromo-6-(3-methyl-3-(methylsulfonyl)but-1-yn-1-yl)pyridin-2-yl)-2-(3,5-difluorophenyl)ethyl)carbamate **(20):** A reactor was charged with tert-butyl (S)-(1-(3,6-dibromopyridin-2-yl)-2-(3,5-difluorophenyl)ethyl)carbamate (19) (50.00 g, 101.8 mmol), 3-methyl-3-methylsulfonyl-but-1-yne (14) (17.86 g, 122.2 mmol), DMF (dimethylformamide) (90 mL), and Et₃N (trimethylamine) (42.5 mL, 305.4 mmol). The reaction mixture was heated to 50 °C. Bis(triphenylphosphine)palladium(II) dichloride (2.14 g, 3.1 mmol) and copper(I) iodide (0.58 g, 3.1 mmol) were added. After 30 min, the reaction mixture was diluted with MeCN (acetonitrile) (200 mL) and then 7% aq. NH₄Cl (200 mL) was added dropwise. A slurry was formed and adjusted to ambient temperature. After 3h, the solids were collected by filtration. The cake was washed with MeCN/water twice (1:1, 75 mL) and MTBE (methyl *tert*-butyl ether) (75 mL). The solid was dried to provide the title compound. MS *(m*/*z)* 556 [M+H]⁺. ¹H NMR (400 MHz, chloroform-d) δ 7.84 (d, *J =* 8.2 Hz, 1H), 7.29 - 7.15 (m, 1H), 6.70 - 6.55 (m, 2H), 5.79 (d, *J =* 9.0 Hz, 1H), 5.57 - 5.45 (m, 1H), 3.21 - 3.05 (m, 4H), 2.99 - 2.88 (m, 1H), 1.80 (s, 6H), 1.40* (s, 7H), 1.30* (s, 2H). *denotes presence of atropisomers in 4.6:1 ratio.

Synthesis of tert-butyl (S)-(1-(3-(3-amino-4-chloro-1-(2,2,2-trifluoroethyl)-1H-indazol-7-yl)-6-(3-methyl-3-(methylsulfonyl)but-1-yn-1-yl)pyridin-2-yl)-2-(3,5-difluorophenyl)ethyl)carbamate **(21):** tert-Butyl (S)-(1-(3-bromo-6-(3-methyl-3-(methylsulfonyl)but-1-yn-1-yl)pyridin-2-yl)-2-(3,5-difluorophenyl)ethyl)carbamate **(20)** (1000.0 mg, 1.79 mmol), 4-chloro-7-(4,4,5,5-tetramethyl-1,3,2-dioxaborolan-2-yl)-1-(2,2,2-trifluoroethyl)-1H-indazol-3-amine **(12)** (808.5 mg, 2.15 mmol), [1,1'-bis(diphenylphosphino)ferrocene]dichloropalladium (II) (65.6 mg, 0.09 mmol), and cesium carbonate (876.7 mg, 2.69 mmol) were charged in a round bottom flask and placed under argon. Dioxane (10 mL) and water (2 mL) were added, and the suspension was degassed by bubbling argon for 60 seconds. After degassing, the reaction flask was fitted with a reflux condenser and heated to 80 °C overnight. The reaction mixture was cooled to room temperature, and the aqueous layer was removed. The organic layer was concentrated under vacuum, and the resulting residue was purified by silica gel column chromatography to provide the title compound. MS *(m*/*z)* 726.1 [M+H]⁺. ¹H NMR (400 MHz, chloroform-*d*) δ 7.69 - 7.55 (m), 7.55 - 7.42 (m), 7.16 - 7.06 (m), 7.07 - 6.96 (m), 6.89 (d), 6.60 (tt), 6.44 (dd), 6.20 (d), 6.16 (d), 6.08 (s), 5.69 - 5.53 (m), 5.29 (s), 5.26 (d), 4.95 - 4.85 (m), 4.64 (q), 4.59 - 4.46 (m), 4.36 - 4.19 (m), 3.94 - 3.76 (m), 3.64 - 3.54 (m), 3.18 (s), 3.17 (s), 3.01 - 2.84 (m), 2.78 - 2.68 (m), 1.86 - 1.82 (m), 1.38 (s), 1.34 (s), 1.26 (s), 1.23 (s), 1.15 (s).

Synthesis of tert-butyl (S)-(1-(3-(4-chloro-3-(N-(methylsulfonyl)methylsulfonamido)-1-(2,2,2-trifluoroethyl)-1H-indazol-7-yl)-6-(3-methyl-3-(methylsulfonyl)but-1-yn-1-yl)pyridin-2-yl)-2-(3,5-difluorophenyl)ethyl)carbamate **(22):** tert-Butyl (S)-(1-(3-(3-amino-4-chloro-1-(2,2,2-trifluoroethyl)-1H-indazol-7-yl)-6-(3-methyl-3-(methylsulfonyl)but-1-yn-1-yl)pyridin-2-yl)-2-(3,5-difluorophenyl)ethyl)carbamate **(21)** (37.89 g, 52.18 mmol) was dissolved in methylene chloride (380 mL) with stirring at ambient temperature. Triethylamine (21.82 mL, 156.54 mmol) was added, followed by slow addition of methanesulfonyl chloride (8.08 mL, 104.36 mmol). When the reaction was complete, water (200 mL) was added and stirred for 0.5 hours. The organic layer was separated and the aqueous layer was extracted with methylene chloride once. The combined organic layers were washed with water and brine, dried over MgSO₄, filtered, and concentrated to a small volume. Hexanes was added. The liquid suspension was decanted. The remaining solid was dried under reduced pressure to afford the title compound. MS (*m*/*z*): 882.69 [M+H]⁺. ¹H NMR (400 MHz, methanol-*d*₄) δ 7.87 (d), 7.83 (d), 7.76 (s), 7.74 (s), 7.69 (s), 7.67 (s), 7.65 (s), 7.52 - 7.47 (m), 7.46 (s), 7.37 (d), 7.33 (d), 7.11 - 7.03 (m), 4.79 - 4.55 (m), 4.51 (t), 4.36 (dt), 4.20 - 4.05 (m), 3.64 (s), 3.62 (s), 3.60 (s), 3.59 (s), 3.23 (s), 3.04 (d), 3.01 (d), 2.95 - 2.83 (m), 1.81 (s), 1.34 (s), 1.29 (s), 0.98 (s).

Synthesis of (S)-N-(7-(2-(1-amino-2-(3,5-difluorophenyl)ethyl)-6-(3-methyl-3-(methylsulfonyl)but-1-yn-1-yl)pyridin-3 -yl)-4-chloro-1-(2,2,2-trifluoroethyl)-1H-indazol-3-yl)-N-(methylsulfonyl)methanesulfonamide **(23):** To tert-butyl (S)-(1-(3-(4-chloro-3-(N-(methylsulfonyl)methylsulfonamido)-1-(2,2,2-trifluoroethyl)-1H-indazol-7-yl)-6-(3-methyl-3-(methylsulfonyl)but-1-yn-1-yl)pyridin-2-yl)-2-(3,5-difluorophenyl)ethyl)carbamate **(22)** (39 g, 44 mmol) dissolved in methylene chloride (120 mL) was added trifluoroacetic acid (80 mL). The reaction mixture was stirred at ambient temperature for 50 minutes. The reaction mixture was diluted with methylene chloride and slowly poured into ice cold saturated aqueous NaHCO₃. The organic layer was separated, washed with water and brine, dried over MgSO₄, filtered, and concentrated to dryness to afford the title compound. MS (*m*/*z*): 782.84 [M+H]⁺. ¹H NMR (400 MHz, chloroform-d) δ 7.61 (d), 7.54 - 7.44 (m), 7.40 (d), 7.33 (d), 7.20 (d), 6.66 - 6.57 (m), 6.44 (d), 6.33 (d), 6.17 (d), 4.64 (s), 3.68 (s), 3.64 (s), 3.61 (s), 3.55 (s), 3.19 (s), 3.05 (dd), 2.85 - 2.72 (m), 1.86 (s), 1.62 (s).

Synthesis of N-((S)-1-(3-(4-chloro-3-(methylsulfonamido)-1-(2,2,2-trifluoroethyl)-1H-indazol-7-yl)-6-(3-methyl-3-(methylsulfonyl)but-1-yn-1-yl)pyridin-2-yl)-2-(3,5-difluorophenyl)ethyl)-2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamide (**Ia**): (S)-N-(7-(2-(1-Amino-2-(3,5-difluorophenyl)ethyl)-6-(3-methyl-3-(methylsulfonyl)but-1-yn-1-yl)pyridin-3-yl)-4-chloro-1-(2,2,2-trifluoroethyl)-1H-indazol-3-yl)-N-(methylsulfonyl)methanesulfonamide **(23)** (1757 mg, 2.25 mmol), 2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetic acid **(8a)** (666 mg, 2.36 mmol), and HATU (1-[bis(dimethylamino)methylene]-1H-1,2,3-triazolo[4,5-b]pyridinium 3-oxid hexafluorophosphate) (854 mg, 2.25 mmol) were charged in a round bottom flask and dissolved in DMF (dimethylformamide) (10.0 mL). To the solution was added N,N-diisopropylethylamine (0.80 mL, 4.49 mmol) at a rapid dropwise rate. After addition was complete, the reaction mixture was stirred at room temperature for 15 minutes to provide the intermediate **(23b)** which was not isolated (MS *(m*/*z)* 1046.65 [M+H]⁺). To the solution was added a 2 N aq. sodium hydroxide solution (5.0 mL). The mixture was stirred at room temperature for 30 minutes. The reaction mixture was partitioned between water and ethyl acetate. The organic layer was collected and washed with two portions of 5% lithium chloride solution followed by brine. The organic layer was isolated, dried over sodium sulfate, filtered, and concentrated under vacuum. The resulting residue was purified by silica gel column chromatography to yield the title compound **(Ia)** as an amorphous solid. MS (*m*/*z*) 968.24 [M+H]⁺. ¹H NMR (400 MHz, methanol-*d*₄) δ 7.87 - 7.57 (m), 7.33 - 7.09 (m), 6.80 - 6.70 (m), 6.54 (d), 6.47 (d), 6.37 - 6.19 (m), 5.02-4.94(m), 4.90 - 4.70 (m), 4.70 - 4.51 (m), 3.94 (dq), 3.32-3.28 (m), 3.23 (d), 3.07 (dd, *J* = 13.1, 7.6 Hz), 2.93 (dd), 2.68 - 2.35 (m), 1.81 (s), 1.41 (q), 1.12 - 1.00 (m). ¹⁹F NMR (377 MHz, methanol-*d*₄) δ -63.65 , -71.78 (t), -72.35 (t), -82.75 (dd), -105.70 (ddd), -111.73 - - 113.10 (m).

Compound **(23b)** was isolated and characterized. ¹H NMR (400 MHz, DMSO-*d*₆) δ 9.20 (d), 8.99 (d), 7.96 (d), 7.83 (d), 7.80 (d), 7.76 (d), 7.45 (d), 7.41 (d), 7.31 (d), 7.02 (tt), 6.92 (m), 6.91 (d), 6.48 (m), 4.92 (m) 4.88 (d), 4.79 (d), 4.73 (d), 4.71 (m), 4.69 (m), 4.62 (m), 4.60 (m), 4.38 (dq), 4.12 (dq), 3.68 (s), 3.66 (s), 3.63 (s), 3.58 (s), 3.26 (s), 3.12 (dd), 3.05 (dd), 2.97 (dd), 2.78 (dd), 2.59 (m), 2.53 (m), 1.75 (s), 1.39 (m), 0.98 (m).

### Example 2: Formulations

The invention provides a pharmaceutical composition comprising a sodium salt of the compound of Formula **(Ia)** wherein the pharmaceutical composition is in the form of a solution and the solution comprises 35 w/w% to 75 w/w% PEG 300, 10 w/w% to 40 w/w% water and 5 w/w% to 35 w/w% of the sodium salt of the compound of Formula **(Ia).** Certain examples of other compositions are described herein for illustrative or comparative purposes only and do not fall within the scope of the claimed invention.

Formulations containing the compound of Formula **(Ia)** were prepared as aqueous solutions or oral formulations and administered subcutaneously to rats, dogs, cynomolgus monkey, and/or rhesus monkey.

### A. 10% ethanol/13% water/77% PEG 200 solution (400 mg/mL Formula (Ia))

A solution of the compound of Formula **(Ia)** in 10% ethanol, 13% water, and 77% PEG 200 (400 mg/mL) was prepared. The solution was administered subcutaneously to dogs at a dose of 12 mg/kg and rats at a dose of 50 mg/kg and the pharmacokinetic (PK) profile was determined. FIG. 3 shows a plot of the plasma concentration of the compound of Formula **(Ia)** as a function of time. In both species, plasma concentrations of the compound of Formula **(Ia)** were maintained above the target trough concentration of 20 nM (corresponding to an IQ of 5) for at least 84 days (12 weeks). The results are summarized in Table 1 below.

**Table 1. PK parameters of the compound of Formula (Ia) following SC administration in solution**

| **Species** | **Dose (mg/kg)** | **Dose Concentration (mg/mL)** | **Dose Volume (mL/kg)** | **AUC_{0- 84d} (µM•h)** | **AUC_{0- 140d} (µM•h)** | **Cₘₐₓ (µM)** | **Tₘₐₓ (h)** | **C84d (µM)** |
|---|---|---|---|---|---|---|---|---|
| Wistar Han Rat | 50 | 400 | 0.125 | 404 ± 139 | 569 ± 157 | 0.283 ± 0.119 | 840 ± 291 | 0.162 ± 0.030 |
| Beagle Dog | 12 | 400 | 0.030 | 89.6 ± 10.1 | 111 ± 14 | 0.107 ± 0.013 | 336 ± 0 | 0.0234 ± 0.0057 |

| | | | | | | | | |
|---|---|---|---|---|---|---|---|---|
| Formula **(Ia):** 1 nM = 0.968 ng/mL; d = day Values are the mean ± SD from 3 animals | | | | | | | | |

### B. 15% water/85% PEG 300 solution (300 mg/mL Formula (Ia))

A solution of the free acid of the compound of Formula **(Ia)** in 15% water and 85% PEG 300 (300 mg/mL) was prepared. The solution was administered subcutaneously to male Wistar Han rats at a dose level of 50 mg/kg and dose volume of 0.167 mL/kg and the pharmacokinetic (PK) profile was determined. FIG. 4 shows a plot of the plasma concentration of the compound of Formula **(Ia)** as a function of time. The results are summarized in Tables 2 and 3 below.

**Table 2. PK parameters of the compound of Formula (Ia) following single SC dose in rats (mean ± SD, n=3)**

| | |
|---|---|
| **Dosing Concentration (mg/mL)** | 300 |
| **Dosing Volume (mL/kg)** | 0.167 |
| **Dose (mg/kg)** | 50 |
| **Formulation** | 85:15 w/w PEG 300:water |
| **AUC₀₋₁₆₈ₕ (µM•h)** | 13.3 ± 0.3 |
| **AUC₀₋₃₃₆ₕ (µM•h)** | 47.6 ± 1.2 |
| **AUC₀₋₆₇₂ₕ (µM•h)** | 146 ± 6 |
| **AUC₀₋₁₃₄₄ₕ (µM•h)** | 338 ± 15 |
| **AUC₀₋₁₆₈₀ₕ (µM•h)** | 412 ± 21 |
| **ADC_{inf} (µM•h)** | NA |
| **t_{1/2} (days)** | NA |
| **Cₘₐₓ (µM)** | NA |
| **Tₘₐₓ (h)** | NA |

**Table 3. Plasma concentration-time data of the compound of Formula (Ia) in rats after subcutaneous administration (mean ± SD, n=3)**

| **Time (h)** | **Plasma concentration (nM)** | | | **Mean** | **SD** |
|---|---|---|---|---|---|
| | **#1** | **#2** | **#3** | | |
| 0 | BLQ | BLQ | BLQ | NC | NC |
| 1 | 8.9 | 11.4 | 5.6 | 8.6 | 2.9 |
| 3 | 27.0 | 17.3 | 20.1 | 21.5 | 5.0 |
| 8 | 37.0 | 32.8 | 26.8 | 32.2 | 5.1 |
| 24 | 52.6 | 49.2 | 42.8 | 48.2 | 5.0 |
| 48 | 69.8 | 71.1 | 70.8 | 70.6 | 0.7 |
| 72 | 65.6 | 66.9 | 70.1 | 67.5 | 2.3 |
| 96 | 65.5 | 76.8 | 85.2 | 75.8 | 9.9 |
| 168 | 143 | 132 | 137 | 137 | 5.5 |
| 336 | 258 | 273 | 284 | 272 | 13.1 |
| 504 | 327 | 267 | 281 | 292 | 31.4 |
| 672 | 333 | 299 | 304 | 312 | 18.4 |
| 840 | 322 | 316 | 302 | 313 | 10.3 |
| 1008 | 300 | 258 | 289 | 282 | 21.8 |
| 1344 | 251 | 229 | 278 | 253 | 24.5 |
| 1680 | 202 | 166 | 200 | 189 | 20.2 |

### C. 15% water/85% PEG 300 solution (400 mg/mL Formula (Ia))

A solution of the compound of Formula **(Ia)** in 15% water and 85% PEG 300 (400 mg/mL) was prepared. The solution was administered subcutaneously to male beagle dogs at a dose level of 12 mg/kg and dose volume of 0.04 mL/kg and the pharmacokinetic (PK) profile was determined. FIG. 5 shows a plot of the plasma concentration of the compound of Formula **(Ia)** as a function of time. The results are summarized in Tables 4 and 5 below.

**Table 4. PK parameters of the compound of Formula (Ia) following single SC dose in male beagle dogs (mean ± SD, n=3)**

| | |
|---|---|
| **Dosing Concentration (mg/mL)** | 400 |
| **Dosing Volume (mL/kg)** | 0.03 |
| **Dose (mg/kg)** | 12 |
| **Formulation** | 85% PEG300 and 15% water |
| **AUC₀₋₂₄ₕ (µM•h)** | 0.304 ± 0.082 |
| **AUC₀₋₄₈ₕ (µM•h)** | 1.08 ± 0.44 |
| **AUC₀₋₇₂ₕ (µM•h)** | 2.04 ± 0.98 |
| **AUC₀₋₁₆₈ₕ (µM•h)** | 5.82 ± 3.04 |
| **AUC₀₋₆₇₂ₕ (µM•h)** | 51.2 ± 21.0 |
| **AUC₀₋₁₃₄₄ₕ (µM•h)** | 102 ± 30 |
| **AUC₀₋₁₆₈₀ₕ (µM•h)** | 116 ± 35 |
| **t_{1/2} (days)** | NA |
| **Cₘₐₓ (µM)** | NA |
| **Tₘₐₓ (h)** | NA |

**Table 5. Plasma concentration-time data of the compound of Formula (Ia) in beagle dogs after subcutaneous administration (mean ± SD, n=3)**

| **Time (h)** | **Plasma concentration (nM)** | | | **Mean** | **SD** |
|---|---|---|---|---|---|
| | **#1** | **#2** | **#3** | | |
| 0 | BLQ | BLQ | BLQ | NC | NC |
| 1.0 | BLQ | 1.2 | 1.2 | 1.2 | NC |
| 3.0 | 1.2 | 2.6 | 2.3 | 2.0 | 0.8 |
| 8.0 | 10.6 | 15.5 | 9.2 | 11.8 | 3.3 |
| 24.0 | 19.8 | 27.8 | 16.8 | 21.5 | 5.7 |
| 48.0 | 54.6 | 60.6 | 13.2 | 42.8 | 25.8 |
| 72.0 | 62.8 | 37.8 | 13.2 | 37.9 | 24.8 |
| 96.0 | 58.9 | 28.8 | 10.4 | 32.7 | 24.5 |
| 168 | 71.9 | 43.1 | 30.8 | 48.6 | 21.1 |
| 336 | 135 | 64.7 | 53.0 | 84.2 | 44.4 |
| 504 | 172 | 114 | 82.9 | 123 | 45.2 |
| 672 | 93.1 | 81.5 | 56.9 | 77.2 | 18.5 |
| 840 | 150 | 110 | 107 | 122.3 | 24.0 |
| 1008 | 80.2 | 70.7 | 54.1 | 68.3 | 13.2 |
| 1344 | 42.7 | 41.2 | 30.2 | 38.0 | 6.8 |
| 1680 | 69.5 | 42.2 | 28.5 | 46.7 | 20.9 |

### D. 22.6% water/50% PEG 300/1.1%NaOH/26.3% Formula (Ia) solution

A solution of the compound of Formula **(Ia)** sodium salt in water and 300 (300 mg/mL) was prepared that contained 22.6% water/50% PEG 300/1.1%NaOH/26.3% Formula **(Ia).** The solution was administered subcutaneously to male beagle dogs at a dose level of 12 mg/kg and dose volume of 0.04 mL/kg and the pharmacokinetic (PK) profile was determined. FIG. 6 shows a plot of the plasma concentration of the compound of Formula **(Ia)** as a function of time. The results are summarized in Tables 6 and 7 below. The results for the male beagle dogs is also shown in Table 6a.

**Table 6. PK parameters of the compound of Formula (Ia) sodium salt following single SC dose in male beagle dogs (mean ± SD, n=3)**

| | |
|---|---|
| **Dosing Concentration (mg/mL)** | 300 |
| **Dosing Volume (mL/kg)** | 0.04 |
| **Dose (mg/kg)** | 12 |
| **Formulation** | 50, 22.6, 1.1, 26.3/PEG300: Water: NaOH: GS-6207 w/w/w/w% |
| **AUC₀₋₂₄ₕ (µM•h)** | 0.85 ± 0.31 |
| **AUC₀₋₄₈ₕ (µM•h)** | 2.03 ± 0.76 |
| **AUC₀₋₇₂ₕ (µM•h)** | 3.16 ± 1.27 |
| **AUC₀₋₁₆₈ₕ (µM•h)** | 6.83 ± 3.24 |
| **t_{1/2} (days)** | NA |
| **Cₘₐₓ (µM)** | NA |
| **Tₘₐₓ (h)** | NA |

**Table 6a. PK parameters of the compound of Formula (Ia) sodium salt following single SC dose in male beagle dogs (mean ± SD, n=3)**

| | |
|---|---|
| **Dosing Concentration (mg/mL)** | 300 |
| **Dosing Volume (mL/kg)** | 0.04 |
| **Dose (mg/kg)** | 12 |
| **Formulation** | 50, 22.6, 1.1, 26.3/PEG300:Water:NaOH:compound of Formula **(Ia)** w/w/w/w% |
| **AUC₀₋₂₄ₕ (µM•h)** | 0.85 ± 0.31 |
| **AUC₀₋₄₈ₕ (µM•h)** | 2.03 ± 0.76 |
| **AUC₀₋₇₂ₕ (µM•h)** | 3.16 ± 1.27 |
| **AUC₀₋₁₆₈ₕ (µM•h)** | 6.83 ± 3.24 |
| **t_{1/2} (days)** | NA |
| **Cₘₐₓ (µM)** | NA |
| **Tₘₐₓ (h)** | NA |

**Table 7. Plasma concentration-time data of the compound of Formula (Ia) sodium salt in beagle dogs after subcutaneous administration (mean ± SD, n=3)**

| **Time (h)** | **Plasma concentration (nM)** | | | **Mean** | **SD** |
|---|---|---|---|---|---|
| | **#1** | **#2** | **#3** | | |
| 0 | BLQ | BLQ | BLQ | NC | NC |
| 1.0 | 14.0 | 7.2 | 8.6 | 10.0 | 3.6 |
| 3.0 | 26.0 | 17.0 | 16.8 | 19.9 | 5.3 |
| 8.0 | 56.3 | 25.8 | 32.1 | 38.1 | 16.1 |
| 24 | 57.1 | 25.4 | 54.5 | 45.7 | 17.6 |
| 48 | 71.1 | 29.0 | 57.8 | 52.6 | 21.5 |
| 72 | 51.5 | 16.2 | 58.0 | 41.9 | 22.5 |
| 96 | 46.4 | 14.0 | 59.2 | 39.9 | 23.3 |
| 168 | 47.7 | 13.6 | 43.1 | 34.8 | 18.5 |

### E. 5% ethanol/20% propylene glycol/45% PEG 300/30% 0.01 N HCl oral formulation

An oral formulation containing a compound of Formula **(Ia)** in 5% ethanol, 20% propylene glycol, 45% PEG 300, and 30% 0.01 N HCl was prepared. The formulation was orally administered to male rat, dog, cynomolgus monkey, and rhesus monkey according to the doses shown in Table 8 below. The plasma PK parameters are also summarized in Table 8. The compound of Formula **(Ia)** demonstrated moderate F% (bioavailability) in rats and dogs and low F% in cynomolgus monkey and rhesus monkey. The compound of Formula **(Ia)** was slowly absorbed with an average Tₘₐₓ range from 8.7 to 18.7 hours.

**Table 8. Plasma PK parameters following a single oral administration of the compound of Formula (Ia) in solution to rat, dog, cynomolgus monkey, and rhesus monkey**

| **Species** | **Dose (mg/kg)** | **AUC₀₋₂₄ₕ (nM•h)** | **AUC₀₋₇₂ₕ (nM•h)** | **AUC_{inf} (nM•h)** | **Cₘₐₓ (nM)** | **Tₘₐₓ (h)** | **F (%)** |
|---|---|---|---|---|---|---|---|
| Sprague-Dawley Rat | 5.0 | ND | 13467 ± 3927 | 25200 ± 10221 | 336 ± 116 | 10.0 ± 3.5 | 21.7 ± 8.9^{a} |
| Beagle Dog | 4.0 | ND | 11410 ± 4324 | 16533 ± 10366 | 471 ± 203 | 10.7 ± 11.5 | 22.1 ± 8.4^{a} |
| Cynomolgus Monkey | 4.0 | 298 ± 85 | ND | ND | 17.7 ± 5.6 | 8.7 ± 3.1 | 2.2 ± 0.6^{b} |
| Rhesus Monkey | 4.0 | ND | 972 ± 367 | 1133 ± 446 | 29.4 ± 7.5 | 18.7 ± 9.2 | 4.4 ± 1.7^{a} |

| | | | | | | | |
|---|---|---|---|---|---|---|---|
| AUC₀₋₂₄ₕ = area under the plasma concentration-time curve from 0 to 24 h; AUC₀₋₇₂ₕ = area under the plasma concentration-time curve from 0 to 72 h; AUC_{inf} = area under the plasma concentration-time curve extrapolated to time infinity; Cₘₐₓ = maximum plasma concentration; F = oral bioavailability; ND = not determined; Tₘₐₓ = time to reach the maximum plasma concentration ^{a} Calculated based on AUC₀₋₇₂ₕ ^{b} Calculated based on AUC₀₋₂₄ₕ Values are the mean ± standard deviation from 3 animals | | | | | | | |

### F. Liquid-filled capsules

A single dose of the compound of Formula **(Ia),** formulated as liquid-filled capsules (LFC) containing either 100% glycerol monocaprylocaprate or 20% caprylocaproyl polyoxyl-8 glycerides and 80% glycerol monocaprylocaprate was administered orally at 7.5 mg fixed dose to 6 male beagle dogs (Table 9). At 100 mg/mL of the compound of Formula **(Ia),** the AUC_{inf} increased with increasing glycerol monocaprylocaprate content. For the formulation with 100% glycerol monocaprylocaprate, the AUC_{inf} at 50 mg/mL was higher than that at 100 mg/mL.

**Table 9. Pharmacokinetics of the compound of Formula (Ia) following oral administration in liquid-filled capsules at 7.5 mg fixed dose to fasted male beagle dogs pretreated with pentagastrin^{a,b}**

| **Formulation** | **(Ia) Dose Concentration (mg/mL)** | **AUC₀₋₇₂ₕ (nM•h)** | **AUC_{inf} (nM•h)** | **Cₘₐₓ (nM)** | **Tₘₐₓ (h)** | **F%^{c}** |
|---|---|---|---|---|---|---|
| 20% caprylocaproyl polyoxyl-8 glycerides and 80% | 100 | 1181 ± 575 | 1303 ± 634 | 53.2 ± 16.7 | 7.7 ± 8.0 | 11 ± 4 |
| glycerol monocaprylocaprate | | | | | | |
| 100% glycerol monocaprylocaprate | 100 | 1667 ± 1045 | 1867 ± 1188 | 71.2 ± 25.8 | 5.0 ± 1.1 | 16 ± 9 |
| | 50 | 2178 ± 1243 | 2329 ± 1348 | 82.7 ± 32.1 | 4.0 ± 0.0 | 22 ± 10 |

| | | | | | | |
|---|---|---|---|---|---|---|
| ^{a} Animals were fasted at least 12 hours prior to dosing and fed 4 hours post dose ^{b} Pentagastrin (6 µg/kg) was administered intramuscularly ~30 minutes prior to dosing to ensure an acidic pH in the gastrointestinal tract ^{c} %F calculated based on AUC₀₋₇₂ₕ with average IV AUC₀₋₇₂ₕ = 13,467 nM•h at 1 mg/kg Values are the mean ± standard deviation from 6 animals | | | | | | |

### G. 9.8 w/w% water, 65.0 w/w% PEG 300, and 25.2 w/w% of a compound of Formula (Ia) solution (300 mg/mL of compound of Formula (Ia))

A 300 mg/ml solution of a compound of Formula **(Ia)** having 9.8 w/w% water, 65.0 w/w% PEG 300, and 25.2 w/w% of the compound of Formula **(Ia)** was prepared. The solution was administered subcutaneously to rats at a dose of 50 mg/kg and to dogs at a dose of 12 mg/kg and the pharmacokinetic (PK) profile was determined. FIG. 7A shows a plot of the plasma concentration of the compound of Formula **(Ia)** as a function of time in the rat and FIG. 7B shows a plot of the plasma concentration of the compound of Formula **(Ia)** as a function of time in the dog. In the rat, plasma concentrations of the compound of Formula **(Ia)** were maintained above the target trough concentration of 20 nM for at least 168 days (24 weeks). In the dog, plasma concentrations of the compound of Formula **(Ia)** were maintained above the target trough concentration of 20 nM for at least 84 days (12 weeks). The results are summarized in Tables A and B below.

**Table A. PK parameters of the compound of Formula (Ia) following subcutaneous (SC) administration in solution in rats**

| **Species** | **Dose (mg/kg)** | **Dose Concentration (mg/mL)** | **Dose Volume (mL/kg)** | **AUC_{0- 98d} (µM•h)** | **AUC_{0- 168d} (µM•h)** | **Cₘₐₓ (µM)** | **Tₘₐₓ (h)** | **C_{d168} (µM)** |
|---|---|---|---|---|---|---|---|---|
| Wistar Han Rat | 50 | 300 | 0.167 | 531 ± 30 | 730 ± 55 | 0.318 ± 0.146 | 728 ± 97 | 0.0784 0.0138 |

| | | | | | | | | |
|---|---|---|---|---|---|---|---|---|
| Formula **(Ia):** 1 nM = 0.968 ng/mL; d = day; h = hour Values are the mean ± SD from 3 animals | | | | | | | | |

**Table B. PK parameters of the compound of Formula (Ia) following SC administration in solution in dogs**

| **Species** | **Dose (mg/kg)** | **Dose Concentration (mg/mL)** | **Dose Volume (mL/kg)** | **AUC_{0- 98d} (µM•h)** | **AUC_{0- 126d} (µM•h)** | **Cₘₐₓ (µM)** | **Tₘₐₓ (h)** | **C_{84d} (µM)** |
|---|---|---|---|---|---|---|---|---|
| Beagle Dog | 12 | 300 | 0.04 | 130.0 ± 9.1 | 139 ± 8 | 0.133 ± 0.010 | 336 ± 0 | 0.0217 ± 0.0065 |

| | | | | | | | | |
|---|---|---|---|---|---|---|---|---|
| Formula (Ia): 1 nM = 0.968 ng/mL; d = day; h = hour Values are the mean ± SD from 3 animals | | | | | | | | |

### H. 27.92 w/w% water, 58.04 w/w% PEG 300, 13.47 w/w% of a compound of Formula (Ia), and 1.1 w/w% NaOH solution (150 mg/mL of compound of Formula (Ia))

A 150 mg/ml solution of a compound of Formula **(Ia)** having 27.92 w/w% water, 58.04 w/w% PEG 300, 13.47 w/w% of a compound of Formula **(Ia),** and 1.1 w/w% NaOH was prepared. The solution was administered subcutaneously to rats at a dose of 50 mg/kg and to dogs at a dose of 12 mg/kg and the pharmacokinetic (PK) profile was determined. FIG. 8A shows a plot of the plasma concentration of the compound of Formula **(Ia)** as a function of time in the rat and FIG. 8B shows a plot of the plasma concentration of the compound of Formula **(Ia)** as a function of time in the dog. In the rat, plasma concentrations of the compound of Formula **(Ia)** were maintained above the target trough concentration of 20 nM for at least 126 days (18 weeks). In the dog, plasma concentrations of the compound of Formula **(Ia)** were maintained above the target trough concentration of 20 nM for at least 56 days (8 weeks). The results are summarized in Tables C and D below.

**Table C. PK parameters of the compound of Formula (Ia) following SC administration in solution in rats**

| **Species** | **Dose (mg/kg)** | **Dose Concentration (mg/mL)** | **Dose Volume (mL/kg)** | **AUC_{0- 98d} (µM•h)** | **Cₘₐₓ (µM)** | **Tₘₐₓ (h)** | **C_{d98} (µM)** |
|---|---|---|---|---|---|---|---|
| Wistar Han Rat | 50 | 150 | 0.333 | 801 ± 119 | 0.809 ± 0.021 | 448 ± 97 | 0.0370 ± 0.0107 |

| | | | | | | | |
|---|---|---|---|---|---|---|---|
| Formula (Ia): 1 nM = 0.968 ng/mL; d = day; h = hour Values are the mean ± SD from 3 animals | | | | | | | |

**Table D. PK parameters of the compound of Formula (Ia) following SC administration in solution in dogs**

| **Species** | **Dose (mg/kg)** | **Dose Concentration (mg/mL)** | **Dose Volume (mL/kg)** | **AUC_{0- 28d} (µM•h)** | **AUC_{0- 126d} (µM•h)** | **Cₘₐₓ (µM)** | **Tₘₐₓ (h)** | **C_{56d} (µM)** |
|---|---|---|---|---|---|---|---|---|
| Beagle Dog | 12 | 150 | 0.08 | 137.0 ± 38 | 196 ± 31 | 0.312 ± 0.093 | 392 ± 97 | 0.0229 ± 0.0056 |

| | | | | | | | | |
|---|---|---|---|---|---|---|---|---|
| Formula **(Ia):** 1 nM = 0.968 ng/mL; d = day; h = hour Values are the mean ± SD from 3 animals | | | | | | | | |

### I. 23.2 w/w% water, 50.0 w/w% PEG 300, 25.7 w/w% of a compound of Formula (Ia), and 1.1 w/w% NaOH solution (300 mg/mL of compound of Formula (Ia))

A 300 mg/ml solution of a compound of Formula **(Ia)** having 23.2 w/w% water, 50.0 w/w% PEG 300, 25.7 w/w% of a compound of Formula **(Ia),** and 1.1 w/w% NaOH was prepared. The solution was administered subcutaneously to rats at a dose of 50 mg/kg and to dogs at a dose of 12 mg/kg and the pharmacokinetic (PK) profile was determined. FIG. 9A shows a plot of the plasma concentration of the compound of Formula **(Ia)** as a function of time in the rat and FIG. 9B shows a plot of the plasma concentration of the compound of Formula **(Ia)** as a function of time in the dog. In the rat, plasma concentrations of the compound of Formula **(Ia)** were maintained above the target trough concentration of 20 nM for at least 126 days (18 weeks). In the dog, plasma concentrations of the compound of Formula **(Ia)** were maintained above the target trough concentration of 20 nM for at least 56 days (8 weeks). The results are summarized in Tables E and F below.

**Table E. PK parameters of the compound of Formula (Ia) following SC administration in solution in rats**

| **Species** | **Dose (mg/kg)** | **Dose Concentration (mg/mL)** | **Dose Volume (mL/kg)** | **AUC_{0- 126d} (µM•h)** | **Cₘₐₓ (µM)** | **Tₘₐₓ (h)** | **C_{d168} (µM)** |
|---|---|---|---|---|---|---|---|
| Wistar Han Rat | 50 | 300 | 0.167 | 698 ± 13 | 0.355 ± 0.036 | 616 ± 96 | 0.0128 ± 0.0035 |

| | | | | | | | |
|---|---|---|---|---|---|---|---|
| Formula **(Ia):** 1 nM = 0.968 ng/mL; d = day; h = hour Values are the mean ± SD from 3 animals | | | | | | | |

**Table F. PK parameters of the compound of Formula (Ia) following SC administration in solution in dogs**

| **Species** | **Dose (mg/kg)** | **Dose Concentration (mg/mL)** | **Dose Volume (mL/kg)** | **AUC_{0- 56d} (µM•h)** | **Cₘₐₓ (µM)** | **Tₘₐₓ (h)** | **C_{56d} (µM)** |
|---|---|---|---|---|---|---|---|
| Beagle Dog | 12 | 300 | 0.04 | 166 ± 110 | 0.267 ± 0.176 | 296 ± 231 | 0.0296 ± 0.0308 |

| | | | | | | | |
|---|---|---|---|---|---|---|---|
| Formula **(Ia):** 1 nM = 0.968 ng/mL; d = day; h = hour Values are the mean ± SD from 3 animals | | | | | | | |

### J. 30.07 w/w% water, 64.40 w/w% PEG 300, 4.68 w/w% of a sodium salt of the compound of Formula (Ia), and 0.85 w/w% poloxamer 188 solution (50 mg/mL of compound of Formula (Ia))

A 50 mg/ml solution of a compound of Formula **(Ia)** having 30.07 w/w% water, 64.40 w/w% PEG 300, 4.68 w/w% of a sodium salt of the compound of Formula **(Ia),** and 0.85 w/w% poloxamer 188 was prepared. The solution was administered subcutaneously to rats at a dose of 50 mg/kg and to dogs at a dose of 6 mg/kg and the pharmacokinetic (PK) profile was determined. FIG. 10A shows a plot of the plasma concentration of the compound of Formula **(Ia)** as a function of time in the rat and FIG. 10B shows a plot of the plasma concentration of the compound of Formula **(Ia)** as a function of time in the dog. In the rat, plasma concentrations of the compound of Formula **(Ia)** were maintained above the target trough concentration of 20 nM for at least 28 days (4 weeks). In the dog, plasma concentrations of the compound of Formula **(Ia)** were maintained above the target trough concentration of 20 nM for at least 14 days (2 weeks). The results are summarized in Tables G and H below.

**Table G. PK parameters of the compound of Formula (Ia) following SC administration in solution in rats**

| **Species** | **Dose (mg/kg)** | **Dose Concentration (mg/mL)** | **Dose Volume (mL/kg)** | **AUC_{0- 7d} (µM•h)** | **AUC_{0- 28d} (µM•h)** | **Cₘₐₓ (µM)** | **Tₘₐₓ (h)** | **C_{d28} (µM)** |
|---|---|---|---|---|---|---|---|---|
| Wistar Han Rat | 50 | 50 | 1.0 | 156 ± 48 | 643 ± 75 | N/A | N/A | 0.082 ± 0.035 |

| | | | | | | | | |
|---|---|---|---|---|---|---|---|---|
| Formula **(Ia):** 1 nM = 0.968 ng/mL; d = day; h = hour Values are the mean ± SD from 3 animals | | | | | | | | |

**Table H. PK parameters of the compound of Formula (Ia) following SC administration in solution in dogs**

| **Species** | **Dose (mg/kg)** | **Dose Concentration (mg/mL)** | **Dose Volume (mL/kg)** | **AUC_{0- 24h} (µM•h)** | **AUC_{0- 14d} (µM•h)** | **Cₘₐₓ (µM)** | **Tₘₐₓ (h)** | **C_{14d} (µM)** |
|---|---|---|---|---|---|---|---|---|
| Beagle Dog | 6 | 50 | 0.12 | 14.7 ± 4.4 | 108 ± 16 | N/A | N/A | 0.0372 0.0139 |

| | | | | | | | | |
|---|---|---|---|---|---|---|---|---|
| Formula **(Ia):** 1 nM = 0.968 ng/mL; d = day; h = hour Values are the mean ± SD from 3 animals | | | | | | | | |

### K. 29.21 w/w% water, 62.55 w/w% PEG 300, 6.97 w/w% of a sodium salt of the compound of Formula (Ia), and 1.68 w/w% poloxamer 188 solution (75 mg/mL of compound of Formula (Ia))

A 75 mg/ml solution of a compound of Formula (Ia) having 29.21 w/w% water, 62.55 w/w% PEG 300, 6.97 w/w% of a sodium salt of the compound of Formula (Ia), and 1.68 w/w% poloxamer 188 was prepared. The solution was administered subcutaneously to rats at a dose of 50 mg/kg and to dogs at a dose of 6 mg/kg and the pharmacokinetic (PK) profile was determined. FIG. 11A shows a plot of the plasma concentration of the compound of Formula (Ia) as a function of time in the rat and FIG. 11B shows a plot of the plasma concentration of the compound of Formula (Ia) as a function of time in the dog. In the rat, plasma concentrations of the compound of Formula (Ia) were maintained above the target trough concentration of 20 nM for at least 42 days (6 weeks). In the dog, plasma concentrations of the compound of Formula (Ia) were maintained above the target trough concentration of 20 nM for at least 21 days (3 weeks). The results are summarized in Tables I and J below.

**Table I. PK parameters of the compound of Formula (Ia) following SC administration in solution in rats**

| **Species** | **Dose (mg/kg)** | **Dose Concentration (mg/mL)** | **Dose Volume (mL/kg)** | **AUC_{0- 7d} (µM•h)** | **AUC_{0- 28d} (µM•h)** | **Cₘₐₓ (µM)** | **Tₘₐₓ (h)** | **C_{d28} (µM)** |
|---|---|---|---|---|---|---|---|---|
| Wistar Han Rat | 50 | 75 | 0.667 | 241 ± 22 | 690 ± 32 | N/A | N/A | 0.0325 ± 0.0185 |

| | | | | | | | | |
|---|---|---|---|---|---|---|---|---|
| Formula **(Ia):** 1 nM = 0.968 ng/mL; d = day; h = hour Values are the mean ± SD from 3 animals | | | | | | | | |

**Table J. PK parameters of the compound of Formula (Ia) following SC administration in solution in dogs**

| **Species** | **Dose (mg/kg)** | **Dose Concentration (mg/mL)** | **Dose Volume (mL/kg)** | **AUC_{0- 24h} (µM•h)** | **AUC_{0- 28d} (µM•h)** | **Cₘₐₓ (µM)** | **Tₘₐₓ (h)** | **C_{21d} (µM)** |
|---|---|---|---|---|---|---|---|---|
| Beagle Dog | 6 | 75 | 0.06 | 5.88 ± 1.95 | 101 ± 11 | N/A | N/A | 0.040 ± 0.024 |

| | | | | | | | | |
|---|---|---|---|---|---|---|---|---|
| Formula **(Ia):** 1 nM = 0.968 ng/mL; d = day; h = hour Values are the mean ± SD from 3 animals | | | | | | | | |

### L. 28.36 w/w% water, 60.73 w/w% PEG 300, 9.23 w/w% of a sodium salt of a compound of Formula (Ia), and 1.68 w/w% poloxamer 188 solution (100 mg/mL of compound of Formula (Ia))

A 100 mg/ml solution of a compound of Formula **(Ia)** having 28.36 w/w% water, 60.73 w/w% PEG 300, 9.23 w/w% of a sodium salt of a compound of Formula **(Ia),** and 1.68 w/w% poloxamer 188 was prepared. The solution was administered subcutaneously to rats at a dose of 50 mg/kg and the pharmacokinetic (PK) profile was determined. FIG. 12A shows a plot of the plasma concentration of the compound of Formula **(Ia)** as a function of time. Plasma concentrations of the compound of Formula **(Ia)** were maintained above the target trough concentration of 20 nM for at least 28 days (4 weeks). The results are summarized in Table K below.

**Table K. PK parameters of the compound of Formula (Ia) following SC administration in solution in rats**

| **Species** | **Dose (mg/kg)** | **Dose Concentration (mg/mL)** | **Dose Volume (mL/kg)** | **AUC_{0- 7d} (µM•h)** | **AUC_{0- 28d} (µM•h)** | **Cₘₐₓ (µM)** | **Tₘₐₓ (h)** | **C_{d28} (µM)** |
|---|---|---|---|---|---|---|---|---|
| Wistar Han Rat | 50 | 100 | 0.5 | 202 ± 27 | 716 ± 62 | N/A | N/A | 0.415 ± 0.098 |

| | | | | | | | | |
|---|---|---|---|---|---|---|---|---|
| Formula **(Ia):** 1 nM = 0.968 ng/mL; d = day; h = hour Values are the mean ± SD from 3 animals | | | | | | | | |

### M. 28.36 w/w% water, 60.73 w/w% PEG 300, 9.23 w/w% of a sodium salt of a compound of Formula (Ia), and 1.68 w/w% poloxamer 188 solution (100 mg/mL of compound of Formula (Ia))

A 100 mg/ml solution of a compound of Formula **(Ia)** having 28.36 w/w% water, 60.73 w/w% PEG 300, 9.23 w/w% of a sodium salt of a compound of Formula **(Ia),** and 1.68 w/w% poloxamer 188 was prepared. The solution was administered subcutaneously to dogs at a dose of 6 mg/kg and the pharmacokinetic (PK) profile was determined. FIG. 12B shows a plot of the plasma concentration of the compound of Formula **(Ia)** as a function of time. Plasma concentrations of the compound of Formula **(Ia)** were maintained above the target trough concentration of 20 nM for at least 14 days (2 weeks). The results are summarized in Table L below.

**Table L. PK parameters of the compound of Formula (Ia) following SC administration in solution in dogs**

| **Species** | **Dose (mg/kg)** | **Dose Concentration (mg/mL)** | **Dose Volume (mL/kg)** | **AUC_{0- 24h} (µM•h)** | **AUC_{0- 14d} (µM•h)** | **Cₘₐₓ (µM)** | **Tₘₐₓ (h)** | **C_{14d} (µM)** |
|---|---|---|---|---|---|---|---|---|
| Beagle Dog | 6 | 100 | 0.06 | 6.05 ± 3.43 | 104 ± 26 | N/A | N/A | 219 ± 70 |

| | | | | | | | | |
|---|---|---|---|---|---|---|---|---|
| Formula **(Ia):** 1 nM = 0.968 ng/mL; d = day; h = hour Values are the mean ± SD from 3 animals | | | | | | | | |

### N. 27.51 w/w% water, 58.92 w/w% PEG 300, 11.48 w/w% of a sodium salt of the compound of Formula (Ia), and 2.09 w/w% poloxamer 188 solution (125 mg/mL of compound of Formula (Ia))

A 125 mg/ml solution of a compound of Formula **(Ia)** having 27.51 w/w% water, 58.92 w/w% PEG 300, 11.48 w/w% of a sodium salt of the compound of Formula **(Ia),** and 2.09 w/w% poloxamer 188 was prepared. The solution was administered subcutaneously to rats at a dose of 50 mg/kg and to dogs at a dose of 6 mg/kg and the pharmacokinetic (PK) profile was determined. FIG. 13A shows a plot of the plasma concentration of the compound of Formula **(Ia)** as a function of time in the rat and FIG. 13B shows a plot of the plasma concentration of the compound of Formula **(Ia)** as a function of time in the dog. In the rat, plasma concentrations of the compound of Formula **(Ia)** were maintained above the target trough concentration of 20 nM for at least 28 days (4 weeks). In the dog, plasma concentrations of the compound of Formula **(Ia)** were maintained above the target trough concentration of 20 nM for at least 14 days (2 weeks). The results are summarized in Tables M and N below.

**Table M. PK parameters of the compound of Formula (Ia) following SC administration in solution in rats**

| **Species** | **Dose (mg/kg)** | **Dose Concentration (mg/mL)** | **Dose Volume (mL/kg)** | **AUC_{0- 7d} (µM•h)** | **AUC_{0- 28d} (µM•h)** | **Cₘₐₓ (µM)** | **Tₘₐₓ (h)** | **C_{d28} (µM)** |
|---|---|---|---|---|---|---|---|---|
| Wistar Han Rat | 50 | 125 | 0.4 | 153 ± 38 | 673 ± 67 | N/A | N/A | 0.0279 ± 0.0262 |

| | | | | | | | | |
|---|---|---|---|---|---|---|---|---|
| Formula **(Ia):** 1 nM = 0.968 ng/mL; d = day; h = hour Values are the mean ± SD from 3 animals | | | | | | | | |

**Table N. PK parameters of the compound of Formula (Ia) following SC administration in solution in dogs**

| **Species** | **Dose (mg/kg)** | **Dose Concentration (mg/mL)** | **Dose Volume (mL/kg)** | **AUC_{0- 24h} (µM•h)** | **AUC_{0- 14d} (µM•h)** | **Cₘₐₓ (µM)** | **Tₘₐₓ (h)** | **C_{14d} (µM)** |
|---|---|---|---|---|---|---|---|---|
| Beagle Dog | 6 | 125 | 0.048 | 4.71 ± 1.51 | 90.3 ± 3.5 | N/A | N/A | 173 ± 70 |

| | | | | | | | | |
|---|---|---|---|---|---|---|---|---|
| Formula **(Ia):** 1 nM = 0.968 ng/mL; d = day; h = hour Values are the mean ± SD from 3 animals | | | | | | | | |

### O. 26.6 w/w% water, 56.97 w/w% PEG 300, 13.39 w/w% of a compound of Formula (Ia), 0.57 w/w% NaOH, and 2.49 w/w% poloxamer 188 solution (150 mg/mL of compound of Formula (Ia))

A 150 mg/ml solution of a compound of Formula **(Ia)** having 26.6 w/w% water, 56.97 w/w% PEG 300, 13.39 w/w% of a compound of Formula **(Ia),** 0.57 w/w% NaOH, and 2.49 w/w% poloxamer 188 was prepared. The solution was administered subcutaneously to rats at a dose of 50 mg/kg and the pharmacokinetic (PK) profile was determined. FIG. 14A shows a plot of the plasma concentration of the compound of Formula **(Ia)** as a function of time. Plasma concentrations of the compound of Formula **(Ia)** were maintained above the target trough concentration of 20 nM for at least 98 days (14 weeks). The results are summarized in Table O below.

**Table O. PK parameters of the compound of Formula (Ia) following SC administration in solution in rats**

| **Species** | **Dose (mg/kg)** | **Dose Concentration (mg/mL)** | **Dose Volume (mL/kg)** | **AUC_{0- 98d} (µM•h)** | **AUC_{0- 112d} (µM•h)** | **Cₘₐₓ (µM)** | **Tₘₐₓ (h)** | **C_{d84} (µM)** |
|---|---|---|---|---|---|---|---|---|
| Wistar Han Rat | 50 | 150 | 0.330 | 949 ± 114 | 963 ± 102 | N/A | N/A | 0.0533 ± 0.0457 |

| | | | | | | | | |
|---|---|---|---|---|---|---|---|---|
| Formula **(Ia):** 1 nM = 0.968 ng/mL; d = day; h = hour Values are the mean ± SD from 3 animals | | | | | | | | |

### P. 26.6 w/w% water, 56.97 w/w% PEG 300, 13.39 w/w% of a compound of Formula (Ia), 0.57 w/w% NaOH, and 2.49 w/w% poloxamer 188 solution (150 mg/mL of compound of Formula (Ia))

A 150 mg/ml solution of a compound of Formula **(Ia)** having 26.6 w/w% water, 56.97 w/w% PEG 300, 13.39 w/w% of a compound of Formula **(Ia),** 0.57 w/w% NaOH, and 2.49 w/w% poloxamer 188 was prepared. The solution was administered subcutaneously to dogs at a dose of 6 mg/kg and the pharmacokinetic (PK) profile was determined. FIG. 14B shows a plot of the plasma concentration of the compound of Formula **(Ia)** as a function of time. Plasma concentrations of the compound of Formula **(Ia)** were maintained above the target trough concentration of 20 nM for at least 56 days (8 weeks). The results are summarized in Table P below.

**Table P. PK parameters of the compound of Formula (Ia) following SC administration in solution in dogs**

| **Species** | **Dose (mg/kg)** | **Dose Concentration (mg/mL)** | **Dose Volume (mL/kg)** | **AUC_{0- 56d} (µM•h)** | **AUC_{0- 84d} (µM•h)** | **Cₘₐₓ (µM)** | **Tₘₐₓ (h)** | **C_{56d} (µM)** |
|---|---|---|---|---|---|---|---|---|
| Beagle Dog | 6 | 150 | 0.04 | 69.0 ± 13.9 | 76.6 ± 8.5 | 0.101 ± 0.044 | 240 ± 374 | 0.0249 ± 0 |

| | | | | | | | | |
|---|---|---|---|---|---|---|---|---|
| Formula **(Ia):** 1 nM = 0.968 ng/mL; d = day; h = hour Values are the mean ± SD from 3 animals | | | | | | | | |

### Q. 21.97 w/w% water, 47.05 w/w% PEG 300, 25.21 w/w% of a compound of Formula (Ia), 1.08 w/w% of NaOH, and 4.69 w/w% poloxamer 188 solution (300 mg/mL of compound of Formula (Ia))

A 300 mg/ml solution of a compound of Formula **(Ia)** having 21.97 w/w% water, 47.05 w/w% PEG 300, 25.21 w/w% of a compound of Formula **(Ia),** 1.08 w/w% of NaOH, and 4.69 w/w% poloxamer 188 was prepared. The solution was administered subcutaneously to rats at a dose of 50 mg/kg and to dogs at a dose of 6 mg/kg and the pharmacokinetic (PK) profile was determined. FIG. 15A shows a plot of the plasma concentration of the compound of Formula

**(Ia)** as a function of time in the rat and FIG. 15B shows a plot of the plasma concentration of the compound of Formula **(Ia)** as a function of time in the dog. In the rat, plasma concentrations of the compound of Formula **(Ia)** were maintained above the target trough concentration of 20 nM for at least 98 days (14 weeks). In the dog, plasma concentrations of the compound of Formula **(Ia)** were maintained above the target trough concentration of 20 nM for at least 56 days (8 weeks). The results are summarized in Tables Q and R below.

**Table Q. PK parameters of the compound of Formula (Ia) following SC administration in solution in rats**

| **Species** | **Dose (mg/kg)** | **Dose Concentration (mg/mL)** | **Dose Volume (mL/kg)** | **AUC_{0- 98d} (µM•h)** | **AUC_{0- 140d} (µM•h)** | **Cₘₐₓ (µM)** | **Tₘₐₓ (h)** | **C_{d98} (µM)** |
|---|---|---|---|---|---|---|---|---|
| Wistar Han Rat | 50 | 300 | 0.170 | 928 ± 175 | 948 ± 169 | 0.915 ± 0.248 | 504 ± 0 | 0.0513 ± 0.0365 |

| | | | | | | | | |
|---|---|---|---|---|---|---|---|---|
| Formula **(Ia):** 1 nM = 0.968 ng/mL; d = day; h = hour Values are the mean ± SD from 3 animals | | | | | | | | |

**Table R. PK parameters of the compound of Formula (Ia) following SC administration in solution in dogs**

| **Species** | **Dose (mg/kg)** | **Dose Concentration (mg/mL)** | **Dose Volume (mL/kg)** | **AUC_{0- 56d} (µM•h)** | **AUC_{0- 84d} (µM•h)** | **Cₘₐₓ (µM)** | **Tₘₐₓ (h)** | **C_{56d} (µM)** |
|---|---|---|---|---|---|---|---|---|
| Beagle Dog | 6 | 300 | 0.02 | 55.4 ± 20.8 | 65.4 ± 21.5 | 0.069 ± 0.022 | 840 ± 0 | 0.0304 ± 0.0072 |

| | | | | | | | | |
|---|---|---|---|---|---|---|---|---|
| Formula **(Ia):** 1 nM = 0.968 ng/mL; d = day; h = hour Values are the mean ± SD from 3 animals | | | | | | | | |

### R. Liquid-filled Capsules

A single dose of the compound of Formula **(Ia),** formulated as liquid-filled capsules (LFC) containing 100% glycerol monocaprylocaprate, was administered orally at 7.5 mg fixed dose to 6 beagle dogs (Table S) and the pharmacokinetic (PK) profile was determined. FIG. 16 shows a plot of the plasma concentration of the compound of Formula **(Ia)** as a function of time. Plasma concentrations of the compound of Formula **(Ia)** were maintained above the target trough concentration of 20 nM for at least 72 hours (3 days).

**Table S. PK parameters of the compound of Formula (Ia) following oral administration in liquid-filled capsules at 7.5 mg fixed dose to dogs**

| **Species** | **Dose (mg/kg)** | **Dose Concentration (mg/mL)** | **Dose Volume (mL/kg)** | **AUC_{0- 72h} (µM•h)** | **AUC_{0- 14d} (µM•h)** | **Cₘₐₓ (µM)** | **Tₘₐₓ (h)** | **C₇₂ₕ (µM)** |
|---|---|---|---|---|---|---|---|---|
| Beagle Dog | 6 | 50 | 0.12 | 5.070 ± 4.649 | 108 ± 16 | 201 ± 129 | 11.0 ± 10.1 | 0.0201 ± 0.0244 |

| | | | | | | | | |
|---|---|---|---|---|---|---|---|---|
| Formula **(Ia):** 1 nM = 0.968 ng/mL; d = day; h = hour Values are the mean ± SD from 6 animals | | | | | | | | |

### S. Spray-Dried Dispersion (SDD) Tablets

A tablet, prepared from a spray-dried disperstion of a sodium salt of the compound of Formula **(Ia),** was prepared in a composition shown in Table T below (with an outer film coating made of Opadry^{®} II White 85F 18422, wherein the outer film coating provides a 3% weight gain based on the uncoated tablet) and was administered orally at 300 mg fixed dose to 6 beagle dogs (Table U) and the pharmacokinetic (PK) profile was determined. FIG. 17 shows a plot of the plasma concentration of the compound of Formula **(Ia)** as a function of time. Plasma concentrations of the compound of Formula **(Ia)** were maintained above the target trough concentration of 20 nM for at least 14 days (2 weeks).

**Table T. Composition of SDD tablets at 300 mg fixed dose**

| Ingredient | Amount (w/w%) |
|---|---|
| Sodium salt of compound of Formula **(Ia)** | 20.46 |
| Copovidone | 4.88 |
| Poloxamer 407 | 1.33 |
| Microcrystalline Cellulose | 21.28 |
| Mannitol | 42.55 |
| Croscarmellose Sodium | 8.00 |
| Magnesium Stearate | 1.50 |

**Table U. PK parameters of the compound of Formula (Ia) following oral administration in SDD tablets at 300 mg fixed dose to dogs**

| **Species** | **Dose** | **AUC_{0- 72h} (µM•h)** | **AUC_{inf} (µM•h)** | **Cₘₐₓ (µM)** | **Tₘₐₓ (h)** | **C₇₂ₕ (µM)** |
|---|---|---|---|---|---|---|
| Beagle Dog | 300 mg fixed | 15.8 ± 10.0 | 18.5 ± 11.1 | 0.718 ± 0.409 | 5.3 ± 3.3 | 0.0614 ± 0.0300 |

| | | | | | | |
|---|---|---|---|---|---|---|
| Formula **(Ia):** 1 nM = 0.968 ng/mL; h = hour Values are the mean ± SD from 6 animals | | | | | | |

### Example 4: Pharmacology

### A. Activity against HIV clinical isolates

The antiretroviral activity of the compound of Formula **(Ia)** was tested against a panel of 22 HIV-1 clinical isolates of HIV-1 and two HIV-2 isolates in freshly isolated, PHA-stimulated human peripheral blood mononuclear cells (PBMCs). The HIV-1 isolates included representative strains from subtypes A, B, C, D, E, F, G, N, O, AE, and AG. Following a 7-day infection, virus production was determined by measuring reverse transcriptase activity in cellfree culture supernatants. The compound of Formula **(Ia)** displayed potent antiviral activity against all HIV-1 clinical isolates tested, with a mean EC₅₀ value of 0.05 ± 0.03 nM (range of 0.02 to 0.16 nM) (Table 4). When tested in parallel against the same panel of viruses, DTG and AZT exhibited mean EC₅₀ values of 0.70 ± 0.26 nM and 19.0 ± 13.5 nM, respectively. The compound of Formula **(Ia)** was 11- to 20-fold less active against HIV-2 relative to HIV-1 as evidenced by its higher EC₅₀ value of 0.57 nM and 1.02 nM against HIV-2 strains CBL-20 and CDC310319, respectively (Table 10). Both of these HIV-2 isolates were susceptible to dolutegravir (DTG) and atazanavir (AZT).

**Table 10. Antiviral activity against HIV clinical isolates**

| | | | **EC₅₀ in human PBMCs (nM)^{a}** | | |
|---|---|---|---|---|---|
| **Subtype** | **HIV Isolate** | **Genbank Accession #** | **(Ia)** | **DTG** | **AZT** |
| A | 92UG031 | L34667 | 0.04 | 0.63 | 51.6 |
| A | 92UG037 | AB253428 | 0.07 | 0.47 | 21.7 |
| B | 89BZ_167 | AY173956 | 0.08 | 1.18 | 10.1 |
| B | 90US_873 | AY713412 | 0.04 | 0.82 | 13.2 |
| B | 91US001 | AY173952 | 0.03 | 0.53 | 20.3 |
| B | 91US004 | AY173955 | 0.04 | 0.40 | 19.7 |
| B | 96TH_NP1538 | AY713408 | 0.05 | 0.40 | 4.10 |
| B | Ba-L | AY713409 | 0.04 | 0.40 | 43.2 |
| B | JR-CSF | M38429 | 0.04 | 0.41 | 4.53 |
| C | 92BR025 | U52953 | 0.05 | 0.91 | 16.9 |
| C | 98US_MSC5016 | AY444801 | 0.10 | 0.77 | 37.8 |
| D | 92UG001 | AJ320484 | 0.02 | 1.09 | 7.29 |
| D | 98UG_57128 | AF484502 | 0.03 | 0.70 | 6.61 |
| E | CMU02 | AY494967 | 0.05 | 0.81 | 8.13 |
| E | CMU08 | U48268 | 0.05 | 0.96 | 34.1 |
| F | 93BR020 | AF005494 | 0.05 | 0.38 | 25.1 |
| G | JV1083 | U88826 | 0.16 | 0.93 | 20.5 |
| Group N | YBF30 | AJ006022 | 0.02 | 0.54 | 8.96 |
| Group O | BCF01 | AF458283 | 0.04 | 0.64 | 10.5 |
| CRF01_AE | 90TH_CM235 | AF259954 | 0.04 | 1.02 | 10.7 |
| CRF02_AG | 01CM0008BBY | AY371124 | 0.05 | 1.04 | 8.21 |
| CRF02_AG | 91DJ263 | AF063223 | 0.04 | 0.45 | 34.9 |
| **HIV-1** | | **Mean ± s.d.** | **0.05 ± 0.03** | **0.70 ± 0.26** | **19.0 ± 13.5** |
| **EC₅₀ (nM)** | | **Median** | **0.04** | **0.67** | **15.1** |
| | | **Range** | **0.02 - 0.16** | **0.38 - 1.18** | **4.1 - 51.6** |
| HIV-2 | CBL-20 | AY965906 | 0.57 | 0.36 | 14.3 |
| HIV-2 | CDC310319 | AT965902 | 1.02 | 1.81 | 28.8 |

| | | | | | |
|---|---|---|---|---|---|
| ^{a} EC₅₀ values represented the mean of triplicate measurements in human PBMCs | | | | | |

### B. Activity against viruses resistant to other antiretroviral classes

The antiviral activity of the compound of Formula **(Ia)** was tested against a panel of known HIV-1 mutants resistant to HIV PIs (ATV and DRV), NRTIs (TFV and FTC), NNRTIs (RPV and EFV) or INSTIs (RAL, EVG and DTG) using a cytopathic antiviral assay in MT-2 cells. The compound of Formula **(Ia)** was fully active against all tested mutants resistant to PIs (Table 11), NRTIs (Table 12), NNRTIs (Table 13) or INSTIs (Table 14). These results support the use of the compound of Formula **(Ia)** in the treatment of infection with HIV-1 variants resistant to these four approved classes of antiretrovirals.

**Table 11. Activity of the compound of Formula (Ia) against PI-resistant viruses**

| **Compound^{a}** | **WT HXB2D EC₅₀ (nM)^{b}** | **EC₅₀ Fold-Change^{b,c}** | | | |
|---|---|---|---|---|---|
| | | **150V** | **184V/L90M** | **G48V/V82A/L90M** | **G48V/V82S** |
| **(Ia)** | 0.24 ± 0.14 | 0.6 ± 0.2 | 0.3 ± 0.1 | 0.4 ± 0.2 | 0.4 ± 0.1 |
| **ATV** | 4.18 ± 0.46 | 3.5 ± 0.4 | 33.1 ± 7.8 | 32.5 ± 11.9 | 15.4 ± 3.1 |
| **DRV** | 2.82 ± 0.47 | 31.6 ± 22.8 | 1.9 ± 0.9 | 0.5 ± 0.2 | 0.3 ± 0.1 |
| **EFV** | 1.64 ± 0.55 | 3.0 ± 0.9 | 0.4 ± 0.1 | 0.9 ± 0.4 | 0.7 ± 0.1 |
| **EVG** | 2.02 ± 0.88 | 1.7 ± 1.0 | 1.0 ± 0.7 | 0.8 ± 0.4 | 0.7 ± 0.3 |

| | | | | | |
|---|---|---|---|---|---|
| ^{a} ATV = atazanavir (PI); DRV = darunavir (DRV); EFV = efavirenz (NNRTI); EVG = elvitegravir (INSTI) ^{b} The data represent the mean (± s.d.) of three independent experiments performed in triplicate ^{c} Mutant/WT EC₅₀ ratio | | | | | |

**Table 12. Activity of the compound of Formula (Ia) against NRTI-resistant viruses**

| **Compound^{a}** | **WT LAI EC₅₀ (nM)^{b}** | **EC₅₀ Fold-Change^{b,c}** | | |
|---|---|---|---|---|
| | | **K65R** | **M184V** | **6TAMs** |
| **(Ia)** | 0.14 ± 0.03 | 0.7 ± 0.2 | 0.5 ± 0.3 | 0.4 ± 0.2 |
| **TFV** | 4,458 ± 1,872 | 2.4 ± 1.0 | 0.9 ± 1.0 | 3.0 ± 1.1 |
| **FTC** | 2,406 ± 1,156 | 12.8 ± 2.4 | > 20.8 | 4.1 ± 2.5 |
| **EVG** | 2.64 ± 0.90 | 0.8 ± 0.2 | 1.1 ± 0.7 | 0.6 ± 0.4 |
| **ATV** | 4.78 ± 1.26 | 0.9 ± 0.3 | 0.6 ± 0.2 | 0.5 ± 0.2 |

| | | | | |
|---|---|---|---|---|
| ^{a} TFV = tenofovir (NRTI); FTC = emtracitabine (NRTI); EVG = elvitegravir (INSTI); ATV = atazanavir (PI) ^{b} The data represent the mean (± s.d.) of three independent experiments performed in triplicate ^{c} Mutant/WT EC₅₀ ratio | | | | |

**Table 13. Activity of the compound of Formula (Ia) against NNRTI-resistant viruses**

| **Compound^{a}** | **WT HXB2D EC₅₀ (nM)^{b}** | **EC₅₀ Fold-Change^{b,c}** | | | | |
|---|---|---|---|---|---|---|
| | | **K103N** | **Y181C** | **Y188L** | **L100I / K103N** | **K103N / Y181C** |
| **(Ia)** | 0.18 ± 0.02 | 0.3 ± 0.1 | 1.6 ± 1.0 | 0.5 ± 0.1 | 0.5 ± 0.2 | 0.5 ± 0.2 |
| **RPV** | 0.93 ± 0.23 | 0.7 ± 0.3 | 5.9 ± 4.3 | 6.9 ± 1.3 | 7.8 ± 2.6 | 3.2 ± 1.4 |
| **EFV** | 2.23 ± 0.18 | 14.2 ± 2.7 | 3.3 ± 2.5 | > 22.4 | > 22.4 | > 22.4 |
| **ATV** | 4.60 ± 1.48 | 0.6 ± 0.3 | 1.8 ± 1.0 | 0.6 ± 0.3 | 0.6 ± 0.3 | 0.6 ± 0.3 |

| | | | | | | |
|---|---|---|---|---|---|---|
| ^{a} RPV = rilpivirine (NNRTI); EFV = efavirenz (NNRTI); ATV = atazanavir (PI) ^{b} The data represent the mean (± s.d.) of three independent experiments performed in triplicate ^{c} Mutant/WT EC₅₀ ratio | | | | | | |

**Table 14. Activity of the compound of Formula (Ia) against INSTI-resistant viruses**

| **Compound^{a}** | **WT HXB2D EC₅₀ (nM)^{b}** | **EC₅₀ Fold-Change^{b,c}** | | | | | |
|---|---|---|---|---|---|---|---|
| | | **Y143R** | **E138K / Q148K** | **G140S / Q148R** | **E92Q / N155H** | **N155H / Q148R** | **R263K / M50I** |
| **(Ia)** | 0.13 ± 0.03 | 0.5 ± 0.1 | 0.7 ± 0.3 | 0.8 ± 0.3 | 0.9 ± 0.4 | 1.3 ± 0.7 | 0.9 ± 0.5 |
| **RAL** | 6.41 ± 2.15 | 10.1 ± 1.2 | > 15.6 | > 15.6 | > 15.6 | 13.9 ± 2.2 | 1.6 ± 1.2 |
| **EVG** | 1.87 ± 0.19 | 2.1 ± 0.2 | > 53.5 | > 53.5 | > 53.5 | 51.0 ± 2.5 | 5.1 ± 3.7 |
| **DTG** | 2.24 ± 0.46 | 0.8 ± 0.3 | 7.3 ± 4.1 | 6.9 ± 3.1 | 3.0 ± 1.1 | 1.5 ± 0.6 | 2.8 ± 2.1 |
| **EFV** | 1.37 ± 0.51 | 0.9 ± 0.6 | 0.7 ± 0.3 | 1.0 ± 0.3 | 1.2 ± 0.6 | 1.1 ± 0.5 | 1.3 ± 0.9 |
| **ATV** | 3.7 ± 1.07 | 0.8 ± 0.2 | 0.8 ± 0.3 | 1.1 ± 0.5 | 1.0 ± 0.4 | 0.9 ± 0.4 | 1.1 ± 0.6 |

| | | | | | | | |
|---|---|---|---|---|---|---|---|
| ^{a} RAL = raltegravir (INSTI); EVG = elvitegravir (INSTI); DTG = dolutegravir (INSTI); EFV = efavirenz (NNRTI); ATV = atazanavir (PI) ^{b} The data represent the mean (± s.d.) of three independent experiments performed in triplicate ^{c} Mutant/WT EC₅₀ ratio | | | | | | | |

### C. Activity in combination with other antiretrovirals

The antiviral activity of the compound of Formula **(Ia)** was evaluated in MT-2 cells infected with HIV-1 IIIb in combination with selected approved anti-HIV drugs: tenofovir alafenamide (TAF), efavirenz (EFV), dolutegravir (DTG), and darunavir (DRV). The combinatorial effects of the compound of Formula **(Ia)** were analyzed by the Prichard and Shipman method using MacSynergy II software. The results of the combination assays were expressed as the mean combination volumes (µM²%) that were calculated at the 95% confidence level from three independent experiments performed in triplicate (Table 15). The combination effects were defined as:
- Highly synergistic: > 100 µM2%
- Moderate synergy: > 50 and < 100 µM2%
- Additive: < 50 and > -50 µM2%
- Moderate antagonism: < -50 and > -100 µM2%
- Highly antagonistic: < -100 µM2%

Combinations of the compound of Formula **(Ia)** with EFV, DTG or DRV showed highly synergistic anti-HIV activity. The compound of Formula **(Ia)** was slightly synergistic when combined with TAF. The compound of Formula **(Ia),** combined with itself, TAF combined with EVG, and ribavirin combined with d4T were used as additivity, synergy and antagonism controls, respectively.

**Table 15. Antiviral activity of the compound of Formula (Ia) in combination with other antiretrovirals**

| **Drug Combination^{a}** | **Combination Volume (µM²%)^{b}** | | **Combination Effect** |
|---|---|---|---|
| | **Type** | **Mean** | |
| **(Ia) + TAF** | Synergy | 87 ± 32 | Moderate Synergy |
| | Antagonism | -8 ± 7 | |
| **(Ia) + EFV** | Synergy | 101 ± 40 | Highly Synergistic |
| | Antagonism | -8 ± 8 | |
| **(Ia) + DTG** | Synergy | 116 ± 13 | Highly Synergistic |
| | Antagonism | -8 ± 7 | |
| **(Ia) + DRV** | Synergy | 119 ± 39 | Highly Synergistic |
| | Antagonism | -3 ± 3 | |
| **(Ia) + (Ia)** | Synergy | 18 ± 8 | Additive |
| | Antagonism | -16 ± 6 | |
| **TAF + EVG** | Synergy | 164 ± 24 | Highly Synergistic |
| | Antagonism | -3 ± 3 | |
| **RBV + d4T** | Synergy | 0 ± 0 | Highly Antagonistic |
| | Antagonism | -398 ± 23 | |

| | | | |
|---|---|---|---|
| ^{a} TAF = tenofovir alafenamide (NRTI); EFV = efavirenz (NNRTI); DTG = dolutegravir (INSTI); DRV = darunavir (PI); EVG = elvitegravir (INSTI); RBV = ribavirine; d4T = stavudine ^{b} The synergy/antagonism volumes represent the mean (± s.d.) of three independent experiments performed in triplicate | | | |

## Claims

1. A pharmaceutical composition comprising a sodium salt of the compound of Formula (Ia): 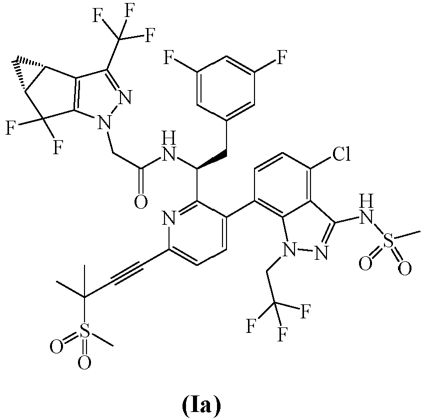 wherein the pharmaceutical composition is in the form of a solution and the solution comprises 35 w/w% to 75 w/w% PEG 300, 10 w/w% to 40 w/w% water and 5 w/w% to 35 w/w% of the sodium salt of the compound of Formula **(Ia).**

2. The pharmaceutical composition of claim 1, wherein the solution comprises 15 w/w% to 35 w/w% water, 45 w/w% to 65 w/w% PEG 300, and 10 w/w% to 30 w/w% of the sodium salt of the compound of Formula **(Ia).**

3. The pharmaceutical composition of claim 1, wherein the solution comprises 20 w/w% to 30 w/w% water, 48 w/w% to 60 w/w% PEG 300, and 11 w/w% to 28 w/w% of the sodium salt of the compound of Formula **(Ia).**

4. The pharmaceutical composition of claim 1, wherein the solution comprises 21 w/w% to 29 w/w% water, 50 w/w% to 59 w/w% PEG 300, and 13 w/w% to 27 w/w% of the sodium salt of the compound of Formula **(Ia).**

5. The pharmaceutical composition of claim 1, wherein the solution comprises 23.4 w/w% to 27.5 w/w% water, 50.1 w/w% to 58.8 w/w% PEG 300, and 13.7 w/w% to 26.5 w/w% of the sodium salt of the compound of Formula **(Ia).**

6. The pharmaceutical composition of any one of claims 1-5, wherein the solution comprises 23.41 w/w% water, 50.13 w/w% PEG 300, and 26.46 w/w% of the sodium salt of the compound of Formula **(Ia).**

7. The pharmaceutical composition of any one of claims 1-6 for use in a method of treating human immunodeficiency virus (HIV) infection in a heavily treatment-experienced patient.

8. The pharmaceutical composition for use according to claim 7, wherein the HIV infection is an HIV-1 infection **characterized by** HIV-1 mutant resistance to one or more antiretroviral medications, or two or more antiretroviral medications, or three or more antiretroviral medications.

9. The pharmaceutical composition for use according to claim 8, wherein the HIV-1 mutant is resistant to a protease inhibitor (PI), a nucleoside or nucleotide reverse transcriptase inhibitor (NRTI), a non-nucleoside or non-nucleotide reverse transcriptase inhibitor (NNRTI), or an integrase strand transfer inhibitor (INSTI), optionally wherein
(i) the NRTI is selected from emtricitabine, lamivudine (3TC), zidovudine (azidothymidine (AZT)), didanosine (ddI), dideoxyinosine, tenofovir, tenofovir alafenamide, tenofovir disoproxil fumarate, stavudine (d4T), zalcitabine (dideoxycytidine, ddC), and abacavir; or
(ii) the NNRTI is selected from efavirenz, etravirine, rilpivirine, nevirapine, and delavirdine; or
(iii) the PI is selected from amprenavir, atazanavir, darunavir, fosamprenavir, indinavir, lopinavir, nelfinavir, ritonavir, saquinavir, and tipranavir; or
(iv) the INSTI is selected from raltegravir, elvitegravir, dolutegravir, cabotegravir, and bictegravir.

10. The pharmaceutical composition for use according to claim 9, wherein
(i) the HIV-1 mutant resistant to a protease inhibitor is selected from I50V, I84V/L90M, G48V/V82A/L90M, and G48V/V82S; or
(ii) the HIV-1 mutant resistant to a nucleoside or nucleotide reverse transcriptase inhibitor is selected from K65R, M184V, and 6TAMs; or
(iii) the HIV-1 mutant resistant to a non-nucleoside or non-nucleotide reverse transcriptase inhibitor is selected from K103N, Y181C, Y188L, L100I/K103N, and K103N/Y181C; or
(iv) the HIV-1 mutant resistant to an integrase strand transfer inhibitor is selected from Y143R, E138K/Q148K, G140S/Q148R, E92Q/N155H, N155H/Q148R, and R263K/M50I.

11. The pharmaceutical composition for use according to claim 9 or claim 10, wherein the patient had been previously treated with at least one antiretroviral medication for at least 3 months or at least 6 months or at least 9 months or at least 12 months.

12. The pharmaceutical composition for use according to any one of claims 9-11, wherein the patient failed a prior HIV treatment regimen comprising administration of at least one antiretroviral medication, optionally wherein the prior treatment regimen comprised administration of at least one antiretroviral medication from each of two different classes or at least three classes of antiretroviral medications, wherein the different classes of antiretroviral medications are selected from a nucleoside reverse transcriptase inhibitor (NRTI), a non-nucleoside reverse transcriptase inhibitor (NNRTI), a protease inhibitor (PI), and an integrase strand transfer inhibitor (INSTI).

## Patentansprüche

1. Pharmazeutische Zusammensetzung, umfassend ein Natriumsalz der Verbindung der Formel (Ia): 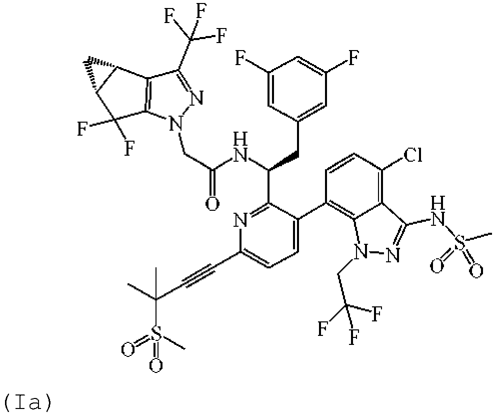 wobei die pharmazeutische Zusammensetzung in Form einer Lösung vorliegt und die Lösung 35 Gew.-% bis 75 Gew.-% PEG 300, 10 Gew.-% bis 40 Gew.-% Wasser und 5 Gew.-% bis 35 Gew.-% des Natriumsalzes der Verbindung der Formel (Ia) umfasst.

2. Pharmazeutische Zusammensetzung nach Anspruch 1, wobei die Lösung 15 Gew.-% bis 35 Gew.-% Wasser, 45 Gew.-% bis 65 Gew.-% PEG 300 und 10 Gew.-% bis 30 Gew.-% des Natriumsalzes der Verbindung der Formel (Ia) umfasst.

3. Pharmazeutische Zusammensetzung nach Anspruch 1, wobei die Lösung 20 Gew.-% bis 30 Gew.-% Wasser, 48 Gew.-% bis 60 Gew.-% PEG 300 und 11 Gew.-% bis 28 Gew.-% des Natriumsalzes der Verbindung der Formel (Ia) umfasst.

4. Pharmazeutische Zusammensetzung nach Anspruch 1, wobei die Lösung 21 Gew.-% bis 29 Gew.-% Wasser, 50 Gew.-% bis 59 Gew.-% PEG 300 und 13 Gew.-% bis 27 Gew.-% des Natriumsalzes der Verbindung der Formel (Ia) umfasst.

5. Pharmazeutische Zusammensetzung nach Anspruch 1, wobei die Lösung 23,4 Gew.-% bis 27,5 Gew.-% Wasser, 50,1 Gew.-% bis 58,8 Gew.-% PEG 300 und 13,7 Gew.-% bis 26,5 Gew.-% des Natriumsalzes der Verbindung der Formel (Ia) umfasst.

6. Pharmazeutische Zusammensetzung nach einem der Ansprüche 1-5, wobei die Lösung 23,41 Gew.-% Wasser, 50,13 Gew.-% PEG 300 und 26,46 Gew.-% des Natriumsalzes der Verbindung der Formel (Ia) umfasst.

7. Pharmazeutische Zusammensetzung nach einem der Ansprüche 1-6 zur Verwendung in einem Verfahren zur Behandlung einer Infektion mit dem humanen Immundefizienzvirus (HIV) bei einem erheblich behandlungserfahrenen Patienten.

8. Pharmazeutische Zusammensetzung zur Verwendung nach Anspruch 7, wobei die HIV-Infektion eine HIV-1-Infektion ist, die durch HIV-1-Mutantenresistenz gegen ein oder mehrere antiretrovirale Medikamente oder zwei oder mehr antiretrovirale Medikamente oder drei oder mehr antiretrovirale Medikamente gekennzeichnet ist.

9. Pharmazeutische Zusammensetzung zur Verwendung nach Anspruch 8, wobei die HIV-1-Mutante gegenüber einem Proteaseinhibitor (PI), einem Nukleosid- oder Nukleotid-Reverse-Transkriptase-Inhibitor (NRTI), einem Nicht-Nukleosid- oder Nicht-Nukleotid-Reverse-Transkriptase-Inhibitor (NNRTI) oder einem Integrase-Strangtransfer-Inhibitor (INSTI) resistent ist, wobei optional
(i) der NRTI ausgewählt ist aus Emtricitabin, Lamivudin (3TC), Zidovudin (Azidothymidin (AZT)), Didanosin (ddI), Didesoxyinosin, Tenofovir, Tenofoviralafenamid, Tenofovirdisoproxilfumarat, Stavudin (d4T), Zalcitabin (Didesoxycytidin, ddC) und Abacavir; oder
(ii) der NNRTI ausgewählt ist aus Efavirenz, Etravirin, Rilpivirin, Nevirapin und Delavirdin; oder
(iii) der PI ausgewählt ist aus Amprenavir, Atazanavir, Darunavir, Fosamprenavir, Indinavir, Lopinavir, Nelfinavir, Ritonavir, Saquinavir und Tipranavir; oder
(iv) der INSTI ausgewählt ist aus Raltegravir, Elvitegravir, Dolutegravir, Cabotegravir und Bictegravir.

10. Pharmazeutische Zusammensetzung zur Verwendung nach Anspruch 9, wobei
(i) die HIV-1-Mutante, die gegen einen Proteaseinhibitor resistent ist, ausgewählt ist aus I50V, I84V/L90M, G48V/V82A/L90M und G48V/V82S; oder
(ii) die HIV-1-Mutante, die gegen einen Nukleosid- oder Nukleotid-Reverse-Transkriptase-Inhibitor resistent ist, ausgewählt ist aus K65R, M184V und 6TAMs; oder
(iii) die HIV-1-Mutante, die gegen einen Nicht-Nukleosid- oder Nicht-Nukleotid-Reverse-Transkriptase-Inhibitor resistent ist, ausgewählt ist aus K103N, Y181C, Y188L, L100I/K103N und K103N/Y181C; oder
(iv) die HIV-1-Mutante, die gegen einen Integrase-Strangtransfer-Inhibitor resistent ist, ausgewählt ist aus Y143R, E138K/Q148K, G140S/Q148R, E92Q/N155H, N155H/Q148R und R263K/M50I.

11. Pharmazeutische Zusammensetzung zur Verwendung nach Anspruch 9 oder Anspruch 10, wobei der Patient zuvor mit mindestens einem antiretroviralen Medikament für mindestens 3 Monate oder mindestens 6 Monate oder mindestens 9 Monate oder mindestens 12 Monate behandelt worden war.

12. Pharmazeutische Zusammensetzung zur Verwendung nach einem der Ansprüche 9-11, wobei ein vorheriges HIV-Behandlungsschema bei dem Patienten fehlschlug, das die Verabreichung mindestens eines antiretroviralen Medikaments umfasste, optional wobei das vorherige Behandlungsschema die Verabreichung mindestens eines antiretroviralen Medikaments aus jeder von zwei verschiedenen Klassen oder mindestens drei Klassen von antiretroviralen Medikamenten umfasste, wobei die verschiedenen Klassen von antiretroviralen Medikamenten aus einem Nukleosid-Reverse-Transkriptase-Inhibitor (NRTI), einem Nicht-Nukleosid-Reverse-Transkriptase-Inhibitor (NNRTI), einem Proteaseinhibitor (PI) und einem Integrase-Strangtransfer-Inhibitor (INSTI) ausgewählt sind.

## Revendications

1. Composition pharmaceutique comprenant un sel de sodium du composé de formule (Ia) : 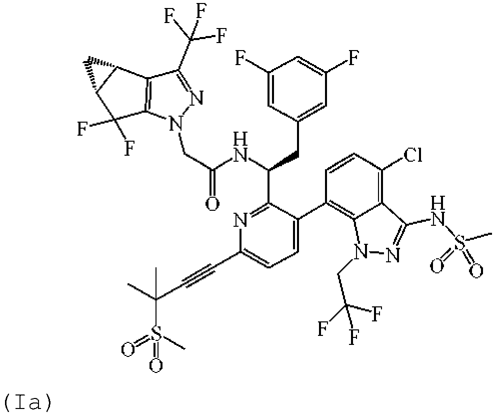 dans laquelle la composition pharmaceutique est sous la forme d'une solution et la solution comprend 35 % p/p à 75 % p/p de PEG 300, 10 % p/p à 40 % p/p d'eau et 5 % p/p à 35 % p/p du sel de sodium du composé de formule (Ia).

2. Composition pharmaceutique selon la revendication 1, dans laquelle la solution comprend 15 % p/p à 35 % p/p d'eau, 45 % p/p à 65 % p/p de PEG 300, et 10 % p/p à 30 % p/p du sel de sodium du composé de formule (Ia).

3. Composition pharmaceutique selon la revendication 1, dans laquelle la solution comprend 20 % p/p à 30 % p/p d'eau, 48 % p/p à 60 % p/p de PEG 300, et 11 % p/p à 28 % p/p du sel de sodium du composé de formule (Ia).

4. Composition pharmaceutique selon la revendication 1, dans laquelle la solution comprend 21 % p/p à 29 % p/p d'eau, 50 % p/p à 59 % p/p de PEG 300, et 13 % p/p à 27 % p/p du sel de sodium du composé de formule (Ia).

5. Composition pharmaceutique selon la revendication 1, dans laquelle la solution comprend 23,4 % p/p à 27,5 % p/p d'eau, 50,1 % p/p à 58,8 % p/p de PEG 300, et 13,7 % p/p à 26,5 % p/p du sel de sodium du composé de formule (Ia).

6. Composition pharmaceutique selon l'une quelconque des revendications 1 à 5, dans laquelle la solution comprend 23,41 % p/p d'eau, 50,13 % p/p de PEG 300, et 26,46 % p/p du sel de sodium du composé de formule (Ia).

7. Composition pharmaceutique selon l'une quelconque des revendications 1 à 6 pour une utilisation dans un procédé de traitement d'une infection par le virus de l'immunodéficience humaine (VIH) chez un patient ayant déjà reçu de nombreux traitements.

8. Composition pharmaceutique destinée à être utilisée selon la revendication 7, dans laquelle l'infection par VIH est une infection par VIH-1 **caractérisée par** une résistance de mutant de VIH-1 à un ou plusieurs médicaments antirétroviraux, ou deux médicaments antirétroviraux ou plus, ou trois médicaments antirétroviraux ou plus.

9. Composition pharmaceutique destinée à être utilisée selon la revendication 8, dans laquelle le mutant de VIH-1 est résistant à un inhibiteur de protéase (PI), un inhibiteur nucléosidique ou nucléotidique de transcriptase inverse (NRTI), un inhibiteur non nucléosidique ou non nucléotidique de transcriptase inverse (NNRTI), ou un inhibiteur de transfert de brin d'intégrase (INSTI), facultativement dans laquelle
(i) le NRTI est choisi parmi l'emtricitabine, la lamivudine (3TC), la zidovudine (azidothymidine (AZT)), la didanosine (ddI), la didéoxyinosine, le ténofovir, le ténofovir alafénamide, le fumarate de ténofovir disoproxil, la stavudine (d4T), la zalcitabine (didéoxycytidine, ddC) et l'abacavir ; ou
(ii) le NNRTI est choisi parmi l'éfavirenz, l'étravirine, la rilpivirine, la névirapine et la delavirdine ; ou
(iii) le PI est choisi parmi l'amprénavir, l'atazanavir, le darunavir, le fosamprénavir, l'indinavir, le lopinavir, le nelfinavir, le ritonavir, le saquinavir et le tipranavir ; ou
(iv) l'INSTI est choisi parmi le raltégravir, l'elvitégravir, le dolutégravir, le cabotégravir et le bictégravir.

10. Composition pharmaceutique destinée à être utilisée selon la revendication 9, dans laquelle
(i) le mutant de VIH-1 résistant à un inhibiteur de protéase est choisi parmi I50V, I84V/L90M, G48V/V82A/L90M, et G48V/V82S ; ou
(ii) le mutant de VIH-1 résistant à un inhibiteur nucléosidique ou nucléotidique de transcriptase inverse est choisi parmi K65R, M184V et 6TAMs ; ou
(iii) le mutant de VIH-1 résistant à un inhibiteur non nucléosidique ou non nucléotidique de transcriptase inverse est choisi parmi K103N, Y181C, Y188L, L100I/K103N, et K103N/Y181C ; ou
(iv) le mutant de VIH-1 résistant à un inhibiteur de transfert de brin d'intégrase est choisi parmi Y143R, E138K/Q148K, G140S/Q148R, E92Q/N155H, N155H/Q148R, et R263K/M50I.

11. Composition pharmaceutique destinée à être utilisée selon la revendication 9 ou la revendication 10, dans laquelle le patient a été préalablement traité par au moins un médicament antirétroviral pendant au moins 3 mois ou au moins 6 mois ou au moins 9 mois ou au moins 12 mois.

12. Composition pharmaceutique destinée à être utilisée selon l'une quelconque des revendications 9 à 11, dans laquelle le patient a échoué à un schéma thérapeutique antérieur contre le VIH comprenant l'administration d'au moins un médicament antirétroviral, facultativement dans laquelle le schéma thérapeutique antérieur comprend l'administration d'au moins un médicament antirétroviral de chacune de deux classes différentes ou d'au moins trois classes de médicaments antirétroviraux, dans laquelle les différentes classes de médicaments antirétroviraux sont choisies parmi un inhibiteur nucléosidique de transcriptase inverse (NRTI), un inhibiteur non nucléosidique de transcriptase inverse (NNRTI), un inhibiteur de protéase (PI) et un inhibiteur de transfert de brin d'intégrase (INSTI).
